# EUROPEAN PATENT APPLICATION

(11) **EP 2 682 127 A1**
(43) Date of publication of application: **08.01.2014**
(21) Application number: 13187404.2
(22) Date of filing: 30.04.2008
(51) Int. Cl.: A61K 39/116, A61P 31/04

(54) **Vaccine**

(30) Priority: 02.05.2007 GB 0708522; 28.06.2007 GB 0712658; 05.02.2008 GB 0802108
(62) Divisional of application: 08749959.6
(71) Applicant: GlaxoSmithKline Biologicals S.A., 1330 Rixensart (BE)
(72) Inventor: Poolman, Jan, 1330 Rixensart (BE)
(74) Representative: Whitaker, Duncan

(57) **Abstract**

The present invention relates to the field of vaccines and in particular to combination vaccines and co-administration schedules. The present inventors disclose that overuse of CRM in paediatric vaccines can result in bystander immune interference to certain antigens and provide solutions to this problem.

## Description

### FIELD OF THE INVENTION

The present invention relates to the field of vaccines and in particular to primary immunisation schedules, and kits for carrying our such immunisation schedules.

### BACKGROUND

The increasing number of vaccines recommended for administration in routine infant immunisation schedules makes the use of combination vaccines essential in order to minimise discomfort and maintain high compliance. Incorporation of newly introduced vaccines will be greatly facilitated if they can be used in combination with current vaccines. However, combination vaccines carry the risk of antigens interfering with the immune response to other antigens within the vaccines, and likewise co-administration of different vaccines carries a similar risk. The WHO recently stated in the Weekly epidemiological record (No. 12, 23 March, 2007) that vaccines "should not interfere significantly with the immune response to other vaccines given simultaneously". Therefore, there is a world-wide recognition of the importance of monitoring immune responses in these situations and to minimise risk of immune interference.

CRM-197 is a popular carrier for saccharide antigens, and has already been used in primary immunisation schedules in licensed vaccines, including for instance Prevnar® and Meningitec®. However, the present inventor has found that CRM can have a negative effect on the immune response to certain antigens, which are herein termed sensitive antigens. The inventor has also found other newly appreciated ways in which immune interference can occur with sensitive antigen, and methods by which this may be lessened.

Vaccination against pertussis disease introduced in the 1940's has been very successful in reducing the morbidity and mortality due to this disease in children and infants. In countries with high infant pertussis vaccination coverage, this success has been accompanied in recent decades by a shift in the epidemiology of the disease to older age groups and to very young infants, who are at a higher risk of severe complications.

Vaccination schedules that begin at 6 to 8 weeks of age leave a window of several months where the un-immunized or partially immunized infant may be vulnerable to pertussis infection from close contacts. Very early neonatal vaccination against pertussis may be a way to protect very young infants, by reducing the period in which they are vulnerable to disease. By virtue of the partial immaturity of the immune system at birth, neonatal immunization does not generally lead to rapid antibody responses, but may result in efficient immunologic priming which can act as a basis for future responses (Siegrist CA. Neonatal and early life vaccinology. Vaccine 2001, 19: 3331-3346).

This approach was investigated in the 60's using the DTPw vaccine available at the time, which resulted in temporary "immune paralysis", with reduced immune responses as compared to the classical later vaccination schedule (Provenzano W, Wetterlow LH, Sullivan CL. Immunization and antibody response in the newborn infant. I - Pertussis inoculation within twenty-four hours of birth. 1965 NEJM; Vol. 273 No. 17: 959-965). The feasibility of the approach using acellular pertussis DTPa combination vaccines was later demonstrated in a pre-clinical study by Siegrist and group (Roduit C, Bozzotti P, Mielcarek N, et al. Immunogenicity and protective efficacy of neonatal vaccination against Bordetella pertussis in a murine model: evidence for early control of pertussis. Infect Immun 2002 Jul;70(7):3521-8).

The inventors designed a clinical study to assess the feasibility of a birth dose of Pa vaccine to accelerate the development of antibody responses against pertussis. The effect of the antibody response to a primary immunisation schedule was investigated.

### SUMMARY OF THE INVENTION

The present inventor has found that overuse of CRM or other strong antigens (see definition below), for instance as a saccharide conjugate carrier, can result in immune responses to sensitive antigens that are reduced; surprisingly even if CRM is not conjugated to the sensitive antigen and even if sensitive antigen and CRM are not in the same container but are co-administered or administered in staggered fashion during primary immunisation.

Accordingly, in one embodiment of the invention, there is provided a vaccine kit comprising at least nine saccharide conjugates, wherein between two and seven saccharide conjugates inclusive are conjugated to CRM carrier protein, said kit being suitable for use in a primary immunisation schedule, said kit comprising:
a first container comprising
   a) a Hib saccharide conjugate in the presence of CRM, DT or any other DT derivative, but which is not conjugated to the CRM, DT or any other DT derivative;
   b) optionally at least one saccharide conjugate conjugated to CRM; and
   c) optionally at least one other saccharide conjugate not conjugated to CRM, DT or any other DT derivative,
and a second container comprising
   d) at least one saccharide conjugate conjugated to CRM;
   e) optionally at least one other saccharide conjugate not conjugated to CRM, DT or any other DT derivative,
and optionally a third container optionally comprising at least one saccharide conjugate, wherein
   f) optionally at least one saccharide conjugate is conjugated to CRM;
   g) optionally at least one saccharide conjugate is not conjugated to CRM, DT or any other DT derivative.

Unless specifically defined above, the nine or more saccharides may be distributed in any way amongst the containers of the invention.

Therefore, where Hib (a sensitive antigen) is in a vaccine in a first container of the invention in the presence of a DT derivative (e.g. free DT in a DTP vaccine), it may be coadministered with up to 7 conjugates that are on CRM (e.g. Prevnar® in a second container). However, if a ninth or further saccharide conjugate is added to the primary immunisation scheme (e.g. a *Neisseria meningitidis* saccharide such as MenC) in the first, second or third container then this should be conjugated to a carrier protein other than CRM.

Thus, when CRM is not present in the same container as the Hib sensitive antigen, the DT source in the first container may be a DTP vaccine.

The following table provides examples of vaccines which may be coadministered using the kits of the invention as illustrated in the above embodiment.

| 1^{st} container | 2^{nd} container | 3^{rd} container |
|---|---|---|
| Infanrix® hexa | Prevnar® | MenC(Y)-TT |
| Infanrix® hexa/MenC(Y)-TT | Prevnar® | / |
| Infanrix®-HB-IPV/Hib-MenC(Y)-TT | Prevnar® | / |

The average CRM dose per saccharide conjugate should optionally not exceed a certain load. Therefore in one embodiment of the invention, the kit as described above contains an average CRM dose per CRM-conjugated saccharide conjugate of 1-15µg, 1-10µg, 1-5 µg or 1-3µg. In a further embodiment of the invention, the kit as described above contains a total CRM load of less than 35µg, for instance 2-30µg, 5-25µg or 10-20µg.

Hence, in the kits of the invention, 3 of the 9 (or more) saccharides may be conjugated to CRM. Of these 3, one may have 2µg CRM, one may have 4µg CRM and one may have 6µg CRM. In this scenario, the average CRM load in the kit is 12µg/3 = 4µg.

Similarly, the HB surface antigen (HB) has been found to be a sensitive antigen e.g. to CRM, and the inventor has found a limit of CRM use, for instance as a carrier to a saccharide, beyond which the immune response to the sensitive antigen is reduced.

Accordingly in a further embodiment of the invention there is provided a vaccine kit comprising at least seven saccharide conjugates, wherein between two and six saccharide conjugates inclusive are conjugated to CRM carrier protein, said kit being suitable for use in a primary immunisation schedule, said kit comprising:
a first container comprising
   a) HB in the presence of CRM, DT or any other DT derivative, optionally adsorbed onto aluminium phosphate;
   b) optionally at least one saccharide conjugate conjugated to CRM; and
   c) optionally at least one saccharide conjugate not conjugated to CRM, DT or any other DT derivative,
and a second container comprising
   d) at least one saccharide conjugate conjugated to CRM;
   e) optionally at least one saccharide conjugate not conjugated to CRM, DT or any other DT derivative,
   and optionally a third container optionally comprising at least one saccharide conjugate wherein
f) optionally at least one saccharide conjugate is conjugated to CRM; optionally at least one saccharide conjugate is not conjugated to CRM, DT or any other DT derivative.

Therefore, HB may be present in the first container in the context of a DTP vaccine such as Infanrix® hexa, a 7-valent streptococcus vaccine where no more than 6 saccharides are conjugated to CRM may be present in the second container and MenC-TT may be present in the third container.

The average CRM dose per saccharide conjugate should optionally not exceed a certain load. Therefore in one embodiment of the invention, the kit as described above contains an average CRM dose per CRM-conjugated saccharide conjugate of 1-9µg, 1-6µg, 1-5µg or 1-3µg. In a further embodiment of the invention, the kit as described above contains a total CRM load of less than 20µg, for instance 2-18µg or 5-15µg.

Furthermore, the inventor suggests a way in which more than 7/8 saccharides in relation to HB/Hib sensitive antigens may be conjugated to CRM and administered with the sensitive antigen(s), specifically by making sure the sensitive antigen is not in the same container as a CRM, DT or any other DT derivative containing vaccines. Thus, in a further embodiment of the invention there is provided a vaccine kit comprising at least eight saccharide conjugates conjugated to CRM carrier protein, suitable for use in a primary immunisation schedule, said kit comprising:
a first container comprising
   a) a sensitive antigen not in the presence of CRM, DT or any other DT derivative; and
   b) optionally at least one saccharide conjugate not conjugated to CRM, DT or any other DT derivative,
and a second container comprising
   c) at least seven, eight, ten, eleven, thirteen, fourteen or fifteen saccharide conjugates conjugated to CRM;
   d) optionally at least one other saccharide conjugate not conjugated to CRM, DT or any other DT derivative,
and optionally a third container optionally comprising at least one saccharide conjugate wherein
   e) optionally at least one saccharide conjugate is conjugated to CRM;
   f) optionally at least one saccharide conjugate is not conjugated to CRM, DT or any other DT derivative

This kit has a vaccine in a first container which is not in the presence of DT derivative, e.g. not in the presence of a DTP vaccine or a CRM conjugated saccharide, it will therefore not be prone to CRM related bystander interference and may be coadministered with eight or more conjugates conjugated to CRM, e.g. a 13-valent pneumococcal saccharide conjugate conjugated to CRM in the second container or Prevnar® + MenC-CRM in a second/third container.

The following table provides examples of vaccines which may be coadministered using the kits of the invention as illustrated in the above embodiment.

| 1^{st} container | 2^{nd} container | 3^{rd} container |
|---|---|---|
| Hib or HB | Prevnar® (or 9, 10, 11, 13 or more saccharides on CRM) | DTP |
| Hib or HB | Prevnar® + MenC-CRM | DTP |
| Hib or HB | Prevnar® + DTP | MenC-CRM |
| Hib or HB | Prevnar® + MenC-CRM + DTP | / |
| Hib or HB + MenC-TT | Prevnar® (or 9, 10, 11, 13 or more saccharides on CRM) | DTP |
| Hib or HB + MenC-TT | Prevnar® + DTP | / |
| Hib or HB | Prevnar® (or 9, 10, 11, 13 or more saccharides on CRM) + MenC-TT | DTP |
| Hib or HB | Prevnar® + DTP | MenC-TT |
| Hib or HB | Prevnar® (or 9, 10, 11, 13 or more saccharides on CRM) + MenC-TT + DTP | / |

Furthermore, the inventor suggests a way in which to improve the reduction in immune response to the sensitive antigen by minimising the number of saccharides that are conjugated to CRM. Thus, in a further embodiment of the invention there is provided a vaccine kit comprising seven or more saccharide conjugates wherein fewer than seven (e.g. 6, 5, 4, 3, 2, 1 or 0) saccharide conjugates are conjugated to CRM carrier protein, said kit being suitable for use in a primary immunisation schedule, said kit comprising:
a first container comprising
   a) Hib saccharide conjugate, not conjugated to CRM, DT or any other DT derivative;
   b) optionally at least one saccharide conjugate not conjugated to CRM, DT or any other DT derivative,
and a second container comprising optionally at least 7 saccharide conjugates wherein
   c) fewer than seven (e.g. 6, 5, 4, 3, 2, 1 or 0) saccharides conjugated to CRM,
and optionally a third container comprising
   d) optionally at least one saccharide conjugate not conjugated to CRM, DT or any other DT derivative.

Such a kit could comprise Infanrix® hexa in the first container, Synflorix® in the second container and a meningococcal capsular saccharide conjugate vaccine in the third container.

The following table provides examples of vaccines, where no antigens are on CRM, which may be coadministered using the kits of the invention as illustrated in the above embodiment.

| DTP-Hib containing vaccine, such as Infanrix® hexa | Synflorix® | Men(AW)C(Y)-TT |
|---|---|---|
| Hib | Synflorix® + MenC-TT | DTP |
| Hib | DTP + Synflorix® | MenC-TT |
| Hib | Synflorix® | DTP + MenC-TT |
| Hib | DTP + Synflorix® + | / |
| | MenC-TT | |
| Hib + MenC-TT | Synflorix® | DTP |
| HibMenC(Y)(AW)-TT | Synflorix® | DTPaHBIPV |
| DTPaHBIPVHibMenC(Y)(AW) | Synflorix® | / |

The following table indicates which vaccines should and should not be coadministered according to the embodiments of the invention:

| **Primary immunisation schedules** | **Possible immune titre effects** |
|---|---|
| | |
| Hexavac® + Prevnar® | HB↓ PS6B↓ |
| Infanrix® hexa + Prevnar® | PS6B↑ HB OK |
| Pediacel® + Prevnar® | PS6B↓ |
| | |
| Infanrix® hexa + MenC-CRM | OK |
| Infanrix® penta + MenC-CRM | OK |
| Pediacel® + MenC-CRM | OK |
| Infanrix®-Hib + MenC-CRM | Hib↓ |
| | |
| Hexavac® + MenC-TT | Hib↑ |
| Infanrix® hexa + MenC-TT | Hib↑ |
| Pediacel® + MenC-TT | Hib↑ |
| | |
| Pediacel® + Prevnar® + MenC-CRM | Hib↓ |
| | |
| DTPa + HibMenC(Y)-TT | OK |
| | |
| DTPa(HB)IPVHib + Synflorix® | Hib↑ |
| Infanrix®-HB-IPV/Hib-MenC(Y)-TT + Synflorix®* | OK |
| Infanrix®-HB-IPV/Hib-MenC(Y)-TT + Prevnar®* | Hib↓ |
| | |
| DTPa(HBIPV)Hib + 13v Prevnar | Hib↓ |
| | |
| Pa at birth, then Infanrix® hexa | Hib↓ HB↓ (see example 3) |

| | |
|---|---|
| *Hypotheses given the principles outlined herein, however yet to be tested. ↑ and ↓ signify that the response to the relevant antigen is increased or decreased respectively compared to if the DTP containing vaccine (e.g. Infanrix® hexa) is administered without any other additional vaccines (i.e. is not co-administered). | |

In a further embodiment of the present invention there is provided a combination vaccine suitable for primary immunisation comprising nine or more saccharide conjugates;
a) wherein Hib saccharide conjugate is present but is not conjugated to CRM, DT or any other DT derivative;
b) wherein between two and seven saccharide conjugates inclusive are conjugated to CRM;
c) wherein one or more other saccharide conjugate(s) is not conjugated to CRM.

The average CRM dose per saccharide conjugate should optionally not exceed a certain load. Therefore in one embodiment of the invention, the combination vaccine as described above contains an average CRM dose per CRM-conjugated saccharide conjugate of 1-15µg, 1-10µg, 1-5 µg or 1-3µg. In a further embodiment of the invention, the combination as described above contains a total CRM load of less than 35µg, for instance 2-30µg, 5-25µg or 10-20µg.

In a further embodiment of the present invention there is provided a combination vaccine suitable for primary immunisation comprising seven or more saccharides;
a) wherein HB is present;
b) wherein between two and six saccharide conjugates inclusive are conjugated to CRM;
c) wherein one or more other saccharide conjugate(s) is not conjugated to CRM.

The average CRM dose per saccharide conjugate should optionally not exceed a certain load. Therefore in one embodiment of the invention, the combination vaccine as described above contains an average CRM dose per CRM-conjugated saccharide conjugate of 1-9µg, 1-6µg, 1-5µg or 1-3µg. In a further embodiment of the invention, the combination as described above contains a total CRM load of less than 20µg, for instance 2-18µg or 5-15µg.

IPV may be administered together with the sensitive antigen in kits or combination vaccines of the invention as it has a positive effect on the immune response to the sensitive antigen (i.e. it act as an immune modulator - see definition). For such positive effects, it is important that the IPV vaccine is present in the same container as the sensitive antigen. Pw may similarly act as an immune modulator.

Therefore, in one embodiment, there is provided a kit or combination vaccine of the invention wherein the container with the sensitive antigen further comprises IPV.

In a further embodiment, there is provided a kit or combination vaccine of the invention wherein the container with the sensitive antigen further comprises Pw.

In another embodiment there is provided a method of administering the kits or combination vaccines of the invention.

In one embodiment of the present invention there is provided a method of decreasing bystander interference of CRM on a sensitive antigen in a primary immunisation schedule of a vaccine comprising one or more of the following steps
a) decreasing the amount of CRM and/or number of conjugates on CRM in the vaccine (e.g. to 7, 6, 5, 4, 3, 2, 1 or 0);
b) including IPV in the vaccine comprising the sensitive antigen;
c) including Pw in the vaccine comprising the sensitive antigen;
d) decreasing DT dose in the vaccine comprising the sensitive antigen;
e) increasing dose of the sensitive antigen;
f) if Pa is present in vaccine comprising sensitive antigen, reducing the Pa dose or number of Pa components;
g) removing CRM from the vaccine comprising the sensitive antigen, or removing CRM entirely from the kit, or removing CRM, DT and DT derivatives from the vaccine comprising the sensitive antigen.

In a further embodiment of the present invention there is provided a method of decreasing bystander interference on a sensitive antigen when using a kit comprising eight or more saccharide conjugates conjugated to CRM, comprising a first container comprising
a) a sensitive antigen(s) in the presence of CRM, DT or any other DT derivative;
   and a second container comprising
b) seven or more saccharide conjugates conjugated to CRM;
c) optionally at least one other saccharide conjugate not conjugated to CRM, DT or any other DT derivative;
   and optionally a third container optionally comprising at least one saccharide conjugate which is
d) optionally conjugated to CRM;
e) optionally not conjugated to CRM,
   comprising the step of removing all CRM, DT or any other DT derivative from the container comprising the sensitive antigen or reducing the number of saccharide conjugates conjugated to CRM to no more than seven (e.g. 6, 5, 4, 3, 2, 1 or 0).

In a further embodiment of the present invention there is provided a method of immunising against disease caused by *Bordetella pertussis, Clostridium tetani, Corynebacterium diphtheriae,* Hepatitis B virus, *Haemophilus influenzae* type b, *Streptococcus pneumonia* and *Neisseria meningitidis* using the kit or combination vaccines of the invention, wherein
a) each antigen in the kit or the combination vaccine is administered 2-3 times in a primary immunisation schedule;
b) Hib is not conjugated to CRM, DT or any other DT derivative;
c) there are 7 or more *Streptococcus pneumonia* capsular saccharide antigen conjugates;
d) there is one or more neisserial capsular saccharide antigen conjugate(s);
e) the number of *Streptococcus pneumonia* and *Neisseria meningitidis* capsular saccharide antigens conjugated to CRM are fewer than 8.

In a further embodiment of the present invention there is provided a method of immunising against disease caused by *Bordetella pertussis, Clostridium tetani, Corynebacterium diphtheriae,* Hepatitis B virus, *Haemophilus influenzae* type b, *Streptococcus pneumonia* and *Neisseria meningitidis* using the kit or combination vaccine of the invention, wherein
a) each antigen in the kit or combination vaccine is administered 2-3 times in a primary immunisation schedule;
b) the Pa dose or number of Pa components are reduced.

The present inventor has observed that Pa antigens can have a negative effect on the immune response to sensitive antigens.

Therefore, in one embodiment, there is provided a kit, combination vaccine or method of the invention wherein if Pa is present, PT (or PT derivative) is present in Pa at a dose which does not exceed 10µg, 1-9, 1.5-8, 2-6, 2.5-5µg per 0.5 mL dose.

In a further embodiment there is provided a kit, combination vaccine or method of the invention wherein if Pa is present, FHA is present in Pa at a dose which does not exceed 10µg, 1-9, 1.5-8, 2-6, 2.5-5µg per 0.5 mL dose.

In a further embodiment there is provided a kit, combination vaccine or method of the invention wherein if Pa is present, PRN is present in Pa at a dose which does not exceed 6µg, 0.5-6, 0.8-5, 1-4, 2-3µg per 0.5 mL dose.

In a further embodiment there is provided a kit, combination vaccine or method of the invention wherein if Pa is present PT is present in Pa at a dose of approximately 2.5µg, FHA is present in Pa at a dose of approximately 2.5µg and PRN is present in Pa at a dose of approximately 0.8µg per 0.5mL dose.

In a further embodiment there is provided a kit, combination vaccine or method of the invention wherein if Pa is present PT is present in Pa at a dose of approximately 5µg, FHA is present in Pa at a dose of approximately 5µg and PRN is present in Pa at a dose of approximately 2.5µg per 0.5mL dose.

### DEFINITIONS

**Approximately or around:** ±10% of the stated value, but should be in keeping with the context of use.

**Bystander interference:** The effect on the immune response to a sensitive antigen (such as Hib capsular saccharide or Hepatitis B surface antigen) when strong antigen(s) such as CRM197 are administered together with sensitive antigen(s). Such interference may be observed even if the strong and sensitive antigens are not administered in the same vaccine container, but are coadministered or administered in a staggered primary immunisation schedule. E.g. CRM coadministered with a composition comprising sensitive antigen and DT.

**Co-administration:** The administration of two or more antigens in separate vaccines administered at the same or different sites, during the same visit to the practitioner. Commonly, multiple vaccines are administered at different sites - i.e. sites draining to different lymph nodes, e.g. different limbs. Though optionally this need not be the case (vaccines may be administered at sites draining to the same lymph node).

**Combined vaccine:** A vaccine conferring protection against two or more diseases using two or more separate antigen moieties.

**CRM:** Any mutant of diphtheria toxin that detoxifies the wild-type toxin and which has not been chemically detoxified. CRM-197 is a commonly used DT mutant. Other DT mutants may also include CRM176, CRM228, CRM 45 (Uchida et al J. Biol. Chem. 218; 3838-3844, 1973); CRM 9, CRM 45, CRM102, CRM 103 and CRM107 and other mutations described by Nicholls and Youle in Genetically Engineered Toxins, Ed: Frankel, Maecel Dekker Inc, 1992; deletion or mutation of Glu-148 to Asp, Gln or Ser and/or Ala 158 to Gly and other mutations disclosed in US 4709017 or US 4950740; mutation of at least one or more residues Lys 516, Lys 526, Phe 530 and/or Lys 534 and other mutations disclosed in US 5917017 or US 6455673; or fragment disclosed in US 5843711. CRM does not cover diphtheria toxin, diphtheria toxoid or toxoids of diphtheria mutants.

**DT derivative:** An antigen which is either a detoxified mutant of diphtheria toxin (e.g. CRM - see above) or a chemically detoxified form of diphtheria toxin or CRM, or any other mutant or truncate of diphtheria toxin which retains the function of eliciting antibodies which specifically bind diphtheria toxin.

**Hexavac®:** Combined diphtheria-tetanus-acellular pertussis-inactivated polio vaccine-hepatitis B*-Haemophilus influenzae* type b vaccine (DTPa-IPV-HB/Hib vaccine, Sanofi-Aventis). It contains 20IU DT, 40IU TT, 25µg PT, 25µg FHA, 40 D-antigen units poliovirus type 1, 8 D-antigen units poliovirus type 2 and 32 D-antigen units poliovirus type 3, 12µg PRP, 5µg HBsAg.

**Hib:** PRP capsular saccharide conjugated to a carrier protein. Kits or combination vaccines of the invention may comprise 1-10µg saccharide (e.g. 2-8µg, 3-7µg, 4-6µg) per dose, conjugated with or without a linker such as ADH. Example of Hib is the antigen contained in known conjugate Hib vaccines such as Hiberix® (GlaxoSmithKline Biologicals s.a.). Various protein carriers may be used in the Hib of the invention, for instance TT or NTHi PD (EP 0594610). Another possible protein carrier in Hib of the invention is OMC or OMP (outer membrane protein complex) of *Neisseria meningitidis* (e.g. as for the Hib-OMC conjugate within PedvaxHIB from Merck & Co. Inc).

**Immune modulator:** Antigen administered in a vaccine, that when administered together with a sensitive antigen and a strong antigen, improves the immune response to the disease caused by the organism from which the sensitive antigen is derived compared with if the sensitive antigen is administered together with the strong antigen only. Examples include IPV and Pw.

**Infanrix® hexa:** Combined diphtheria-tetanus-acellular pertussis-hepatitis B-inactivated polio *vaccine-Haemophilus influenzae* type b vaccine (DTPa-HBV-IPV/Hib vaccine, GlaxoSmithKline). It contains at least 30 international units (IU) DT, at least 40IU TT, 25µg PT, 25µg FHA, 8µg PRN, 10µg Hepatitis B surface antigen (HBsAg), 40 D-antigen units poliovirus type 1, 8 D-antigen units poliovirus type 2, 32 D-antigen units poliovirus type 3 and 10µg polyribosyl ribitol phosphate (PRP) conjugated to TT.

**Infanrix® penta:** Combined diphtheria-tetanus-acellular pertussis-hepatitis B-inactivated polio vaccine (DTPa-HBV-IPV vaccine, GlaxoSmithKline). It contains at least 30 IU DT, at least 40IU TT, 25µg PT, 25µg FHA, 8µg PRN, 10µg HBsAg, 40 D-antigen units poliovirus type 1, 8 D-antigen units poliovirus type 2 and 32 D-antigen units poliovirus type 3.

**Kit:** Vaccines in separate containers may be packaged together with instructions for their use together (but not in the sense of mixing the contents of the containers before administration). Alternatively vaccines may be packaged separately with instructions of how they may be used with other vaccines described in the kits of the invention. Vaccines in kits of the invention may be coloured or numbered or adopt another system for practitioners to readily recognise which vaccines/containers should be administered together in the kits of the invention and how and when they should be administered (coadministered or staggered administration in a primary immunisation schedule).

**Optionally:** herein in each instance is intended to be express basis for either the recited optional feature being present OR being absent.

**Pa:** Acellular pertussis vaccine, typically comprising PT, FHA and PRN, and optionally agglutinogens 2 and 3.

**Pediacel®**: Combined diphtheria-tetanus-acellular pertussis-inactivated polio vaccine-*Haemophilus influenzae* type b vaccine (DTPa-IPV/Hib vaccine, Sanofi-Aventis). It contains at least 30IU DT, at least 40IU TT, 20µg PT, 20µg FHA, 3µg PRN, 5µg FIM2 and FIM3, 40 D-antigen units poliovirus type 1, 8 D-antigen units poliovirus type 2 and 32 D-antigen units poliovirus type 3 and 10µg PRP conjugated to TT.

**Prevnar**®: A 7 valent *Streptococcus pneumoniae* vaccine consisting of capsular saccharides derived from the following serotypes: 4, 6B, 9V, 14, 18C, 19F, and 23F conjugates, all conjugated to CRM-197 (Wyeth).

**13-valent Prevnar:** A 13 valent *Streptococcus pneumonia* vaccine consisting of capsular saccharides conjugated to CRM-197 (Wyeth).

**Primary immunisation:** A schedule of immunisations usually in the first year of life, often comprised of 2 or 3 immunisations for each antigen, e.g. at 2, 4, 6 months or 1, 3, 5 months or 2, 3, 4 months. The antigens can be co-administered (given at the same visit, usually in different limbs and thus usually draining to different lymph nodes) or administered in a staggered protocol. Co-administered is usually preferred as it involves fewer visits to the practitioner and therefore results in better compliance.

**PS6B:** Capsular polysaccharide conjugate derived from *Streptococcus pneumoniae* serotype 6B.

**PT derivative:** toxoided pertussis toxin, or alternatively a mutant that is detoxified and therefore does not need to be chemically detoxified.

**Saccharide:** May indicate polysaccharide or oligosaccharide and includes both. Saccharide often refers to the capsular saccharide antigen from pathogenic bacteria e.g. *Haemophilus influenzae b, Neisseria meningitidis, Streptococcus pneumoniae,* and may be a full length polysaccharide or may be bacterial 'sized-saccharides' and 'oligosaccharides' (which naturally have a low number of repeat units, or which are polysaccharides reduced in size for manageability, but are still capable of inducing a protective immune response in a host) which are well known in the vaccine art (see for instance EP 497525).

**Sensitive antigen:** Antigen particularly susceptible to immune interference - in particular bystander interference in a primary immunisation schedule. Antigen administered in a vaccine, that when administered together with a strong antigen (see below) gives a reduced immune response to the disease caused by the organism from which the antigen is derived, compared with if the sensitive antigen is administered on its own. Examples include Hepatitis B surface antigen, *Haemophilus influenzae* b antigen and PS6B.

**Staggered administration:** The administration of two or more antigens in a primary immunisation schedule in separate vaccines during different visits to the practitioner. These administrations are typically spaced apart by 1-4 weeks or more.

**Strong antigen:** Antigen administered in a vaccine, that when administered together with (or at the same time as) a sensitive antigen results in a reduced immune response to the disease caused by the organism from which the sensitive antigen is derived, compared with if the sensitive antigen is administered on its own. Examples include Pa, CRM-197.

**Synflorix®:** A 10 valent *Streptococcus pneumoniae* vaccine consisting of the following conjugates: PS1-PD, PS4-PD, PS5-PD, PS6B-PD, PS7F-PD, PS9V-PD, PS14-PD, PS18C-TT, PS19F-DT and PS23F-PD conjugates (e.g. at a dose of 1, 3, 1, 1, 1, 1, 1, 3, 3, 1 µg of saccharide, respectively per human dose) (GlaxoSmithKline) (see for example WO2007/071707).

### DETAILED DESCRIPTION

Antigens in kits and combination vaccines of the invention will be present in "immunologically effective amounts" i.e. the administration of that amount to an individual, either in a single dose or as part of a series, is effective for treatment or prevention of disease. Dosage treatment is as per accepted primary immunisation schedules followed by booster doses as necessary.

### DTP vaccine components

DTP vaccines of the invention confer protection against diseases caused by *Corynebacterium diphtheriae, Clostridium tetani* and *Bordetella pertussis.* It is commonly comprised of diphtheria toxoid (DT), tetanus toxoid (TT) and either whole cell pertussis (Pw) or acellular pertussis (Pa) which is comprised of one or more components as described below.

The diphtheria antigen is typically a diphtheria toxoid. The preparation of diphtheria toxoids (DT) is well documented. Any suitable diphtheria toxoid may be used. For instance, DT may be produced by purification of the toxin from a culture of *Corynebacterium diphtheriae* followed by chemical detoxification, but is alternatively made by purification of a recombinant, or genetically detoxified analogue of the toxin (for example, CRM197, or other mutants as described in US 4,709,017, US 5,843,711, US 5,601,827, and US 5,917,017). In one embodiment, the diphtheria toxoid of the invention may be adsorbed onto an aluminium salt such as aluminium hydroxide. In another embodiment, the diphtheria toxoid of the invention may be adsorbed onto an aluminium salt such as aluminium phosphate. In a further embodiment the diphtheria toxoid may be adsorbed onto a mixture of both aluminium hydroxide and aluminium phosphate. Kits or combination vaccines of the invention usually comprise DT at a dose of between 10-120µg, 50-100µg, 70-100µg or 80-95µg.

The tetanus antigen of the invention is typically a tetanus toxoid. Methods of preparing tetanus toxoids (TT) are well known in the art. In one embodiment TT is produced by purification of the toxin from a culture of *Clostridium tetani* followed by chemical detoxification, but is alternatively made by purification of a recombinant, or genetically detoxified analogue of the toxin (for example, as described in EP 209281). Any suitable tetanus toxoid may be used. 'Tetanus toxoid' may encompass immunogenic fragments of the full-length protein (for instance Fragment C - see EP 478602). In one embodiment, the tetanus toxoid of the invention may be adsorbed onto an aluminium salt such as aluminium hydroxide. In another embodiment, the tetanus toxoid of the invention may be adsorbed onto an aluminium salt such as aluminium phosphate. In a further embodiment the tetanus toxoid may be adsorbed onto a mixture of both aluminium hydroxide and aluminium phosphate. Kits or combination vaccines of the invention usually comprise TT at a dose of between 10-60µg, 20-50µg or 30-48µg.

The pertussis component of the invention may be either acellular (Pa) where purified pertussis antigens are used or whole-cell (Pw) where killed whole cell pertussis is used as the pertussis component.

Pa of the invention can be comprised of one or more of the following: Pertussis toxoid (PT), filamentous hemagglutinin (FHA), pertactin (PRN), fimbrial agglutinogens FIM2 and FIM3. In particular it may comprise PT, FHA, PRN, FIM2 or FIM3, or of PT+FHA, PT+PRN, PT+FIM2, PT+FIM3, FHA+PRN, FHA+FIM2, FHA+FIM3, PRN+FIM2, PRN+FIM3 or FIM2+FIM3, or of PT+FHA+PRN, PT+FHA+FIM2, PT+FHA+FIM3, PT+PRN+FIM2, PT+PRN+FIM3, PT+FIM2+FIM3, FHA+PRN+FIM2, FHA+PRN+FIM3, FHA+FIM2+FIM3 or PRN+FIM2+FIM3, or of PT+FHA+PRN+FIM2, PT+FHA+PRN+FIM3 or FHA+PRN+FIM2+FIM3, or of PT+FHA+PRN+FIM2+FIM3.

Kits or combination vaccines of the invention may comprise PT detoxified by a well known method of formaldehyde treatment or by means of mutations (PT derivative). Substitutions of residues within the S 1 subunit of the protein have been found to result in a protein which retains its immunological and protective properties of the PT, but with reduced or no toxicity (EP 322533). Such mutants may be used at doses lower than 20-25µg.

In one embodiment of the invention, Pa components are present at doses commonly used in licensed vaccines (e.g. Infanrix®), such as approximately 25µg PT, 25µg FHA and 8µg PRN.

Pw of the invention is comprised of killed whole cell pertussis. Pw may be inactivated by several methods, including mercury free methods. Such methods may include heat (e.g. 56°C, 10 minutes), formaldehyde (e.g. 0.1% at 37°, 24 hours), glutaraldehyde (e.g. 0.05% at room temperature, 10 minutes), acetone-I (e.g. three treatments at room temperature) and acetone-II (e.g. three treatments at room temperature and fourth treatment at 37°C) inactivation (see for example Gupta et al., 1987, J. Biol. Stand. 15:87; Gupta et al., 1986, Vaccine, 4:185). Methods of preparing killed, whole-cell *Bordetella pertussis* (Pw) suitable for this invention are disclosed in WO 93/24148, as are suitable formulation methods for producing DT-TT-Pw-HepB vaccines. Thiomersal has been used in the past in the preparation of killed whole-cell *Bordetella pertussis.* However, in one embodiment it is not used in the formulation process of the vaccines of the present invention.

A Pw dose of 5-50 IOU, 7-40 IOU, 9-35 IOU, 11-30 IOU, 13-25 IOU, 15-21 IOU or around or exactly 20 IOU is typically used.

In one embodiment, the pertussis component(s) of the invention may be adsorbed onto an aluminium salt such as aluminium hydroxide. In another embodiment, the pertussis component of the invention may be adsorbed onto an aluminium salt such as aluminium phosphate. In a further embodiment the pertussis component may be adsorbed onto a mixture of both aluminium hydroxide and aluminium phosphate.

### IPV vaccine components

IPV of the invention may comprise inactivated polio virus type 1 (e.g. Mahoney or Brunhilde), type 2 (e.g. MEF-1), or type 3 (e.g. Saukett), or a combination of either two or all three of these types. The kits or combination vaccines of the invention may be comprised of IPV type 1 or IPV type 2 or IPV type 3, or IPV types 1 and 2, or IPV types 1 and 3, or IPV types 2 and 3, or IPV types 1, 2 and 3.

Methods of preparing inactivated poliovirus (IPV) are well known in the art. In one embodiment, IPV should comprise types 1, 2 and 3 as is common in the vaccine art, and may be the Salk polio vaccine which is inactivated with formaldehyde (see for example, Sutter et al., 2000, Pediatr. Clin. North Am. 47:287; Zimmerman & Spann 1999, Am Fam Physician 59:113; Salk et al., 1954, Official Monthly Publication of the American Public Health Association 44(5):563; Hennesen, 1981, Develop. Biol. Standard 47:139; Budowsky, 1991, Adv. Virus Res. 39:255).

In one embodiment the IPV is not adsorbed (e.g. before mixing with other components if present). In another embodiment, the IPV component(s) of the invention may be adsorbed onto an aluminium salt such as aluminium hydroxide (e.g. before or after mixing with other components if present). In another embodiment, the IPV component(s) of the invention may be adsorbed onto an aluminium salt such as aluminium phosphate. In a further embodiment the IPV component(s) may be adsorbed onto a mixture of both aluminium hydroxide and aluminium phosphate. If adsorbed, one or more IPV components may be adsorbed separately or together as a mixture. IPV may be stabilised by a particular drying process as described in W02004/039417.

Poliovirus may be grown in cell culture. The cell culture may be a VERO cell line or PMKC, which is a continuous cell line derived from monkey kidney. VERO cells can conveniently be cultured microcarriers. Culture of the VERO cells before and during viral infection may involve the use of bovine-derived material, such as calf serum, and this material should be obtained from sources which are free from bovine spongiform encephalitis (BSE). Culture may also involve materials such as lactalbumin hydrolysate. After growth, virions may be purified using techniques such as ultrafiltration, diafiltration, and chromatography. Prior to administration to patients, the viruses must be inactivated, and this can be achieved by treatment with formaldehyde.

Viruses may be grown, purified and inactivated individually, and then combined to give a bulk mixture for IPV vaccine use or for addition to the adsorbed diphtheria and tetanus antigen and pertussis components for DTPw-IPV or DTPa-IPV comprising vaccines.

Standard doses of polio vaccines today tend to contain 40 D antigen units of inactivated poliovirus type 1, 8 D antigen units of inactivated poliovirus type 2 and 32 D antigen units of inactivated poliovirus type 3 (e.g. Infanrix®-IPV™).

In one embodiment, an IPV vaccine dose of the present invention may comprise 10-36 D-antigen units of IPV type 1.

In one embodiment, an IPV vaccine dose of the present invention may comprise 2-7 D-antigen units of IPV type 2.

In one embodiment, an IPV vaccine dose of the present invention may comprise 8-29 D-antigen units of IPV type 3.

### Hepatitis B antigen

The preparation of Hepatitis B surface antigen (HBsAg) is well documented. See for example, Hartford et al., 1983, Develop. Biol. Standard 54:125, Gregg et al., 1987, Biotechnology 5:479, EP0226846, EP0299108. It may be prepared as follows. One method involves purifying the antigen in particulate form from the plasma of chronic hepatitis B carriers, as large quantities of HBsAg are synthesised in the liver and released into the blood stream during an HBV infection. Another method involves expressing the protein by recombinant DNA methods. The HBsAg may be prepared by expression in the *Saccharomyces cerevisiae* yeast, pichia, insect cells (e.g. Hi5) or mammalian cells. The HBsAg may be inserted into a plasmid, and its expression from the plasmid may be controlled by a promoter such as the "GAPDH" promoter (from the glyceraldehyde-3-phosphate dehydrogenase gene). The yeast may be cultured in a synthetic medium. HBsAg can then be purified by a process involving steps such as precipitation, ion exchange chromatography, and ultrafiltration. After purification, HBsAg may be subjected to dialysis (e.g. with cysteine). The HBsAg may be used in a particulate form.

As used herein the expression "Hepatitis B surface antigen" or "HBsAg" includes any HBsAg antigen or fragment thereof displaying the antigenicity of HBV surface antigen. It will be understood that in addition to the 226 amino acid sequence of the HBsAg S antigen (see Tiollais et al., 1985, Nature 317:489 and references therein) HBsAg as herein described may, if desired, contain all or part of a pre-S sequence as described in the above references and in EP0278940. In particular, the HBsAg may comprise a polypeptide comprising an amino acid sequence comprising residues 133-145 followed by residues 175-400 of the L-protein of HBsAg relative to the open reading frame on a Hepatitis B virus of ad serotype (this polypeptide is referred to as L*; see EP0414374). HBsAg within the scope of the invention may also include the preS1-preS2 -S polypeptide described in EP0198474 (Endotronics) or analogues thereof such as those described in EP0304578 (McCormick and Jones) HBsAg as herein described can also refer to mutants, for example the "escape mutant" described in WO 91/14703 or EP0511855A1, especially HBsAg wherein the amino acid substitution at position 145 is to arginine from glycine.

The HBsAg may be in particle form. The particles may comprise for example S protein alone or may be composite particles, for example L*, S) where L* is as defined above and S denotes the S-protein of HBsAg. The said particle is advantageously in the form in which it is expressed in yeast.

In one embodiment, HBsAg is the antigen used in EngerixB® (GlaxoSmithKline Biologicals S.A.), which is further described in WO93/24148.

Hepatitis B surface antigen may be adsorbed onto aluminium phosphate, which may be done before mixing with the other components (described in WO93/24148). The Hepatitis B component should be substantially thiomersal free (method of preparation of HBsAg without thiomersal has been previously published in EP1307473).

Kits or combination vaccines of the invention may comprise HB at a dose of approximately 10µg.

### Haemophilus influenzae b antigen(s)

Vaccines comprising antigens from *Haemophilus influenzae* type B have been described in WO97/00697. The vaccines of the invention may use any suitable Hib antigen. The antigen may be capsular saccharide (PRP) from Hib conjugated to or mixed with a carrier protein. The saccharide is a polymer of ribose, ribitol and phosphate. The Hib antigen may optionally be adsorbed onto aluminium phosphate as described in WO97/00697, or may be unadsorbed as described in WO02/00249 or may not have undergone a specific process for adsorption.

By an antigen being 'unadsorbed onto an aluminium adjuvant salt' herein it is meant that an express or dedicated adsorption step for the antigen on fresh aluminium adjuvant salt is not involved in the process of formulating the composition.

Hib may be conjugated to any carrier which can provide at least one T-helper epitope, and may be tetanus toxoid, diphtheria toxoid, Protein D or *N. meningitidis* OMC.

Hib may be lyophilised and may be reconstituted extemporaneously (e.g. with diluent, optionally comprising other antigenic components of the vaccines of the invention). In one embodiment, Hib is present at a low dose (e.g. 1-6µg, 2-4µg or around or exactly 2.5µg) as described in WO 02/00249.

In one embodiment, kits and combination vaccines of the invention comprise Hib at a dose of approximately 10µg. In another embodiment, kits and combination vaccines of the invention may comprise Hib at a dose of approximately 2.5µg.

### Neisseria meningitidis types A, B, C, W-135 or Y antigens

The kits or combination vaccines of the invention may comprise one or more capsular saccharides of a bacterium selected from the group consisting of *N. meningitidis* type A, *N. meningitidis* type B, *N. meningitidis* type C, *N. meningitidis* type Y and *N. meningitidis* type W-135 (herein after referred to as W).

In particular the kits or combination vaccines of the invention may comprise *Neisseria meningitidis* capsular saccharide conjugate from strain A, B, C, Y or W, or from strains A+B, A+C, A+Y, A+W, B+C, B+Y, B+W, C+Y, C+W or Y+W, or from strains A+B+C, A+B+Y, A+B+W, A+C+Y, A+C+W, B+C+Y, B+C+W or C+Y+W, or from strains A+B+C+Y, A+B+C+Y, A+C+Y+W, B+C+Y+W or from strains A+B+C+Y+W.

In one embodiment, the *Neisseria meningitidis* component(s) of the invention may be adsorbed onto an aluminium salt such as aluminium hydroxide. In another embodiment, the *Neisseria meningitidis* component(s) of the invention may be adsorbed onto an aluminium salt such as aluminium phosphate. In a further embodiment the *Neisseria meningitidis* component(s) may be adsorbed onto a mixture of both aluminium hydroxide and aluminium phosphate. In one embodiment the *Neisseria meningitidis* component(s) may be unadsorbed onto an adjuvant, e.g. an aluminium adjuvant salt.

### Streptococcus pneumonia antigen(s)

The kits or combination vaccines of the invention may comprise a vaccine conferring protection against *Streptococcus pneumoniae* infection. Such a vaccine is commonly comprised of saccharides from 7, 8, 9, 10, 11, 13 or more *Streptococcus pneumoniae* serotypes or may be comprised of saccharides from all 23 known *Streptococcus pneumoniae* serotypes. Examples of *Streptococcus pneumoniae* vaccines include Prevnar® and Synflorix®, which are described in the definitions section.

In one embodiment, the *Streptococcus pneumoniae* component(s) of the invention may be adsorbed onto an aluminium salt such as aluminium hydroxide. In another embodiment, the *Streptococcus pneumoniae* component(s) of the invention may be adsorbed onto an aluminium salt such as aluminium phosphate. In a further embodiment the *Streptococcus pneumoniae* component(s) may be adsorbed onto a mixture of both aluminium hydroxide and aluminium phosphate. In one embodiment the *Streptococcus pneumoniae* component(s) may be unadsorbed onto an adjuvant, e.g. an aluminium adjuvant salt.

### Conjugates

Bacterial capsular saccharide conjugates of the invention may comprise any carrier peptide, polypeptide or protein comprising at least one T-helper epitope. The carrier protein(s) used may be selected from the group consisting of: tetanus toxoid, diphtheria toxoid, CRM (including CRM197, CRM176, CRM228, CRM 45, CRM 9, CRM 45, CRM102, CRM 103 and CRM107), recombinant diphtheria toxin (as described in any of US 4,709,017, WO 93/25210, WO 95/33481, or WO 00/48638), pneumolysin (optionally chemically detoxified, or a detoxified mutant) from S. *pneumoniae* (see e.g. WO 2004/081515 and references referred to therein), OMPC from *N. meningitidis* (EP 0372501), and protein D (PD) from *H. influenzae* (EP 594610). Other carriers may include synthetic peptides (EP 0378881; EP 0427347), heat shock proteins (WO 93/17712; WO 94/03208), pertussis proteins (WO 98/58668; EP 0471177), cytokines (WO 91/01146), lymphokines (WO 91/01146), hormones (WO 91/01146), growth factors (WO 91/01146), artificial proteins comprising multiple human CD4⁺ T cell epitopes from various pathogen-derived antigens (Falugi et al., 2001, Eur. J. Immunol. 31:3816), pneumococcal surface protein PspA (WO 02/091998), iron uptake proteins (WO 01/72337), toxin A or B from C. *difficile* (WO 00/61761), pneumococcal PhtD (WO 00/37105), pneumococcal PhtDE (e.g. WO 01/98334 & WO 03/054007), PhtX, etc.

Saccharides may all be on the same carrier, particularly all saccharides from a particular organism, for instance MenA, MenC, MenW and MenY saccharides may all be conjugated to TT, DT or CRM-197. However, due to the known effect of carrier suppression, it may be advantageous if in each of the compositions of the invention the saccharide antigens contained therein ('n' antigens) are conjugated to more than one carrier. Thus (n-1) of the saccharides could be carried (separately) on one type of carrier, and 1 on a different carrier, or (n-2) on one, and 2 on two different carriers, etc. For example, in a vaccine containing 4 bacterial saccharide conjugates, 1, 2 or all four could be conjugated to different carriers). Protein D, however, may be used for various (2, 3, 4 or more) saccharides in a composition without a marked carrier suppression effect. Hib may be present as a TT, DT or CRM197 conjugate, and MenA, MenC, MenY and MenW may be either TT, DT, CRM197 or PD conjugates. Vi may be present as a TT, DT or CRM197 conjugate. Protein D is a useful carrier as it provides a further antigen which can provide protection against *H. influenzae.* In one embodiment, all saccharides are conjugated to the same carrier protein.

Vi may be conjugated to a carrier protein for instance by a method using carbodiimide (e.g. EDAC) condensation chemistry (given that the Vi repeat subunit comprises carboxylic acid groups). This could be achieved either by (i) a single carbodiimide reaction between COOH of Vi and NH2 of protein or (ii) a double carbodiimide reaction which can occur either between COOH of Vi and NH2 of a homobifunctional linker molecule and COOH of protein and NH2 of the homobifunctional linker molecule, or between COOH of Vi and NH2 of the heterobifunctional linker molecule and NH2 of protein and COOH of the heterobifunctional linker molecule.

Conjugation may be used in conjunction with free carrier protein(s). In one embodiment, when a given carrier protein is present in both free and conjugated form in a composition of the invention, the unconjugated form is no more than 5% of the total amount of the carrier protein in the composition as a whole, or in another embodiment is present at less than 2% by weight.

The saccharide may be linked to the carrier protein by any known method (for example, by Likhite, U.S. Patent 4,372,945 and by Armor et al., U.S. Patent 4,474,757), with any suitable linker where necessary.

The saccharide will typically be activated or functionalised prior to conjugation. Activation may involve, for example, cyanylating agents such as CDAP (1-cyano-dimethylaminopyridinium tetrafluoroborate) (WO 95/08348 & WO 96/29094). The cyanilation reaction can be performed under relatively mild conditions, which avoids hydrolysis of the alkaline sensitive saccharides. This synthesis allows direct coupling to a carrier protein. Other suitable techniques use carbodiimides, hydrazides, active esters, norborane, p-nitrobenzoic acid, N-hydroxysuccinimide, S-NHS, EDC or TSTU.

Linkages via a linker group may be made using any known procedure, for example, the procedures described in US 4,882,317 and US 4,695,624. One type of linkage involves reductive amination of the saccharide, coupling the resulting amino group with one end of an adipic acid linker group (EP 0477508, Porro et al., 1985, Mol. Immunol. 22:907, EP 0208375), and then coupling a protein to the other end of the adipic acid linker group. Other linkers include B-propionamido (WO 00/10599), nitrophenyl-ethylamine (Gever et al., 1979, Med. Microbiol. Immunol. 165:171), haloacyl halides (US 4,057,685), glycosidic linkages (US 4,673,574; US 4,761,283; US 4,808,700), 6-aminocaproic acid (US 4,459,286), ADH (US 4,965,338), C4 to C12 moieties (US 4,663,160), etc. As an alternative to using a linker, direct linkage can be used. Direct linkages to the protein may comprise oxidation of the saccharide followed by reductive amination with the protein, as described in, for example US 4,761,283 and US 4,356,170 or a direct CDAP reaction.

After conjugation, free and conjugated saccharides can be separated. There are many suitable methods for this separation, including hydrophobic chromatography, tangential ultrafiltration, diafiltration, etc (see also Lei et al., 2000, Dev Biol. (Basel). 103:259; WO 00/38711; US 6,146,902). In one embodiment, if a vaccine comprises a given saccharide in both free and conjugated forms, the unconjugated form is no more than 20% by weight of the total amount of that saccharide in the composition as a whole (e.g. ≤15%, ≤10%, ≤5%, ≤2%, ≤1%).

An amount of saccharide which is capable of conferring protection to a host (an effective amount) can be determined by the skilled person. In one embodiment, each dose will comprise 0.1-100 µg of saccharide, in another embodiment each dose will comprise 0.1-50 µg, in a further embodiment each dose will comprise 0.1-10 µg, in yet another embodiment each dose will comprise 1 to 5 µg.

### Adjuvants

The kits and combination vaccines of the invention may include a pharmaceutically acceptable excipient such as a suitable adjuvant. Suitable adjuvants include an aluminium salt such as aluminium hydroxide or aluminium phosphate, but may also be a salt of calcium, iron or zinc, or may be an insoluble suspension of acylated tyrosine, or acylated sugars, or may be cationically or anionically derivatised saccharides, polyphosphazenes, biodegradable microspheres, monophosphoryl lipid A (MPL), lipid A derivatives (e.g. of reduced toxicity), 3-O-deacylated MPL, quil A, Saponin, QS21, Freund's Incomplete Adjuvant (Difco Laboratories, Detroit, MI), Merck Adjuvant 65 (Merck and Company, Inc., Rahway, NJ), AS-2 (Smith-Kline Beecham, Philadelphia, PA), CpG oligonucleotides, bioadhesives and mucoadhesives, microparticles, liposomes, polyoxyethylene ether formulations, polyoxyethylene ester formulations, muramyl peptides or imidazoquinolone compounds (e.g. imiquamod and its homologues). Human immunomodulators suitable for use as adjuvants in the invention include cytokines such as interleukins (e.g. IL-1, IL-2, IL-4, IL-5, IL-6, IL-7, IL-12, etc), macrophage colony stimulating factor (M-CSF), tumour necrosis factor (TNF), granulocyte, macrophage colony stimulating factor (GM-CSF) may also be used as adjuvants.

In one embodiment of the invention, the adjuvant composition of the formulations induces an immune response predominantly of the TH1 type. High levels of TH1-type cytokines (e.g. IFN-γ, TNFα, IL-2 and IL-12) tend to favour the induction of cell mediated immune responses to an administered antigen. Within one embodiment, in which a response is predominantly TH1-type, the level of TH1-type cytokines will increase to a greater extent than the level of TH2-type cytokines. The levels of these cytokines may be readily assessed using standard assays. For a review of the families of cytokines, see Mosmann and Coffman, 1989, Ann. Rev. Immunol. 7:145.

Accordingly, suitable adjuvant systems which promote a predominantly TH1 response include, derivatives of lipid A (e.g. of reduced toxicity), Monophosphoryl lipid A (MPL) or a derivative thereof, particularly 3-de-O-acylated monophosphoryl lipid A (3D-MPL), and a combination of monophosphoryl lipid A, optionally 3-de-O-acylated monophosphoryl lipid A together with an aluminium salt. An enhanced system involves the combination of a monophosphoryl lipid A and a saponin derivative, particularly the combination of QS21 and 3D-MPL as disclosed in WO 94/00153, or a less reactogenic composition where the QS21 is quenched with cholesterol as disclosed in WO 96/33739. A particularly potent adjuvant formulation involving QS21, 3D-MPL and tocopherol in an oil in water emulsion is described in WO 95/17210. The vaccine may additionally comprise a saponin, which may be QS21. The formulation may also comprise an oil in water emulsion and tocopherol (WO 95/17210). Unmethylated CpG containing oligonucleotides (WO 96/02555) are also preferential inducers of a TH1 response and are suitable for use in the present invention.

The vaccines of the invention may also comprise combinations of aspects of one or more of the adjuvants identified above.

Al(OH)₃ / AlPO₄ ratios may be 0/115, 23/92, 69/46, 46/69, 92/23 or 115/0.

Alternatively certain components of the vaccines of the invention may be not expressly adsorbed onto adjuvant, in particular aluminium salts.

IPV may be adsorbed onto Al(OH)₃, DT may be adsorbed onto Al(OH)₃ or AlPO₄, TT may be adsorbed onto Al(OH)₃ or AlPO₄, Pw may be adsorbed onto AlPO₄, PRN may be adsorbed onto Al(OH)₃, HB may be adsorbed onto AlPO₄, Hib may be adsorbed onto AlPO₄ or unadsorbed, Men ACWY may be adsorbed onto Al(OH)₃ or AlPO₄ or unadsorbed, MenB may be adsorbed onto Al(OH)₃ or AlPO₄ or unadsorbed, Vi may be adsorbed onto Al(OH)₃ or AlPO₄ or unadsorbed, HepA may be adsorbed onto Al(OH)₃ or AlPO₄.

Antigens which are preadsorbed onto an aluminium salt can be preadsorbed individually prior to mixing. In another embodiment, a mix of antigens may be preadsorbed prior to mixing with further adjuvants. In one embodiment, IPV may be adsorbed separately or as a mixture of IPV types 1, 2 and 3.

The meaning of "adsorbed antigen" is taken to mean greater than 30%, 40%, 50%, 60%, 70%, 80%, or 90% adsorbed.

The meaning of the terms "aluminium phosphate" and "aluminium hydroxide" as used herein includes all forms of aluminium hydroxide or aluminium phosphate which are suitable for adjuvanting vaccines. For example, aluminium phosphate can be a precipitate of insoluble aluminium phosphate (amorphous, semi-crystalline or crystalline), which can be optionally but not exclusively prepared by mixing soluble aluminium salts and phosphoric acid salts. "Aluminium hydroxide" can be a precipitate of insoluble (amorphous, semi-crystalline or crystalline) aluminium hydroxide, which can be optionally but not exclusively prepared by neutralising a solution of aluminium salts. Particularly suitable are the various forms of aluminium hydroxide and aluminium phosphate gels available from commercial sources for example, Alhydrogel (aluminium hydroxide, 3% suspension in water) and Adju-for (aluminium phosphate, 2% suspension in saline) supplied by Superfos (Vedbeck, 2950 Denmark).

### Non-immunological components of vaccines of the invention

Combination vaccines of the invention will typically, in addition to the antigenic and adjuvant components mentioned above, comprise one or more "pharmaceutically acceptable carriers or excipients", which include any excipient that does not itself induce the production of antibodies harmful to the individual receiving the composition. Suitable excipients are typically large, slowly metabolised macromolecules such as proteins, saccharides, polylactic acids, polyglycolic acids, polymeric amino acids, amino acid copolymers, sucrose (Paoletti et al., 2001, Vaccine, 19:2118), trehalose (WO 00/56365), lactose and lipid aggregates (such as oil droplets or liposomes). Such carriers are well known to those of ordinary skill in the art. The vaccines may also contain diluents, such as water, saline, glycerol, etc. Additionally, auxiliary substances, such as wetting or emulsifying agents, pH buffering substances, and the like, may be present. Sterile pyrogen-free, phosphate buffered physiologic saline is a typical carrier. A thorough discussion of pharmaceutically acceptable excipients is available in reference Gennaro, 2000, Remington: The Science and Practice of Pharmacy, 20th edition, ISBN:0683306472.

Compositions of the invention may be lyophilised or in aqueous form, i.e. solutions or suspensions. Liquid formulations of this type allow the compositions to be administered direct from their packaged form, without the need for reconstitution in an aqueous medium, and are thus ideal for injection. Compositions may be presented in vials, or they may be presented in ready filled syringes. The syringes may be supplied with or without needles. A syringe will include a single dose of the composition, whereas a vial may include a single dose or multiple doses (e.g. 2 doses).

Liquid vaccines of the invention are also suitable for reconstituting other vaccines from a lyophilised form. Where a vaccine is to be used for such extemporaneous reconstitution, the invention provides a kit, which may comprise two vials, or may comprise one ready-filled syringe and one vial, with the contents of the syringe being used to reactivate the contents of the vial prior to inj ection.

Combination vaccines of the invention may be packaged in unit dose form or in multiple dose form (e.g. 2 doses). For multiple dose forms, vials are preferred to pre-filled syringes. Effective dosage volumes can be routinely established, but a typical human dose of the composition for injection has a volume of 0.5mL.

In one embodiment, combination vaccines of the invention have a pH of between 6.0 and 8.0, in another embodiment vaccines of the invention have a pH of between 6.3 and 6.9, e.g. 6.6±0.2. Vaccines may be buffered at this pH. Stable pH may be maintained by the use of a buffer. If a composition comprises an aluminium hydroxide salt, a histidine buffer may be used (WO03/009869). The composition should be sterile and/or pyrogen free.

Compositions of the invention may be isotonic with respect to humans.

Combination vaccines of the invention may include an antimicrobial, particularly when packaged in a multiple dose format. Thiomersal should be avoided as this causes the IPV component to precipitate. Other antimicrobials may be used, such as 2-phenoxyethanol. Any preservative is preferably present at low levels. Preservative may be added exogenously and/or may be a component of the bulk antigens which are mixed to form the composition (e.g. present as a preservative in pertussis antigens).

In one embodiment, combination vaccines of the invention are thiomersal free or substantially thiomersal free. By thiomersal free or substantially thiomersal free it is meant that there is not enough thiomersal present in the final formulation to negatively impact the potency of the IPV component. For instance, if thiomersal is used during the Pw or Hepatitis B surface antigen purification process it should be substantially removed prior to mixture with IPV. Thiomersal content in the final vaccine should be less than 0.025µg/µg protein, 0.02µg/µg protein, 0.01µg/µg protein or 0.001µg/µg protein, for instance 0µg/µg protein. In one embodiment, thiomersal is not added nor used in the purification of any component. See for instance EP1307473 for Hepatitis B and see above for Pw processes where killing is achieved not in the presence of thiomersal.

Combination vaccines of the invention may comprise detergent e.g. a Tween (polysorbate), such as Tween 80. Detergents are generally present at low levels e.g. <0.01%.

Combination vaccines of the invention may include sodium salts (e.g. sodium chloride) to give tonicity. The composition may comprise sodium chloride. In one embodiment, the concentration of sodium chloride in the composition of the invention is in the range of 0.1 to 100 mg/mL (e.g. 1-50mg/mL, 2-20mg/mL, 5-15mg/mL) and in a further embodiment the concentration of sodium chloride is 10±2mg/mL NaCl e.g. about 9mg/mL.

Combination vaccines of the invention will generally include a buffer. A phosphate or histidine buffer is typical.

Combination vaccines of the invention may include free phosphate ions in solution (e.g. by the use of a phosphate buffer) in order to favour non-adsorption of antigens. The concentration of free phosphate ions in the composition of the invention is in one embodiment between 0.1 and 10.0mM, or in another embodiment between 1 and 5mM, or in a further embodiment about 2.5mM.

### Properties of the combination vaccines of the invention

In one embodiment the combination vaccines of the invention are formulated as a vaccine for *in vivo* administration to the host in such a way that the individual components of the composition are formulated such that the immunogenicity of individual components is not substantially impaired by other individual components of the composition. By not substantially impaired, it is meant that upon immunisation, an antibody titre against each component is obtained which is more than 60%, 70%, 80% or 90%, or 95-100% of the titre obtained when the antigen is administered in isolation. Thus, in preferred embodiments, no (significantly) detrimental effect occurs to the further components (in terms of protective efficacy) in the combination as compared to their administration in isolation.

### Vaccine formulations

In one embodiment, the combination vaccines of the invention are formulated as a vaccine for *in vivo* administration to the host, such that they confer an antibody titre superior to the criterion for seroprotection for each antigenic component for an acceptable percentage of human subjects. This is an important test in the assessment of a vaccine's efficacy throughout the population. Antigens with an associated antibody titre above which a host is considered to be seroconverted against the antigen are well known, and such titres are published by organisations such as WHO. In one embodiment, more than 80% of a statistically significant sample of subjects is seroconverted, in another embodiment more than 90% of a statistically significant sample of subjects is seroconverted, in a further embodiment more than 93% of a statistically significant sample of subjects is seroconverted and in yet another embodiment 96-100% of a statistically significant sample of subjects is seroconverted.

The amount of antigen in each vaccine dose is selected as an amount which induces an immunoprotective response without significant, adverse side effects in typical vaccines. Such amount will vary depending on which specific immunogens are employed. Generally it is expected that each dose will comprise 1-1000µg of total immunogen, or 1-100µg, or 1-40µg, or 1-5µg. An optimal amount for a particular vaccine can be ascertained by studies involving observation of antibody titres and other responses in subjects. A primary vaccination course may include 2-3 doses of vaccine, given one to two months apart, e.g. following the WHO recommendations for DTP immunisation.

### Packaging of vaccines of the invention

Combination vaccines of the invention can be packaged in various types of container e.g. in vials, in syringes, etc. A multidose vial will typically comprise a re-sealable plastic port through which a sterile needle can be inserted to remove a dose of vaccine, which reseals once the needle has been removed.

The vaccine may be supplied in various containers (e.g. 2 or 3). The contents of the containers may be mixed extemporaneously before administering to a host in a single injection or it may be administered concomitantly at different sites. The dose of the vaccine will typically be 0.5mL.

The inventors have surprisingly found that a kit provided in the above ways advantageously presents the various antigens to a host's immune system in an optimal manner. The kit provides a medical practitioner with an optimal method of immunising a host with one or more of the following advantages: protective efficacy for all antigens, minimal reactogenicity, minimal carrier suppression interference, minimal adjuvant/antigen interference, or minimal antigen/antigen interference. In such a way, these goals may be achieved with the minimum number (two) administrations, optionally occurring at the same visit to the practitioner.

In one embodiment the combination vaccines of the first and second containers are administered concomitantly at different sites (as described below under "administration of vaccines of the invention), and in an alternative embodiment the inventors envision that the contents of the first and second containers may be mixed (optionally extemporaneously) before administration as a single vaccine.

### Preparing vaccines of the invention

The present invention also provides a method for producing a vaccine formulation comprising the step of mixing the components of the vaccine together with a pharmaceutically acceptable excipient.

In one embodiment of the present invention there is provided a vaccine as herein described for use in a medicament for the treatment or prevention of diseases caused by infection by *Bordetella pertussis, Clostridium tetani, Corynebacterium diphtheriae,* Hepatitis B virus, *Haemophilus influenzae* type b, *Streptococcus pneumonia* and *Neisseria meningitidis*

Additionally, a method of immunising a human host against disease caused *Bordetella pertussis, Clostridium tetani, Corynebacterium diphtheriae,* Hepatitis B virus, *Haemophilus influenzae* type b, *Streptococcus pneumonia* and *Neisseria meningitidis,* which method comprises administering to the host an immunoprotective dose of the vaccine of the invention is also provided.

The amount of antigen in each vaccine dose is selected as an amount which induces an immunoprotective response without significant, adverse side effects in typical vaccines. Such amount will vary depending upon which specific immunogen is employed and how it is presented. In one embodiment each dose will comprise 0.1-100 µg of saccharide, in another embodiment each dose will comprise 0.1-50 µg, in a further embodiment each dose will comprise 0.1-10 µg, in yet another embodiment each dose will comprise 1 to 5 µg saccharide.

In one embodiment, the content of protein antigens in the vaccine will be in the range 1-100µg, in another embodiment the content of the protein antigens in the vaccines will be in the range 5-50µg, in a further embodiment the content of the protein antigens in the vaccines will be in the range 5 - 25µg.

Vaccine preparation is generally described in Vaccine Design ["The subunit and adjuvant approach" (eds Powell M.F. & Newman M.J.) (1995) Plenum Press New York]. Encapsulation within liposomes is described by Fullerton, US Patent 4,235,877. Conjugation of proteins to macromolecules is disclosed, for example by Likhite, US Patent 4,372,945 and by Armor et al., US Patent 4,474,757. Use of Quil A is disclosed by Dalsgaard et al., 1977, Acta Vet Scand. 18:349. 3D-MPL is available from Ribi immunochem, USA and is disclosed in British Patent Application No. 2220211 and US Patent 4,912,094. QS21 is disclosed in US Patent 5,057,540.

In one embodiment the amount of conjugate per 0.5 mL dose of bulk vaccine is less than 10 µg (of saccharide in the conjugate), in another embodiment the amount of conjugate is 1-7, in another embodiment the amount of conjugate is 2-6 µg, or in a further embodiment about 2.5, 3, 4 or 5 µg.

It will be appreciated that certain components, for example DTPw components, can be combined separately before adding the adsorbed HBsAg or other components.

A method of making combination vaccines of the invention is also provided comprising the step of mixing the antigens with a pharmaceutically acceptable excipient.

### Administration of vaccines of the invention

The invention provides a method for raising an immune response in a mammal, comprising the step of administering an effective amount of a vaccine of the invention. The vaccines can be administered prophylactically (i.e. to prevent infection) or therapeutically (i.e. to treat disease after infection). The immune response is preferably protective and preferably involves antibodies. The method may raise a booster response.

Following an initial vaccination, subjects may receive one or several booster immunisations adequately spaced. Dosing treatment can be a single dose schedule or a multiple dose schedule. Multiple doses may be used in a primary immunisation schedule and/or in a booster immunisation schedule. A primary dose schedule, which may be in the first year of life, may be followed by a booster dose schedule. Suitable timing between priming doses (e.g. between 4-16 weeks), and between priming and boosting can be routinely determined.

In one embodiment, the mammal is a human. Where the vaccine is for prophylactic use, the human is preferably a child (e.g. a toddler of infant) or a teenager; where the vaccine is for therapeutic use, the human is preferably an adult. A vaccine intended for children may also be administered to adults e.g. to assess safety, dosage, immunogenicity, etc.

The vaccine preparations of the present invention may be used to protect or treat a mammal susceptible to infection, by means of administering said vaccine directly to a patient. Direct delivery may be accomplished by parenteral injection (intramuscularly, intraperitoneally, intradermally, subcutaneously, intravenously, or to the interstitial space of a tissue); or by rectal, oral, vaginal, topical, transdermal, intranasal, ocular, aural, pulmonary or other mucosal administration. In one embodiment, administration is by intramuscular injection to the thigh or the upper arm. Injection may be via a needle (e.g. a hypodermic needle), but needle free injection may alternatively be used. A typical intramuscular dose is 0.5mL.

Bacterial infections affect various areas of the body and so the compositions of the invention may be prepared in various forms. For example, the compositions may be prepared as injectables, either as liquid solutions or suspensions. The composition may be prepared for pulmonary administration e.g. as an inhaler, using a fine powder or spray. The composition may be prepared as a suppository or pessary. The composition may be prepared for nasal, aural or ocular administration e.g. as spray, drops, gel or powder (see e.g. Almeida & Alpar, 1996, J Drug Targeting, 3:455; Bergquist et al., 1998, APMIS, 106:800). Successful intranasal administration of DTP vaccines has been reported (Ryan et al., 1999, Infect. Immun., 67:6270; Nagai et al., 2001, Vaccine, 19:4824).

In one embodiment the vaccines of the first and second (and third where applicable) containers are administered concomitantly at different sites, and in an alternative embodiment the inventors envision that the contents of the first and second containers may be mixed (optionally extemporaneously) before administration as a single vaccine.

The invention may be used to elicit systemic and/or mucosal immunity.

One way of checking the efficacy of therapeutic treatment involves monitoring bacterial infection after administration of the composition of the invention. One way of checking efficacy of prophylactic treatment involves monitoring immune responses against the antigens after administration of the composition. Immunogenicity of compositions of the invention can be determined by administering them to test subjects (e.g. children 12-16 months age, or animal models - WO 01/30390) and then determining standard immunological parameters. These immune responses will generally be determined around 4 weeks after administration of the composition, and compared to values determined before administration of the composition. Rather than assessing actual protective efficacy in patients, standard animal and *in vitro* models and correlates of protection for assessing the efficacy of DTP vaccines are well known.

All cited references and publications are incorporated by reference herein.

### EXAMPLES

### Example 1

### Summary

Infant vaccination with DTPa-Hib combinations (with or without HBV and IPV) generally leads to a high percentage of infants with anti-PRP antibody concentrations of ≥ 0.15 µg/ml anti-PRP, a criterion that is linked with a high level of protection against Hib disease after conjugate immunization. Recently it has been observed that vaccination with DTPa3-Hib was associated with atypically low antibody levels in the UK, and this was associated with breakthrough Hib cases. While absence of a toddler booster is generally believed to be a key factor explaining the lowered control of Hib disease, it is here suggested that co-administration of MenC-CRM197 conjugate that coincided with the introduction of DTPa3-Hib in the UK was likely to play a role in the lowered anti-PRP immune responses. Combining DTPa3-vaccines with IPV appears to enhance the response to some antigens, such as hepatitis B and Hib. Such DTPa(HBV)IPV-Hib combinations appear not to suffer from the impact of CRM197 co-administration on the Hib response. These observations underline the need to carefully evaluate upcoming pediatric conjugate vaccines for possible interference effects on the co-administered DTPa, HBV, IPV and Hib antigens, with particular attention to hepatitis B and Hib-TT.

**Key words:** *Haemophilus influenzae* type b, Hib, vaccine, immunity, interference, conjugate vaccine, combination vaccine, booster, inactivated polio vaccine (IPV)

### Key issues

- DTPa-based Hib combination vaccines are immunogenic and effective in preventing Hib disease. Protection is associated with 1) the ability to induce a high proportion of subjects who reach the protective antibody level of 0.15 µg after primary immunization; 2) increase in both titre and quality of the antibodies after the toddler booster; and 3) herd immunity effects seen mainly after the toddler booster. No differences between the various commercially available DTPa-based Hib-TT combinations have been observed in terms of the proportion of subjects who reach the 0.15 5µg cutoff after primary vaccination.
- During the late 1990's the effects of an initial catch-up campaign in the UK waned and population immunity to Hib declined. Waning immunity was not compensated for by a booster dose in the second year of life and Hib conjugate vaccine failures increased from 1999. The absence of a booster is generally believed to be a key factor explaining the lowered Hib control in the UK. During the period of lowered Hib control, the UK switched from DTPw-Hib to DTPa3-Hib, and MenC-CRM197 pediatric immunization was introduced at approximately the same time.
- Clinical trials of DTPa-based Hib combination vaccines co-administered with CRM197-containing vaccines indicate effects consistent with bystander interference on the PRP antibody response. In a situation of declining population immunity and baseline population responses consistently at the lower end of the spectrum of observed anti-PRP antibodies, the abnormally low antibody concentrations induced by DTPa3-Hib co-administered with MenC-CRM197 were insufficient to provide adequate protection to some vaccinated children, probably contributing to the increase in the number of Hib vaccine failures observed while DTPa3-Hib was in use.
- Clinical trials with DTPa3(HBV)IPV-Hib combination vaccines co-administered with MenC-CRM197 or 7vPCV-CRM197 conjugate vaccines did not reveal the co-administration bystander interference, which suggests a protective effect by, most likely, IPV. The few head-to-head studies that compared Hib-containing combination vaccines with and without IPV demonstrated higher anti-PRP and anti-HBs levels when IPV was part of the combination. Additionally, it was found that the DTPa3-Hib combination, but not the DTPa3-HBV-IPV-Hib combination induced anti-PRP antibodies with lowered avidity as compared to Hib conjugate when given separately. This may explain the susceptibility of DTPa3-Hib to CRM197-conjugate bystander interference, whilst DTPa3(HBV)IPV-Hib is not, or less susceptible to this.
- As increasing numbers of conjugate vaccines such as Hib-MenCY-TT, ACWY-DT, ACWY-CRM197, ACWY-TT, 10vPCV-Protein D and 13vPCV-CRM197, are being evaluated to be combined in infant DTPa, HBV, IPV, Hib vaccination programs, it is essential that properly controlled trials be conducted to evaluate the specific immune responses prior to implementation in a public health setting.

### Background: Haemophilus influenzae type b

Each year it is estimated that *Haemophilus influenzae type b* (Hib) causes three million serious illnesses and results in between 400,000 and 700,000 deaths worldwide [1]. Prior to the availability of effective conjugate vaccines the incidence of Hib meningitis in children 0 to 4 years ranged from 32 to 60 per 100,000 with the highest incidence and case fatality rates (up to 30%) observed in developing countries [2]. After introduction of infant vaccination with Hib conjugate vaccines, many countries now record low meningitis incidence rates of <2 per 100,000.

Hib is carried asymptomatically in the upper respiratory tract in up to 15 % of individuals [3], but only a minority of colonized individuals develop severe invasive disease. Disease results from invasion by the bacterium into the bloodstream via the respiratory epithelium, with dissemination to the central nervous system and other sites. Meningitis and septicemia are the most frequently observed clinical syndromes, and epiglottitis, arthritis, cellulitis and osteomyelitis may also occur.

The capsular polysaccharide (CP) is thought to be the most important virulence determinant of Hib due to its interaction with complement that allows it to circumvent the host anti-bacterial defence system [4]. The ability of the host to produce specific antibodies against CP plays a pivotal role in the defence against most encapsulated bacteria [5]. However the characteristics of the immune response to polysaccharide (PS) are a late development in ontogeny. Polysaccharides are in general, poorly immunogenic in infants until the age of 18-21 months, although some polysaccharides are able to induce immune responses earlier. It is believed that the marginal zone of the spleen, which is lacking in human neonates, plays an important role in the initiation and development of a polysaccharide T-independent antibody response [6]. Marginal zone dendritic cells present CP antigens to mature non-re-circulating marginal zone B cells [7]. The marginal zone of the spleen contains relatively mature B cells (IL-2 receptor positive), surface IgM, IgD, and more importantly a high density of CD21 antigen, the receptor for complement component C3d that mediates B cell activation [8]. This corresponds with observations that the immunogenicity of encapsulated bacteria is related to the complement activating properties of their PS capsule, by splitting products of C3 into C3b and C3d and by influencing antibody production to PS by activating B cells [9,10]. It is generally believed that natural priming by way of carriage of the specific or cross-reactive bacteria also is an essential component of the ability to respond to plain polysaccharides. Once the infant has responded to natural priming, immunization with plain polysaccharides becomes possible.

### Hib polysaccharide vaccines

Development of Hib CP vaccines began during the 1970s and age-dependent efficacy against invasive disease was demonstrated, with no protection observed in children vaccinated under 18 months of age [11]. Infants respond infrequently to Hib CP vaccine, with low antibody levels and no evidence of the development of immunological memory [12]. Immune responses improve after 18 months of age, although children aged 18-23 months do not respond as well as those ≥2 years of age. Adult antibody levels following vaccination are reached by the age of approximately 6 years.

The primary limitation of Hib CP vaccines is their inability to induce an immune response in infants <2 years of age, the population in whom invasive Hib disease occurs most frequently. Like other PS vaccines Hib CP vaccines provide neither long-term protection, reduction in nasopharyngeal carriage of the organism nor herd immunity. To overcome the shortcomings of the Hib CP vaccine, improved vaccines were developed by chemical conjugation of Hib CP poly-ribose-ribitol-phosphate (PRP) to T cell-dependent carrier proteins.

### Hib conjugate vaccines

The coupling of PRP to a protein carrier allowed B cells stimulated by PRP to become activated by T-helper cells, leading to early infancy antibody responses maturing over time, with parallel induction of B-cell memory to PRP. Four types of Hib conjugate vaccines with different protein carriers have since been licensed: PRP conjugated to diphtheria toxoid (PRP-D), PRP conjugated to tetanus toxoid (PRP-T), oligosaccharide Hib conjugated to CRM197 (a mutated non-toxic diphtheria toxin [the vaccine was also called HbOC]) and PRP conjugated to *Neisseria meningitidis* outer membrane protein complex (PRP-OMP). As well as differing in the nature of the protein, these vaccines differ in length of polysaccharide, the method of saccharide-protein linkage, and saccharide-protein ratio.

Immune responses to all Hib conjugate vaccines differ profoundly from those to native CP. All conjugate vaccines are highly immunogenic in adults and have proven to be more immunogenic than plain Hib CP in young children [14,15,16,17,18,19]. Re-vaccination with either conjugate vaccine or native CP induces booster responses [20] that are independent of the antibody level at the moment of revaccination [15,18,19].

The antibody response and antibody subclass distribution in adults does not differ after Hib CP conjugate or Hib CP immunization [21]. Children <2 years of age show a predominantly IgG1 response to both Hib CP and Hib conjugate vaccine, whereas both IgG1 and IgG2 antibodies are induced in adults [21]. This age difference is due to delayed maturation of the IgG2 subclass antibody response that only reaches adult levels at 8-12 years of age [22]. The large differences observed in adults with respect to the distribution of IgG1 and IgG2 anti-PRP responses correlate with the level of pre-existing natural antibodies, suggesting that natural priming favors a later IgG2 response [23]. Compared to vaccination with Hib CP, vaccination of infants with Hib conjugate vaccine increases the amount of IgG antibodies produced and increases the IgG to IgM ratio on repeated vaccination. The predominance of the IgG1 subclass increases further upon booster immunization [24].

### Licensed Hib conjugate vaccines

While all conjugate vaccines are immunogenic in young children, differences can be observed between the licensed Hib conjugate vaccines in terms of the antibody level achieved, idiotype expression, timing of antibody response elicited in infants and rate of avidity maturation over time [25].

Clinical studies of Hib conjugate vaccines show substantial variation between vaccines in terms of the magnitude of the post-vaccination antibody geometric mean concentration (GMC) achieved [26], with the lowest GMC (0.28-0.73 µg/ml) following three vaccinations with PRP-D in infants 2 to 6 months of age [13,27,28]. Maintenance of protective antibody levels also varies, with one study showing higher persisting antibody levels following PRP-T compared to PRP-OMP [29]. PRP-OMP is characterized by higher antibody responses after the first primary immunization compared to other conjugate vaccines, although post-primary and booster responses are less pronounced compared to PRP-T and Hib-CRM197 [28]. This early response suggests an intrinsic B cell mitogenic property of the PRP-OMP conjugate in addition to T helper cell activating capacity [20].

In a study of three licensed Hib conjugate vaccines, Hib-CRM197 generated the highest IgG1 levels and IgG1/IgG2 ratio compared to PRP-D and PRP-OMP [30], reflecting the higher total antibody levels induced by Hib-CRM197 [31]. In terms of functional activity of anti-PRP antibodies induced by Hib conjugate vaccines, PRP-TT induces an increased quality of anti-PRP antibodies compared to PRP-OMP [32,33]. All four Hib conjugate vaccines have been evaluated in studies of protective efficacy (Table 1) despite quite marked differences in their immunogenicity profile, all have demonstrated efficacy against invasive Hib disease when administered in at least two doses to infants, with the exception of PRP-D. Although highly effective in Finland, PRP-D failed to protect native Alaskan children [34], to a large extent as a result of the unique epidemiology of Hib disease in this population, characterized by high rates of disease that occur very early in life. Because of the early and high antibody response that is achieved after a single dose of PRP-OMP, PRP-OMP has since been successfully used in Alaska, as well as other mainly indigenous populations with similar epidemiology, such as Australian Aborigines.

### Carriage and herd immunity

In the first years after introduction of routine Hib conjugate vaccination a decrease in disease burden was observed that was disproportionate to the population being vaccinated. After the introduction of PRP-D in the US for children > 18 months of age, the incidence of Hib disease declined in children < 18 months who had not been included in the routine vaccination [39]. These data suggested that Hib conjugate vaccination not only confers protection to vaccinated toddlers and older children but also decreases transmission of Hib to unimmunized susceptible infants [40,41].

Children immunized with Hib conjugate vaccines but not plain Hib CP vaccine are at lower risk of Hib nasopharyngeal colonization than are unvaccinated children [40,41,42,43]. The responsible mechanism is suggested to be the presence of Hib CP antibodies in the nasopharyngeal mucosa [44]. A higher serum anti-PRP antibody concentration (3-7 µg/ml) seems to be needed to prevent colonization than to prevent invasive disease [20], which suggests that most herd immunity is induced by the toddler booster. In vaccinated adults, anti-PRP IgG antibodies detected in nasopharyngeal secretions and saliva are probably derived from high serum antibody concentrations [44]. Lower antibody concentrations in infants vaccinated 2-4 times but not boosted have been associated with less complete prevention of carriage [42,45,46]. Since antibodies will be high only immediately after immunization, immunological memory may also play a role in the prevention or shortening of colonization.

### Serological correlates of protection

Passive immunization studies estimated that protective anti-Hib CP antibody concentrations are between 0.05 and 0.15 µg/ml [5]. Analysis of efficacy trials using Hib CP vaccines demonstrated that 90% of infants immunized at 18-23 months of age still had Hib CP antibodies ≥0.15 µg/ml one-and-a-half years after vaccination, which correlated with the observed protective efficacy [11,12]. These studies established a necessary antibody concentration of 0.05-0.15 µg/ml at the time of exposure to colonization to prevent disease [47,48] which led to the standard procedure to express Hib CP seroprotection as the percentage ≥ 0.15 µg/ml. In the Finnish plain PRP efficacy trial the percentage of children >18 months of age with post-immunization antibody levels ≥ 1 µg/ml three weeks after immunization reflected the efficacy observed in that age group. Since antibody concentrations wane after immunization, a post-vaccination concentration of 1µg/ml was estimated to be necessary to ensure a minimum concentration of at least 0.1 µg/ml over the subsequent year.

Putative long term protective anti-PRP antibody levels derived from Hib PS studies may overestimate the anti-PRP antibody concentration required for long-term protection after Hib conjugate vaccination due to improved functional activity (isotype and avidity maturation) of the antibodies upon repeated vaccination and generation of memory B cells [47,49]. Observations from field trials with Hib conjugate vaccines support this hypothesis, although the exact concentration of serum antibody sufficient to confer protection against Hib disease is difficult to define since functional activity of anti-Hib CP antibodies is dependent on the concentration, Ig isotype and avidity.

Table 1 summarizes conjugate Hib vaccine efficacy trials where data on protective efficacy as well as immunogenicity are available. In many studies the proportion of children exceeding the 1 µg/ml putative protective threshold after the primary immunization series substantially underestimated the demonstrated protective efficacy. In contrast, the proportion of infants achieving anti-PRP antibody concentrations ≥ 0.15 µg/ml more closely reflected the observed vaccine efficacy estimates [13,49,50]. Since the quality of anti-PRP antibody increases over time following primary vaccination [51], the protective level of matured antibody may in fact be lower than 0.15 µg/ml: in the range of 0.05µg/ml [20,52].

Eskola et al. (1990) [35] postulated that any measurable antibody level in the presence of memory is sufficient for protection. In Finland, the observed protective efficacy of 90% more closely approximated the proportion of subjects with post-primary anti-PRP antibody concentrations ≥ 0.06µg/ml (85%) compared to ≥ 0.15µg/ml (70%).

Overall, although 5% to 68 % of children vaccinated with Hib conjugate vaccines do not achieve antibody levels ≥ 1 µg/ml after primary immunization (Table 1), almost all of them are primed for an antibody response to Hib CP, evidenced by the presence of detectable antibody after vaccination and are protected against Hib disease.

### Functional activity of anti-PRP antibodies

Anti-PRP antibodies generated by Hib conjugate vaccines are effective *in vitro* in both opsonophagocytosis and bactericidal tests and *in vivo* via passive immunization of infant rats followed by Hib challenge [52,53]. Complement activity of IgG1 and IgG2 fractions in healthy adults varies when exposed to Hib, although IgG1 is more active in the majority [54]. Other studies have demonstrated that a higher dose of low avidity IgG2 anti-PRP antibody is required to confer protection in an infant rat model compared to higher avidity IgG1 [33,55].

The avidity of anti-PRP antibodies increases from post primary to pre-booster after conjugate immunization [51,52] but does not increase much further after a booster dose in the second year of life. The increased avidity and induced memory probably explain why protection against Hib disease remains high even when considerable numbers of children demonstrate anti-PRP antibody concentrations < 0.15 µg/ml at pre booster periods. The increase in avidity reflects the process of somatic hypermutation of Ig genes and the subsequent selection of resulting high affinity B cells that occur in the germinal center following a T cell dependent response [56]. In some studies, a relationship between increased antibody avidity and more effective antibody function has been shown [32,57]. Antibody avidity appears to correlate with bactericidal activity [32] and has been suggested as a surrogate marker for immunological memory [56].

Although the PRP-OMP conjugate vaccine induces a different antibody repertoire with lower avidity and anti-bactericidal activity compared to other vaccines [32], PRP-OMP has proven to be efficacious. This indicates that a threshold level with respect to antibacterial activity of Hib CP antibodies exists. The relative importance of direct bactericidal or opsonophagocytic activity in the anti-Hib defense mechanism in humans is still questionable. Only very occasionally is Hib disease encountered in individuals with terminal complement component deficiency, whilst this deficiency is commonly associated with meningococcal disease [58]. Studies of Hib meningitis in C5 deficient mice demonstrated a normal Hib clearance capacity, whereas in case of C3 depletion an impaired clearance was observed indicating that opsonophagocytosis is critically important. Virtually all normal adults appear to possess opsonophagocytic capacity, being dependent upon Hib CP antibodies whereas about half of the adults demonstrate bactericidal activity [59].

### DTPa-based Hib combination vaccines

After the successful introduction of licensed Hib conjugate vaccines that resulted in rapid and impressive decreases both in Hib carriage and invasive Hib disease, DTPw and DTPa-based Hib combination vaccines were produced and introduced in a large number of countries. Mixing DTPa-based vaccines with PRP-T or Hib-CRM197 results in a lower percentage of infants with anti-PRP antibody concentrations ≥ 1 µg/ml and lower antibody GMCs than when the vaccines are administered at separate sites [60,61,62] However importantly, the proportion of infants achieving peak anti-PRP antibody concentrations ≥ 0.15 µg/ml is not different.

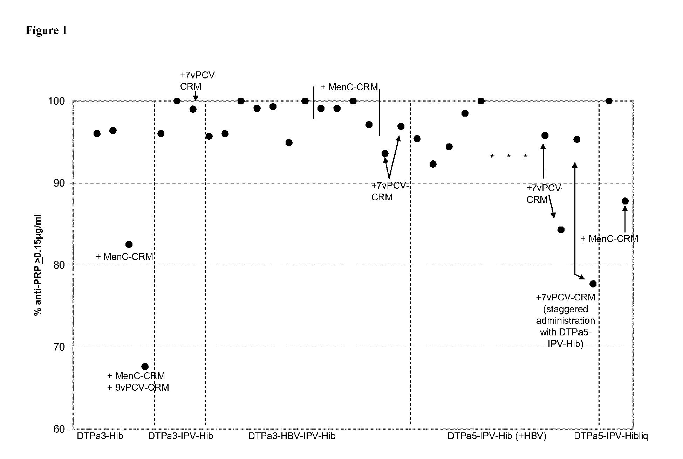

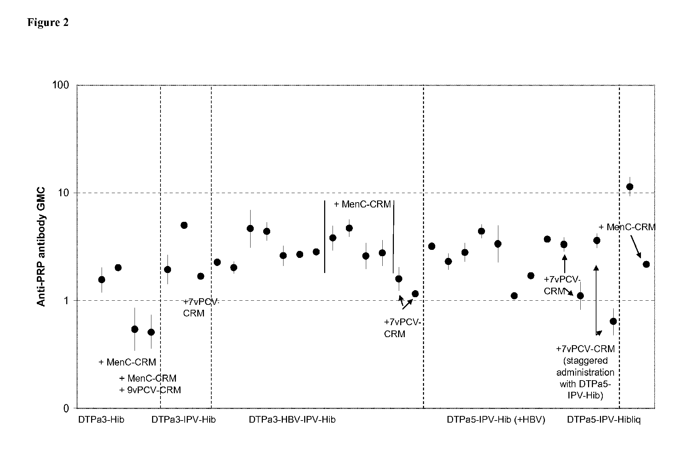

Four different DTPa-based Hib combination vaccines have been developed: one using Hib-CRM197 (no longer available), and three based on Hib-TT, combined with either DTPa2 (2-component Pa [pertussis toxoid-PT + filamentous hemagglutinin-FHA]), DTPa3 (3-component Pa [PT, FHA + pertactin-PRN]) or DTPa5 (5-component Pa [PT, FHA, PRN, Fimbriae-FIM2 + FIM3]) as the basic DTPa partner, sometimes with additional HBV and/or IPV components. The two most widely used combinations DTPa3(HB)IPV-Hib (*Infanrix*® IPV+ Hib) and DTPa5-IPV-Hib (*Pentacel*™ or *Pediacel*™) have recently been reviewed [63]. Comparable anti-PRP antibody levels are induced by the DTPa3 and DTPa5 combinations (Figures 1 and 2), which are lower as compared to separately administered Hib vaccines. One published study with *Pentacel*™ [70] showed no difference between *Pentacel*™ and Hib separate. Yet when all available data are considered, the anti-PRP response is lowered following primary vaccination with the combined *Pediacel*™ and *Pentacel*™ vaccines compared to separately administered Hib [71, 72, 73]: as observed for other DTPa-Hib combinations.

Despite the lower anti-PRP antibody concentrations achieved, DTPa-based Hib combination vaccines have been widely embraced and shown to be highly effective in preventing disease. The factors contributing to the protective effectiveness of DTPa-based Hib combinations were reviewed by Eskola and others in 1999 [13]. Briefly, immunization with DTPa-based Hib combination vaccines or DTPa-based vaccines administered separately with Hib results in >95 % of subjects with antibody levels indicative of protection and immune memory after primary vaccination (≥0.15 µg/ml). Similar immune memory responses as evidenced by similar antibody levels after boosting, develop when Hib is administered separately or mixed together with DTPa-based vaccines, consistent with the observation during Finnish efficacy studies that a detectable antibody response following primary vaccination is evidence of successful priming [35,74]. Functional activity of DTPa-based vaccine administered together with Hib vaccines has been demonstrated in terms of antibody avidity, bactericidal activity, in vivo passive protection in the rat model and opsonophagocytosis [52,53].

The clinical effectiveness of DTPa-based-Hib combinations in preventing Hib disease has been conclusively demonstrated in countries with ongoing comprehensive surveillance mechanisms such as Germany. In Germany DTPa-based Hib (PRP-T) vaccines are given at 2, 3 and 4 months of age with a booster during the second year of life. DTPa-based Hib combination vaccines have been used exclusively since 1996 (1996: DTPa-Hib, 1998; DTPa-IPV-Hib, 2000; DTPa-HBV-IPV-Hib) with estimated continuing vaccine effectiveness of 96.7% after primary immunization [62,75].

Studies with DTPw-based Hib combination vaccines show higher anti-PRP antibody concentrations post-primary immunization compared to DTPa-based Hib combinations, but both regimens show high post-booster antibody concentrations [76]. In recent years DTPa-(HBV)-IPV-Hib combinations and their co-administration with meningococcal and pneumococcal conjugate vaccines have been introduced. This influences the complexity of immune responses to individual components and enhances the risk of possible interferences between the various components.

### Enhancing effect of Inactivated Poliovirus Vaccine (IPV) in DTPa-based Hib combination vaccines

While it has been repeatedly demonstrated that combined DTPa-Hib vaccines show comparable functional activity to separate administration of Hib conjugate vaccine [13,52] there is evidence to suggest that the presence of IPV in some DTPa-based Hib combination vaccines modulates the immune response induced by the Hib conjugate component.

In a trial performed in Sweden it was noted that anti-PRP antibody concentrations were statistically significantly higher after two intramuscular injections of PRP-T mixed with DT-IPV than when mixed with DT alone [77] (Table 2). During a clinical study performed in Germany (1996-1998) subjects were randomized to receive primary vaccination at 3, 4 and 5 months of age with various DTPa3-based Hib combined vaccines. Anti-PRP antibody concentrations in subjects who received vaccines that differed only in the presence or absence of IPV are presented in Table 2.(previously unpublished results) Although there is no difference between IPV and non-IPV-containing vaccines in terms of the proportion of children reaching the 0.15 µg/ml cut-off, anti-PRP antibody levels were higher (statistically significant for DTPa3-HBV-Hib versus DTPa3-HBV-IPV-Hib) in subjects receiving IPV-containing DTPa3-based Hib combinations. It has also been observed that antibody responses to hepatitis B are higher in a combination with DTPa-IPV as compared to a combination of DTPa without IPV (Table 3) [80, 81].

An enhancing effect of IPV on the PRP response has not always been observed: in a US study, co-administration of DTPa2-Hib (PRP-T) with IPV as separate injections was associated with a reduced anti-PRP response compared to co-administration with OPV [78]. This suggests an immunostimulant/adjuvant effect when IPV is part of the DT(Pa)Hib combination and which will therefore be absent if IPV is given separately.

Antibody avidity was found to be reduced following primary vaccination with DTPa3-Hib compared to separate DTPa3 and Hib [53,76], a phenomenon that is not seen with larger DTPa3-based Hib combinations that contain IPV [52,53]. Avidity results from infants who participated in three clinical trials show no difference in anti-PRP antibody avidity maturation between mixed and separately administered DTPa3-HBV-IPV and Hib vaccines or between the DTPa3-HBV-IPV-Hib and DTPw-based Hib vaccines (Table 4). In contrast, there was an apparent difference between maturation of avidity following primary vaccination with DTPa3-Hib compared with DTPa3-HBV-IPV-Hib with a lower avidity index prior to and following the booster dose of DTPa3-Hib compared to separately administered DTPa3 and Hib vaccine. No differences were observed in the ability to protect against disease following Hib challenge in a passive infant rat protection assay [53]. In a recent report Johnson et al [76] also noted reduced antibody avidity following booster vaccination with Hib conjugate vaccine following primary vaccination with DTPa3-Hib compared to DTPw-Hib. Overall, the available data suggests that compared with vaccines containing IPV, vaccines without IPV such as DTPa3-Hib may have reduced ability to induce anti-PRP antibodies and avidity maturation, although the proportion of subjects reaching anti-PRP antibody levels indicative of protection is not altered. A recent Australian report suggested that DTPa3-IPV led to more Th1 polarized responses including enhanced IgG responses as compared to DTPa3, confirming the potential adjuvant activity of IPV [79].

### Hib conjugate vaccine failures

Because Hib conjugate vaccines induce immune memory, functional antibody and show herd immunity effects, vaccine failures have only been described occasionally following primary vaccination. Several studies have demonstrated that some conjugate-vaccinated infants with low or undetectable antibody concentrations were still protected against disease [35,39]. This greater than anticipated degree of protection was attributed in part to herd immunity. However, some role was also attributed to the protective effect of priming and memory, an effect that was evident even in very young infants who were vaccinated according to early and accelerated schedules such as the 2, 3, 4 months schedule employed in the United Kingdom [13,82]. On the other hand, analysis of antibody responses in children who had invasive Hib infection in the pre-vaccine era or those who had received Hib conjugate vaccines clearly indicated that immune memory alone was not sufficient to protect some individuals from invasive disease [83,84]. This has also been the case following MenC conjugate vaccination in the UK [85]. Recent increases in Hib vaccine failures in the UK has generated renewed interest in the effects of schedule, vaccine type, population and potential carrier-specific or bystander interferences on the immune response and mechanisms of protection conveyed by Hib conjugate vaccines.

### Interferences on the immune response to Hib conjugate vaccines

Carrier-specific interferences or enhancements can be explained via T-helper specific effects and are described further below. Bystander interferences are less easily understood. Cytokines and cytokine inhibitors produced by T-cells locally in a lymph node are not antigenically specific, and therefore active immune responses to one antigen may interfere with the immune responses to another simultaneously administered antigen in a vaccine combination given at the same site [120]. Bystander effects may also occur when co-administered vaccines containing similar components are applied in a series of immunizations, such as in pediatric schedules with DTPa and concomitant conjugates employing diphtheria and/or tetanus toxoids (DT/TT) as carrier. In the latter situation, T-cells specific for DT and/or TT may influence the immune responses since the T-cells may have traveled, reaching regional lymph nodes where the co-administered vaccine is injected [121].

### Hib conjugate vaccine failures in the United Kingdom

In the UK, routine vaccination against Hib at 2, 3 and 4 months of age using DTPw-Hib, without a booster dose was initially combined with a catch-up campaign to reach children up to 5 years of age. The campaign was highly successful and between 1989 and 1992 overall vaccine effectiveness of DTPw-Hib (Hib-CRM197 or PRP-T) was 87.1% (95% CI 65.5%; 95.2%, [86]). In an historical case-control study DTPw-Hib vaccine effectiveness of 97.3% after one year of age was considered supportive of continuation of the non-booster policy employed in the UK [87]. However, using the more sensitive screening method it later became apparent that vaccine effectiveness of DTPw-Hib after two years fell from 71.7% (3.4%; 91.7%) during the catch-up campaign to -17.0% (-272%; 63.2%) in 1998-1999 [86]. Hib vaccine failures in children >1 year of age were increasingly reported from 1999 [88] and were exacerbated between 2000 and 2002 after replacement of the DTPw-Hib vaccine with DTPa3-Hib that coincided with the introduction and co-administration with serogroup C. *N. meningitidis* conjugate vaccine MenC-CRM197 [89]. In response to the observed rise in Hib vaccine failures, a second catch-up campaign began in 2003 and all children between 6 months and 4 years received a conjugate Hib booster dose. A booster dose of Hib conjugate vaccine is now recommended at 12 months of age as part of the routine schedule [90].

There were many precipitating events that eventuated in the observed rise in Hib vaccine failures in the UK. Although it is tempting to hold the lack of a booster dose and the DTPa3-Hib vaccine employed wholly accountable, the UK experience with Hib conjugate vaccines illustrates how highly effective combination vaccines may be less effective in some settings.

### Effects of schedule and booster on the immune response to Hib conjugate vaccines

The ability of Hib conjugate vaccines to maintain a minimum antibody level as well as the induction of memory lent support to the notion that a booster dose was not necessary for long-term protection and an immunization schedule without a second year of life booster was subsequently adopted in the UK [91]. Many studies have now established the importance of the Hib booster after a primary vaccination series in generating long term protection against infection, carriage, and in strengthening immune memory [20,92,93,94]. The absence of a booster dose was associated with an increase in Hib disease [94] in Germany and with a reduction of the prevention of Hib colonization [20]. In Germany, DTPa3-based-Hib (PRP-T) vaccines are given in the same early and accelerated 2, 3 and 4 month schedule as that used in the UK, but a booster dose has been given during the second year of life since 1996. No increase in Hib vaccine failures has been reported despite the exclusive use of DTPa-based Hib combination vaccines [62,75]. Many similarities can be drawn between the Hib conjugate and MenC conjugate vaccines, where it has become apparent very early in the UK that vaccine effectiveness of MenC vaccines administered in infancy fell rapidly after the first year in the absence of a booster dose [85].

Assessment of the effect of the booster dose in Germany is potentially confounded by the fact that although DTPa3-Hib was used widely between 1996 and 1998, vaccine combinations used since 1999 contain IPV [62]. In typical circumstances the enhancing effects of IPV on the Hib response are likely to be minimal at a population level: indicators of protection (proportion ≥0.15 µg/ml [Figure 1] and protection in the infant rat Hib challenge model [53]) are similar following DTPa3-Hib and IPV-containing-DTPa-Hib combinations. In contrast, the impact of a booster dose on reducing carriage, increasing antibody concentrations, improving herd immunity and improving the immune response in inadequately primed children is substantial at a population level. The role of IPV may be more important to population immunity in situations where the immunogenicity of DTPa3-Hib is for some reason impaired and where a booster dose is not given - as described below in the UK.

### DTPa3-Hib vaccine

In the UK, for both Hib and MenC conjugate vaccines the occasional failures of protection have been associated with populations in whom baseline levels of serum anti-CP antibodies were likely to be low in relation to those levels considered to be protective [85,95,96]. Such low or even undetectable levels of specific antibody may leave an individual susceptible to rapid invasion before the primed response can take effect. Although not observed in the UK, lower Hib antibody concentrations may result in higher Hib colonization rates and reduced herd immunity, thereby increasing the risk of exposure for immunized, partially immunized and immunocompromised children.

The protective effectiveness of DTPa3-based Hib vaccines has been demonstrated and anti-PRP antibody concentrations achieved after primary vaccination with DTPa3-Hib, including in the UK when given without MenC-CRM197 co-administration, are in the same range as other DTPa3- and DTPa5-based Hib combinations vaccines [63] (Figures 1 and 2). Therefore the reasons why the demonstrably immunogenic DTPa3-Hib vaccine used in the UK exacerbated an underlying trend of increasing Hib conjugate vaccine failures requires careful assessment. In a clinical trial performed in the UK in 1996-1997 the anti-PRP response following primary vaccination with DTPa3-Hib was satisfactory (GMC 1.56) and 96.0% of children achieved anti-PRP antibody concentrations ≥0.15 µg/ml (Table 5). Critically, in practice the DTPa3-Hib vaccine in the UK was co-administered with MenC-CRM197 vaccine during 1999-2000, a co-administration that to this day has not been evaluated in controlled clinical trials.

Subsequent studies performed in the UK strongly suggest an immune interference of MenC-CRM197 (*Meningitec*™ Wyeth Lederle Vaccines, Pearl River NY) on anti-PRP antibody concentrations (Table 5), with markedly lower anti-PRP antibody GMCs and a lower proportion of subjects reaching the 0.15 µg cut-off in UK subjects vaccinated with DTPa3-Hib co-administered with MenC-CRM197 [65,96] than the study in which DTPa3-Hib was administered alone. When samples from the study performed by Slack et al [96] were tested at GlaxoSmithKline Biologicals, the anti-PRP antibody GMC was 0.54 µg/ml (95% CI 0.34;0.59) compared to 1.56 (1.19;2.04) in the 1996 study of DTPa3-Hib, also performed in the same laboratory using validated tests (previously unpublished data). In a study in which DTPa3-Hib was co-administered with *Meningitec*™ (MenC-CRM197) as well an experimental 9-valent pneumococcal vaccine (9vPCV) that also uses CRM197 as protein conjugate [65], the anti-PRP antibody GMC and proportion of subjects with concentrations ≥ 0.15 µg/ml (tested in UK labs) were also found to be exceptionally low (Figures 1 and 2).

Anti-PRP antibody concentrations and avidity maturation may be somewhat lower following priming with DTPa3-Hib than with combination vaccines that contain IPV [53]. A head-to-head study with DTPa3-HBV-Hib versus DTPa3-HBV-IPV-Hib demonstrated significantly higher anti-PRP antibody concentrations following primary vaccination with the IPV containing vaccine (Table 2). In clinical trials, DTPa3-HBV-IPV-Hib or DTPa5-IPV-Hib combination vaccines co-administered with MenC-CRM197 (*Meningitec*™) in Germany [67] and the UK [89,97] resulted in anti-PRP antibody concentrations similar to those observed using DTPa-Hib alone. In particular, the results of two German studies of DTPa3-HBV-IPV-Hib + MenC-CRM197 (*Menjugate*®), Chiron Emeryville, CA in [68] and *Meningitec*™ in [67]) in a 2-3-4 schedule are sharply in contrast to the UK study of DTPa3-Hib + MenC-CRM197 (*Meningitec*™) administered in the same schedule (anti-PRP antibody GMCs of 2.60 µg/ml [68]) or 2.78 µg/ml [67] versus 0.54 µg/ml (Table 5), respectively. These data suggest that the presence of IPV was sufficient to mask the interference of CRM197 on the Hib response. In line with the observation that the anti-PRP response may be enhanced in the presence of IPV (Table 2), other controlled studies in Spain and Germany in which infants received hexavalent DTPa3-HBV-IPV-Hib vaccine with or without MenC-CRM197 (*Meningitec*™) at 2, 4 and 6 months (Spain [98]) or 7vPCV-CRM197 at 2, 3 and 4 months (Germany [99,100]) of age showed no difference between groups in the Hib response with respect to the proportion of subjects with anti-PRP antibodies ≥ 0.15 µg/ml. In one of the studies [100] a lower proportion of subjects reaching the 1.0 µg/ml cut-off was found.

In a Canadian study when DTPa5-IPV-Hib and 7vPCV-CRM197 vaccines were administered in a staggered fashion one month apart, the anti-PRP antibody response was markedly reduced [102] (Figure 1, Figure 2). Additionally it has been found that when DTPa2-HBV-IPV-Hib was co-administered with 7vPCV-CRM197 in Germany, the hepatitis B response was significantly reduced [103] (p<0.05 2-sided t-test): this was not encountered when DTPa3-HBV-IPV-Hib was co-administered with 7vPCV-CRM197 [99].

Altogether, there is strong, albeit indirect evidence suggesting that the presence of IPV in the combined pentavalent and hexavalent vaccines largely obviates the interference observed between DTPa3-Hib and CRM197-containing vaccines. This adjuvant effect (77) appears to largely compensate for the bystander interference related to CRM197 conjugates when they are co-administered with DTPa-based Hib (PRP-TT) vaccines. Nevertheless, it seems evident that the immune enhancing effects of IPV can be overcome in certain circumstances such as a staggered administration of CRM197-containing and Hib-containing vaccines. Furthermore the effect of CRM197 may be dose-related, with greater interference when both MenC-CRM197 and 7vPNC vaccines are jointly co-administered with Hib (Figure 1). Despite the presence of IPV, DTPa2-HBV-IPV-Hib demonstrated reduced hepatitis B responses when co-administered with 7vPCV-CRM197.

Carrier-specific interferences or enhancements can be explained via T-helper-specific effects and are described further below. Bystander interferences are less easily understood. Cytokines and cytokine inhibitors produced by T cells locally in a lymph node are not antigen specific and, therefore, active immune responses to one antigen may interfere with the immune responses to another simultaneously administered antigen in a vaccine combination administered at the same site (Insel, 1995, Ann. NY Acad. Sci 754, 35). Bystander effects may also occur when coadministered vaccines containing similar components are applied in a series of immunizations, such as in pediatric schedules with DTPa and concomitant conjugates employing diphtheria toxoids (DT) or tetanus toxoids (TT) as carrier. In the latter situation, T cells specific for DT and/or TT may influence the immune responses, since the T cells may have traveled reaching regional lymph nodes where the coadministered vaccine is injected (Insel, 1995, Ann. NY Acad. Sci 754, 35).

Co-administration of multiple conjugate vaccines has previously resulted in unexpected effects: Higher anti-PRP and anti-TT immune responses but reduced responses to MenC were observed when PRP-T was co-administered with MenC-TT [97]. Conversely, when 4vPCV-TT was co-administered with DTPw-PRP-T, immune responses to both TT and Hib were inhibited in a manner that was inversely proportional to the dose of TT received [104]. The mechanism of antigen specific enhancement or interference by TT is possibly a function of T-helper cell activity as well the amount of carrier protein and polysaccharide administered [105]. An eleven-valent pneumococcal conjugate containing seven TT-conjugates demonstrated poor responses to the seven TT conjugates when co-administered to a DTPa-Hib combination as compared to a DTPw-Hib combination, which suggests that the TT T-cell responses were different in DTPa5IPVHib as compared to DTPwIPVHib [105]. This effect was not found for the four DT conjugates included in the 11vPCV.

Thus enhancement or interference of the immune response to specific antigens may be mediated by T-cell specific effects as well as non-specific 'bystander' effects, showing that the consequences of co-administration of multiple conjugate vaccines on the immune response are complex and difficult to predict. The bystander interference in relation to co-administration with CRM197 conjugates probably relates to T-cell regulatory mechanisms specific for diphtheria toxoid also being present in the DTPa(HBV)(IPV)Hib-TT combinations.

### Environmental and population factors

The effect of environmental factors on immune responses is a poorly understood but well recognized phenomenon. In a publication reviewing 146 clinical trials performed with ActHib™ (PRP-T) [107] the proportion of UK subjects who achieved anti-PRP antibody concentrations ≥0.15 µg/ml after primary vaccination was 69% (PRP-T alone) and 73% (DTPw-PRP-T) compared to rates over 90% in the other studies presented. In a study of DTPa2-Hib (ActHib™) 82% of UK subjects reached the 0.15 µg/ml cut-off, a figure which was at the lower end of the range reported for DTPa3- and DTPa5-based Hib combinations elsewhere. A possible cause of an impaired or lower immune response to vaccination may be lowered natural priming due to reduced nasopharyngeal colonization as a result of the herd effects of the immunization programme [42,95,108,109].

It has been previously reported that 30% of UK children who experience Hib conjugate vaccine failure showed minor deficiencies of immunoglobulins or subclasses that may be associated with delayed maturation of B cell responsiveness to polysaccharides [95]. Breast-feeding has a positive effect on the immune response to Hib conjugate vaccines [110]. Possible population effects on immunity arising from breast feeding practices in the UK are unknown.

Studies of antibody kinetics following disease and vaccination with Hib conjugate vaccines suggest that serum IgG antibody responses are not detectable earlier than 3-4 days following antigen exposure, even in individuals who are primed [111,112,113]. This might be particularly important if the antibody is poorly functional because of impaired avidity maturation. It therefore is not surprising that for some individuals their immunological memory fails to protect them [114]. Several studies following vaccination of premature infants with conjugate vaccines observed lower primary antibody responses [96] and reduced persistence [115]. Following the booster dose of conjugate vaccine at 12 months, however, preterm and term infants achieved the same antibody levels.

### Hib strain effects

Invasiveness of an individual Hib strain is related to the production of CP and has been associated with production of multiple copies of the capb gene sequences; genes that are involved in Hib capsule expression [116]. In a study by Cerquetti et al [117], a significantly greater proportion of strains with multiple copies of the capb gene sequences (> 2 repeats) were isolated from UK patients with true vaccine failure compared with unvaccinated children, suggesting that the level of capsular polysaccharide expression plays a role in the virulence of the strains.

From 2002 a two-three fold increase in Hib vaccine failures was observed in the Netherlands that unlike the UK, affected all ages [108]. Children in the Netherlands received primary vaccination with DTPw-IPV + Hib (separate) at 2, 3 and 4 months of age with a booster at 11 months. To date no adequate explanation for the increase is apparent, however it has been suggested that increased genetic diversity of Hib may have contributed. Investigation of the genotype of clinical Hib strains has provided evidence that adults carrying diverse Hib strains have become the source of Hib infection for children [118]. No such change in genetic diversity has been recorded in the UK [119]. These data suggest that in The Netherlands, transmission patterns changed in the vaccination period towards adult-to-child transmission versus child-to-child transmission in the pre-vaccination period.

### Expert Opinion

Hib conjugate vaccines have profoundly influenced the epidemiology of Hib disease in countries where their use has been widespread. Efficacy of all currently existing Hib conjugate vaccines has been widely demonstrated and differences between vaccines in terms of the magnitude of the anti-PRP antibody response and antibody avidity have not influenced their efficacy, except in certain groups such as indigenous populations who suffer disease at early ages and who rely on achieving high antibody concentrations after a single dose. Combined DTPa-based Hib (PRP-T) vaccines are widely used and induce antibody concentrations comparable to those produced by standalone PRP-OMP with demonstrated efficacy. Combined DTPa-based Hib vaccines induce anti-PRP with functional characteristics similar to those of separately administered Hib vaccines. After a comprehensive review of the literature [63] the National Advisory Committee in Canada recently concluded that "The anti-PRP response seems to be associated more with age and schedule of vaccine administration than with the type of vaccine." [120, p11].

Co-administering conjugate vaccines may result in either enhancement or interference due to well documented carrier-specific interactions, or less documented bystander enhancement or interference, the mechanism of which is still poorly understood. Bystander interference between DTPa-Hib and CRM-197-containing conjugates appears to be dose related, as well as influenced by the vaccination regimen. T-cell regulation of CRM197/diphtheria toxoid responses are the probable cause; although the exact mechanism still needs to be clarified. When DTPa3 and DTPa5-based (HBV)-IPV-Hib combinations are co-administered simultaneously (ie not staggered) with CRM197 conjugates no interferences have been observed (Table 5).The joint co-administration of MenC-CRM197 and PCV-CRM197 conjugates together with DTPa-(HBV)-IPV-Hib combinations remains to be elucidated. In a recent study of a novel combined 9vPCV-MenC vaccine (all conjugated to CRM197) co-administered with DTPw and PRP-T in the UK, responses to Hib, diphtheria and MenC were reduced [106], despite the known adjuvant effects of DTPw. The unpredictable nature of immune interference between co-administered conjugate vaccines highlights the importance of adequate evaluation of conjugate vaccine co-administration prior to implementation in public health programs.

Although both the lack of a booster dose and the implementation of DTPa3-Hib during a period of suboptimal control of Hib disease were indisputably linked to the rise in Hib conjugate vaccine failures in the UK, there is compelling evidence to suggest that immune interference that occurred when DTPa3-Hib was co-administered with MenC-CRM197 added to the already rising number of Hib conjugate vaccine failures observed.

Interestingly, the immune interference between CRM197-containing and Hib conjugate vaccines appears to be modulated when IPV is present in the administered DTPa3-Hib combinations. This characteristic of larger combinations requires additional investigation and may have very practical consequences for authorities wishing to co-administer several conjugated antigens in a single vaccination visit. In addition to Hib-TT, antibody responses to hepatitis B also appear to be enhanced when IPV is present in the DTPa-HBV-Hib combination. However, the IPV adjuvant effect appeared insufficient to prevent hepatitis B interference when DTPa2-HBV-IPV-Hib was co-administered with 7vPCV-CRM197.

Still many questions remain unanswered in the understanding of cellular and humoral antibody responses to polysaccharide -protein vaccinated humans and the complexity of immunological responses to combination vaccines. Ongoing post licensure testing and surveillance of Hib conjugate vaccines therefore remains critical for early detection of changing circumstances that may influence the effectiveness of administered vaccines.

### Five year review

In the next five years it is unlikely that there will be major changes to currently available and highly effective Hib-TT conjugate and hepatitis B vaccines - although increased use of Hib-conjugate vaccines containing less antigen and in novel combinations may be expected. Further introduction of DTPw-HBV-Hib combinations in developing countries will hopefully and likely take place. Decisions to co-administer conjugate vaccines will need to be supported by evidence from properly conducted clinical trials to avoid wide-ranging negative public health consequences. The area of pediatric co-administrations and potential interferences will be better described. Possible licensure of pediatric Hib-MenCY-TT, ACWY-DT, ACWY-CRM197, ACWY-TT, 10vPCV-Protein D and 13vPCV-CRM197 conjugates will take place. Co-administration of specific DTPa-combinations with specific conjugate vaccines may be recommended to avoid interference or to potentiate immune responses to vaccine components in the DTPa(HBV)IPV-Hib combinations.

Infanrix™ is a trademark of the GlaxoSmithKline group of companies. ActHib™, Pediacel™ and Pentacel™ are trademarks of Sanofi Aventis. Prevenar™ and *Meningitec*™ are trademarks of Wyeth Lederle Vaccines. Menjugate® is a trademark of Chiron.

### References

1. World Health Organization (WHO). WHO position paper on Haemophilus influenzae type b conjugate vaccines. WER. 81, 445-52 (2006).
   * general overview of Hib epidemiology and vaccine use
2. Peltola H. Worldwide Haemophilus influenzae type b disease at the beginning of the 21 st century: global analysis of the disease burden 25 years after the use of the polysaccharide vaccine and a decade after the advent of conjugates. Clin Microbiol Rev. 13, 302-17 (2000).
3. World Health Organization. Global Programme for Vaccines and Immunization (GPV): The WHO position paper on Haemophilus influenzae type b conjugate vaccines. WER. 73, 64-8 (1998).
4. Weller PF, Smith AL, Smith DH, Anderson P. Role of immunity in the clearance of bacteremia due to Haemophilus influenzae. J Infect Dis. 138(4), 427-36 (1978).
5. Santosham M, Reid R, Ambrosino DMet al. Prevention of Haemophilus influenzae type b infections in high-risk infants treated with bacterial polysaccharide immune globulin. NEngl J Med. 317, 923-9 (1987)
6. Humphrey JH. Splenic macrophages: antigen presenting cells for T1-2 antigens. Immunol Lett. 11(3-4), 149-52 (1985).
7. and presentation in vivo: the microenvironment as a crucial factor. Immunol Today. 11(12), 436-9 (1990).
8. Hsu SM. Phenotypic expression of B lymphocytes. III. Marginal zone B cells in the spleen are characterized by the expression of Tac and alkaline phosphatase. J Immunol. 135(1), 123-30 (1985).
9. BJ. Pneumococcal polysaccharides complexed with C3d bind to human B lymphocytes via complement receptor type 2. Infect Immun. 59(5), 1839-45 (1991).
10. Hostetter MK. Serotypic variations among virulent pneumococci in deposition and degradation of covalently bound C3b: implications for phagocytosis and antibody production. J Infect Dis. 153(4), 682-93 (1986).
11. Peltola H, Kayhty H, Sivonen A, Makela H. Haemophilus influenzae type b capsular polysaccharide vaccine in children: a double-blind field study of 100,000 vaccinees 3 months to 5 years of age in Finland. Pediatrics. 60(5), 730-7 (1977).
   * pivotal effectiveness trial of Hib CP vaccine
12. Kayhty H, Karanko V, Peltola H, Makela PH. Serum antibodies after vaccination with *Haemophilus influenzae* type b capsular polysaccharide and responses to reimmunization: no
13. Eskola J, Ward J, Dagan R, Goldblatt D, Zepp F, Siegrist CA. Combined vaccination of Haemophilus influenzae type b conjugate and diphtheria-tetanus-pertussis containing acellular pertussis. Lancet. 354(9195), 2063-8 (1999).
   ** pivotal review of the significance of lower anti-PRP antibody responses following vaccination with combined DTPa-based/Hib vaccines
14. Schneerson R, Robbins JB, Szu SC, Yang Y. Vacines composed of polysachardie-protein conjugates:current status, unanswered questions, and prospects for the future. In: Towards better carbohydrate vaccines. Bell R, Torrigiani G (eds). John Wiley & Sons, New York. 307-31 (1987).
15. Anderson PW, Pichichero ME, Stein EC et al. Effect of oligosaccharide chain length, exposed terminal group, and hapten loading on the antibody response of human adults and infants to vaccines consisting of Haemophilus influenzae type b capsular antigen unterminally coupled to the diphtheria protein CRM197. J Immunol. 142(7), 2464-8 (1989).
16. Berkowitz CD, Ward JI, Meier K et al. Safety and immunogenicity of Haemophilus influenzae type b polysaccharide and polysaccharide diphtheria toxoid conjugate vaccines in children 15 to 24 months of age. J Pediatr. 110(4), 509-14 (1987).
17. Shackelford PG, Granoff DM, Nelson SJ, Scott MG, Smith DS, Nahm MH. Subclass distribution of human antibodies to Haemophilus influenzae type b capsular polysaccharide. J Immunol. 138(2), 587-92 (1987).
18. Granoff DM, Sheetz KE, Nahm MH, Madassery JV, Shackelford PG. Further immunologic evaluation of children who develop haemophilus disease despite previous vaccination with type b polysaccharide vaccine. Monogr Allergy. 23, 256-68 (1988).
19. Weinberg GA, Einhorn MS, Lenoir AA, Granoff PD, Granoff DM. Immunologic priming to capsular polysaccharide in infants immunized with Haemophilus influenzae type b polysaccharide-Neisseria meningitidis outer membrane protein conjugate vaccine. J Pediatr. 111(1), 22-7 (1987).
20. Kayhty H. Difficulties in establishing a serological correlate of protection after immunization with Haemophilus influenzae conjugate vaccines. Biologicals. 22(4), 397-402 (1994).
   **Key review linking clinical and serum antibody data
21. Makela O, Mattila P, Rautonen N, Seppala I, Eskola J, Kayhty H. Isotype concentrations of human antibodies to Haemophilus influenzae type b polysaccharide (Hib) in young adults immunized with the polysaccharide as such or conjugated to a protein (diphtheria toxoid). J Immunol. 139(6), 1999-2004 (1987).
22. Morell A, Skvaril F, Hitzig WH, Barandun S. IgG subclasses: development of the serum concentrations in "normal" infants and children. J Pediatr. 80(6), 960-4 (1972).
23. Barington T, Juul L, Gyhrs A, Heilmann C. Heavy-chain isotype patterns of human antibody-secreting cells induced by Haemophilus influenzae type b conjugate vaccines in relation to age and preimmunity. Infect Immun. 62(8), 3066-74 (1994).
24. Ambrosino DM, Sood SK, Lee MC et al. IgG1, IgG2 and IgM responses to two Haemophilus influenzae type b conjugate vaccines in young infants. Pediatr Infect Dis J. 11, 855-9 (1992).
25. Granoff DM, Shackelford PG, Holmes SJ, Lucas AH. Variable region expression in the antibody responses of infants vaccinated with Haemophilus influenzae type b polysaccharide-protein conjugates. Description of a new lambda light chain-associated idiotype and the relation between idiotype expression, avidity, and vaccine formulation. The Collaborative Vaccine Study Group. J Clin Invest. 91(3), 788-96 (1993).
26. Bulkow LR, Wainwright RB, Letson GW, Chang SJ, Ward JI. Comparative immunogenicity of four Haemophilus influenzae type b conjugate vaccines in Alaska Native infants. Pediatr Infect Dis J. 1993 Jun;12(6):484-92.
27. Eskola J, Kayhty H, Peltola H et al. Antibody levels achieved in infants by course of Haemophilus influenzae type B polysaccharide/diphtheria toxoid conjugate vaccine. Lancet. 1(8439),1184-6 (1985).
28. Decker MD, Edwards KM, Bradley R, Palmer P. Comparative trial in infants of four conjugate Haemophilus influenzae type b vaccines. J Pediatr. 120(2 Pt 1), 184-9 (1992).
29. Carlsson RM, Claesson BA, Lagergard T, Kayhty H. Serum antibodies against Haemophilus influenzae type b and tetanus at 2.5 years of age: a follow-up of 2 different regimens of infant vaccination. Scand J Infect Dis. 28(5), 519-23 (1999).
30. Jelonek MT, Chang SJ, Chiu CY, Park MK, Nahm MH, Ward JI. Comparison of naturally acquired and vaccine-induced antibodies to Haemophilus influenzae type b capsular polysaccharide. Infect Immun. 61(12), 5345-50 (1993).
31. Decker MD, Edwards KM, Bradley R, Palmer P. Responses of children to booster immunization with their primary conjugate Haemophilus influenzae type B vaccine or with polyribosylribitol phosphate conjugated with diphtheria toxoid. J Pediatr. 122(3), 410-3 (1993).
32. Schlesinger Y, Granoff DM. Avidity and bactericidal activity of antibody elicited by different Haemophilus influenzae type b conjugate vaccines. The Vaccine Study Group. JAMA. 267(11), 1489-94 (1992).
33. Lucas AH, Granoff DM. Functional differences in idiotypically defined IgG1 anti-polysaccharide antibodies elicited by vaccination with Haemophilus influenzae type B polysaccharide-protein conjugates. J Immunol. 154(8), 4195-202 (1995).
34. Ward J, Brenneman G, Letson GW, Heyward WL. Limited efficacy of a Haemophilus influenzae type b conjugate vaccine in Alaska Native infants. The Alaska H. influenzae Vaccine Study Group. NEngl J Med. 323(20), 1393-401 (1990).
35. Eskola J, Kayhty H, Takala AK, et al. A randomized, prospective field trial of a conjugate vaccine in the protection of infants and young children against invasive Haemophilus influenzae type b disease. N Engl J Med. 323(20), 1381-7 (1990).
36. Peltola H, Eskola J, Kayhty H, Takala AK, Makela PH. Clinical comparison of the Haemophilus influenzae type B polysaccharide-diphtheria toxoid and the oligosaccharide-CRM197 protein vaccines in infancy. Arch Pediatr Adolesc Med. 148(6), 620-5 (1994).
37. Black SB, Shinefield HR, Fireman B, Hiatt R, Polen M, Vittinghoff E. Efficacy in infancy of oligosaccharide conjugate Haemophilus influenzae type b (HbOC) vaccine in a United States population of 61,080 children. The Northern California Kaiser Permanente Vaccine Study Center Pediatrics Group. Pediatr Infect Dis J. 10(2), 97-104 (1991).
38. Santosham M, Rivin B, Wolff M, et al. Prevention of Haemophilus influenzae type b infections in Apache and Navajo children. J Infect Dis. 165 Suppl 1, S144-51 (1992).
39. Adams WG, Deaver KA, Cochi SL et al. Decline of childhood Haemophilus influenzae type b (Hib) disease in the Hib vaccine era. JAMA. 269(2), 221-6 (1993).
40. Barbour ML. Conjugate vaccines and the carriage of Haemophilus influenzae type b. Emerg Infect Dis. 2(3), 176-82 (1996).
41. Takala AK, Eskola J, Leinonen M et al. Reduction of oropharyngeal carriage of Haemophilus influenzae type b (Hib) in children immunized with an Hib conjugate vaccine. J Infect Dis. 164(5), 982-6 (1991).
42. Murphy TV, Pastor P, Medley F, Osterholm MT, Granoff DM. Decreased Haemophilus colonization in children vaccinated with Haemophilus influenzae type b conjugate vaccine. J Pediatr. 122(4), 517-23 (1993).
43. Adegbola RA, Mulholland EK, Secka O, Jaffar S, Greenwood BM. Vaccination with a Haemophilus influenzae type b conjugate vaccine reduces oropharyngeal carriage of H. influenzae type b among Gambian children. J Infect Dis. 177(6), 1758-61 (1998).
44. Kauppi M, Eskola J, Kayhty H. Anti-capsular polysaccharide antibody concentrations in saliva after immunization with Haemophilus influenzae type b conjugate vaccines. Pediatr Infect Dis J. 14(4), 286-94 (1995).
45. Barbour ML, Booy R, Crook DW et al. Haemophilus influenzae type b carriage and immunity four years after receiving the Haemophilus influenzae oligosaccharide-CRM197 (HbOC) conjugate vaccine. Pediatr Infect Dis J. 12(6), 478-84 (1993).
46. Mohle-Boetani JC, Ajello G, Breneman E et al. Carriage of Haemophilus influenzae type b in children after widespread vaccination with conjugate Haemophilus influenzae type b vaccines. Pediatr Infect Dis J. 12(7), 589-93 (1993).
47. Käyhty H, Peltola H, Karanko V, Mäkelä PH. The protective level of serum antibodies to the capsular polysaccharide of Haemophilus influenzae type b. J Infect Dis. 147, 1100 (1983).
48. Anderson P. The protective level of serum antibodies to the capsular polysaccharide of Haemophilus influenzae type b. J Infect Dis. 149(6), 1034-5 (1984).
49. Granoff DM. Assessing efficacy of Haemophilus influenzae type b combination vaccines. Clin Infect Dis. 33 Suppl 4, S278-87 (2001).
50. Singleton R, Hammitt L, Hennessy T et al. The Alaska Haemophilus influenzae type b experience: lessons in controlling a vaccine-preventable disease. Pediatrics. 118(2), e421-9 (2006).
51. Pichichero ME, Voloshen T, Zajac D, Passador S. Avidity maturation of antibody to Haemophilus influenzae type b (Hib) after immunization with diphtheria-tetanus-acellular pertussis-hib-hepatitis B combined vaccine in infants. J Infect Dis. 180(4), 1390-3 (1999).
52. Poolman J, Kaufhold A, De Grave D, Goldblatt D. Clinical relevance of lower Hib response in DTPa-based combination vaccines. Vaccine. 19(17-19), 2280-5 (2001).
   ** key paper showing antibody avidity and bactericidal activity are not different between separately administered or mixed DTPa-based Hib vaccines containing IPV.
53. Denoel PA, Goldblatt D, Vleeschauwer I, Jacquet J-M, Pichichero ME, Poolman JT. Quality of the Haemophilus influenzae type b antibody response induced by DTPa/Hib combination vaccines. Clin Vacc Immunol.
   ** illustrates the differences between IPV and non-IPV-containing vaccines in terms of in vitro parameters
54. Bredius RG, Driedijk PC, Schouten MF, Weening RS, Out TA. Complement activation by polyclonal immunoglobulin G1 and G2 antibodies against Staphylococcus aureus, Haemophilus influenzae type b, and tetanus toxoid. Infect Immun. 60(11), 4838-47 (1992).
55. Amir J, Scott MG, Nahm MH, Granoff DM. Bactericidal and opsonic activity of IgG1 and IgG2 anticapsular antibodies to Haemophilus influenzae type b. Infect Dis. 162(1), 163-71 (1990).
56. Goldblatt D, Miller E, McCloskey N, Cartwright K., Immunological response to conjugate vaccines in infants: follow up study. BMJ. 316, 1570-1 (1998).
57. Richmond P, Borrow R, Goldblatt D et al. Ability of 3 different meningococcal C conjugate vaccines to induce immunologic memory after a single dose in UK toddlers. J Infect Dis. 183(1), 160-3 (2001).
58. Pallares DE, Figueroa JE, Densen P, Giclas PC, Marshall GS. Invasive Haemophilus influenzae type b infection in a child with familial deficiency of the beta subunit of the eighth component of complement. J Pediatr. 128(1), 102-3 (1996).
59. Musher D, Goree A, Murphy T et al. Immunity to Haemophilus influenzae type b in young adults: correlation of bactericidal and opsonizing activity of serum with antibody to polyribosylribitol phosphate and lipooligosaccharide before and after vaccination. J Infect Dis. 154(6), 935-43 (1986).
60. Schmitt HJ, Zepp F, Muschenborn S et al. Immunogenicity and reactogenicity of a Haemophilus influenzae type b tetanus conjugate vaccine when administered separately or mixed with concomitant diphtheria-tetanus-toxoid and acellular pertussis vaccine for primary and for booster immunizations. Eur J Pediatr. 157(3), 208-14 (1998).
61. Halperin SA, King J, Law B, Mills E, Willems P. Safety and immunogenicity of Haemophilus influenzae-tetanus toxoid conjugate vaccine given separately or in combination with a three-component acellular pertussis vaccine combined with diphtheria and tetanus toxoids and inactivated poliovirus vaccine for the first four doses. Clin Infect Dis. 28(5), 995-1001 (1999).
62. Schmitt HJ, von Kries R, Hassenpflug B et al. Haemophilus influenzae type b disease: impact and effectiveness of diphtheria-tetanus toxoids-acellular pertussis (-inactivated poliovirus)/H. influenzae type b combination vaccines. Pediatr Infect Dis J. 2001 Aug;20(8):767-74.
63. Gilca V, Duval B. Literature review on DTaP based penta- and hexavalent vaccines approved for clinical use in Canada. Institut national de santé publique du Québec, Canada (2006).
64. Schmitt HJ, Faber J, Lorenz I, Schmole-Thoma B, Ahlers N. The safety, reactogenicity and immunogenicity of a 7-valent pneumococcal conjugate vaccine (7VPnC) concurrently administered with a combination DTaP-IPV-Hib vaccine. Vaccine. 21(25-26), 3653-62 (2003).
65. Goldblatt D, Southern J, Ashton L et al. Immunogenicity and boosting after a reduced number of doses of a pneumococcal conjugate vaccine in infants and toddlers. Pediatr Infect Dis J. 25(4), 312-9 (2006).
   * clinical trial showing evidence of bystander interference by CRM197
66. Schmitt HJ, Knuf M, Ortiz E, Sanger R, Uwamwezi MC, Kaufhold A. Primary vaccination of infants with diphtheria-tetanus-acellular pertussis-hepatitis B virus-inactivated polio virus and Haemophilus influenzae type b vaccines given as either separate or mixed injections. J Pediatr. 137(3), 304-12 (2000).
67. Schmitt HJ, Maechler G, Habermehl P, et al. Immunogenicity, reactogenicity and immune memory after primary vaccination with a novel Haemophilus influenzae-Neisseria meningitidis serogroup C conjugate vaccine. Clin Vaccine Immunol. Feb 7, (2007).
68. Habermehl P, Leroux-Roels G, Sänger R, Mächler G, Boutriau D. Immunogenicity and Reactogenicity of a combined Haemophilus influenzae Type b and Neisseria meningitidis serogroup C and Y-tetanus toxoid conjugate (Hib-MenCY-TT) vaccine administered as a primary at 2, 3 and 4 months and as a booster at second year of life. Abstract. 15th International Pathogenic Neisseria Conference, Cairns, Australia, September 10-15 (2006).
69. Halperin BA, Halperin SA, McGrath P, Smith B, Houston T. Use of lidocaine-prilocaine patch to decrease intramuscular injection pain does not adversely affect the antibody response to diphtheria-tetanus-acellular pertussis-inactivated poliovirus-Haemophilus influenzae type b conjugate and hepatitis B vaccines in infants from birth to six months of age. Pediatr Infect Dis J. 21(5), 399-405 (2002).
70. Mills E, Gold R, Thipphawong J, et al. Safety and immunogenicity of a combined five-component pertussis-diphtheria-tetanus-inactivated poliomyelitis-Haemophilus b conjugate vaccine administered to infants at two, four, and six months of age. Vaccine. 16(6), 576-85 (1998).
71. Lin TY, Wang YH, Chang LY et al. A fully liquid diphtheria-tetanus-five component acellular pertussis-inactivated poliomyelitis-Haemophilus influenzae type b conjugate vaccine: immunogenicity and safety of primary vaccination in Taiwanese infants. Int J Infect Dis. Jun 9 (2006).
72. Lee CY, Thipphawong J, Huang LM, et al. An evaluation of the safety and immunogenicity of a five-component acellular pertussis, diphtheria, and tetanus toxoid vaccine (DTaP) when combined with a Haemophilus influenzae type b-tetanus toxoid conjugate vaccine (PRP-T) in Taiwanese infants. Pediatrics 103(1), 25-30 (1999).
73. Pentacel® DTaP-IPV/Hib Combined Diphtheria and Tetanus Toxoids and Acellular Pertussis Adsorbed, Inactivated Poliovirus and Haemophilus b Conjugate (Tetanus Toxoid Conjugate) Vaccine Combined. VRBPAC Briefing Document. Version 2: 2006.
74. Kayhty H, Eskola J, Peltola H, Saarinen L, Makela PH. High antibody responses to booster doses of either Haemophilus influenzae capsular polysaccharide or conjugate vaccine after primary immunization with conjugate vaccines. J Infect Dis. 165 Suppl 1, S165-6 (1992).
75. Kalies H, Verstraeten T, Grote V et al. Four and one-half-year follow-up of the effectiveness of diphtheria-tetanus toxoids-acellular pertussis/Haemophilus influenzae type b and diphtheria-tetanus toxoids-acellular pertussis-inactivated poliovirus/H. influenzae type b combination vaccines in Germany. Pediatr Infect Dis J. 23(10), 944-50 (2004).
   * paper illustrates ongoing effectiveness of DTPa-based/Hib combinations vaccines in preventing Hib disease in Germany (2-3-4 month schedule)
76. Johnson NG, Ruggeberg JU, Balfour GF et al. Haemophilus influenzae type b reemergence after combination immunization. Emerg Infect Dis. 12(6), 937-41 (2006).
77. Carlsson RM, Claesson BA, Kayhty H, Selstam U, Iwarson S. Studies on a Hib-tetanus toxoid conjugate vaccine: effects of co-administered tetanus toxoid vaccine, of administration route and of combined administration with an inactivated polio vaccine. Vaccine. 18(5-6), 468-78 (1999).
78. Rennels MB, Englund JA, Bernstein DI et al. Diminution of the anti-polyribosylribitol phosphate response to a combined diphtheria-tetanus-acellular pertussis/Haemophillus influenzae type b vaccine by concurrent inactivated poliovirus vaccination. Pediatr Infect Dis J. 19(5), 417-23 (2000).
79. Rowe J, Yerkovich ST, Richmond P et al. Th2-associated local reactions to the acellular diphtheria-tetanus-pertussis vaccine in 4- to 6-year-old children. Infect Immun. 73(12), 8130-5 (2005).
80. Yeh SH, Ward JI, Partridge S, et al. Safety and immunogenicity of a pentavalent diphtheria, tetanus, pertussis, hepatitis B and polio combination vaccine in infants. Pediatr Infect Dis J. 20(10), 973-80 (2001).
81. Blatter MM, Reisinger KS, Terwelp DR, DelBuono FJ, Howe BJ. Immunogenicity of a combined diphtheria-tetanus-acellular pertussis (DT-tricomponent Pa) - hepatitis B (HB)-inactivated poliovirus (IPV) admixed with Haemophilus influenzae type b (Hib) vaccine in infants. Abstract 813. American Pediatric Society and the Society for Pediatric Research. New Orleans, USA. May 1-5 (1998).
82. Kelly DF, Moxon ER, Pollard AJ. Haemophilus influenzae type b conjugate vaccines. Immunology. 113(2), 163-74 (2004).
83. Anderson P, Ingram DL, Pichichero ME, Peter G. A high degree of natural immunologic priming to the capsular polysaccharide may not prevent Haemophilus influenzae type b meningitis. Pediatr Infect Dis J. 19(7), 589-91 (2000).
84. McVernon J, Andrews N, Slack MP, Ramsay ME. Risk of vaccine failure after Haemophilus influenzae type b (Hib) combination vaccines with acellular pertussis. Lancet. 361(9368), 1521-3 (2003).
85. Trotter CL, Andrews NJ, Kaczmarski EB, Miller E, Ramsay ME. Effectiveness of meningococcal serogroup C conjugate vaccine 4 years after introduction. Lancet. 364(9431), 365-7 (2004).
   * illustrates waning immunity following primary vaccination with MenC conjugate vaccines without booster
86. Ramsay ME, McVernon J, Andrews NJ, Heath PT, Slack MP. Estimating Haemophilus influenzae type b vaccine effectiveness in England and Wales by use of the screening method. J Infect Dis. 188(4), 481-5 (2003).
   * key paper summarizing Hib vaccine failures in the UK since the introduction of conjugate vaccines.
87. Heath PT, Booy R, Azzopardi HJ et al. Antibody concentration and clinical protection after Hib conjugate vaccination in the United Kingdom. JAMA. 284(18), 2334-40 (2000).
88. Heath PT, McVernon J. The UK Hib vaccine experience. Arch Dis Child. 86(6), 396-9 (2002).
89. Slack MH, Cade S, Schapira D et al. DT5aP-Hib-IPV and MCC vaccines: preterm infants' response to accelerated immunization. Arch Dis Child. 90(4), 338-41 (2005).
   * clinical trial showing evidence of bystander interference by CRM197
90. Cameron C, Pebody R. Introduction of pneumococcal conjugate vaccine to the UK childhood immunization programme, and changes to the meningitis C and Hib schedules. Euro Surveill. 11(3), E060302.4 (2006).
91. Booy R, Heath PT, Slack MP, Begg N, Moxon ER. Vaccine failures after primary immunization with Haemophilus influenzae type-b conjugate vaccine without booster. Lancet. 349(9060), 1197-202 (1997).
92. Steinhoff M, Goldblatt D. Conjugate Hib vaccines. Lancet. 361(9355), 360-1 (2003).
93. Makela PH, Kayhty H. Evolution of conjugate vaccines. Expert Rev Vaccines. 1(3), 399-410 (2002).
94. von Kries R, Bohm O, Windfuhr A. Haemophilus influenzae b-vaccination: the urgency for timely vaccination. Eur J Pediatr. 156(4), 282-7 (1997).
   * illustrates the importance of booster vaccination in preventing Hib vaccine failures
95. Heath PT, Booy R, Griffiths H et al. Clinical and immunological risk factors associated with Haemophilus influenzae type b conjugate vaccine failure in childhood. Clin Infect Dis. 31(4), 973-80 (2000).
96. Slack MH, Schapira D, Thwaites RJ et al. Immune response of premature infants to meningococcal serogroup C and combined diphtheria-tetanus toxoids-acellular pertussis-Haemophilus influenzae type b conjugate vaccines. J Infect Dis. 184(12), 1617-20 (2001).
   * clinical trial showing evidence of bystander interference by CRM197
97. Kitchin N, Southern J, Morris R et al. Evaluation of a diphtheria-tetanus-acellular pertussis- inactivated poliovirus-Hib vaccine given concurrently with meningococcal group C conjugate vaccine at 2, 3 and 4 months of age. Arch Dis Child. May 2 (2006)
98. Tejedor JC, Omenaca F, Garcia-Sicilia J et al. Immunogenicity and reactogenicity of a three-dose primary vaccination course with a combined diphtheria-tetanus-acellular pertussis-hepatitis B-inactivated polio-haemophilus influenzae type b vaccine coadministered with a meningococcal C conjugate vaccine. Pediatr Infect Dis J. 23(12), 1109-15 (2004).
99. Knuf M, Habermehl P, Cimino C, Petersen G, Schmitt HJ. Immunogenicity, reactogenicity and safety of a 7-valent pneumococcal conjugate vaccine (PCV7) concurrently administered with a DTPa-HBV-IPV/Hib combination vaccine in healthy infants. Vaccine. 24(22), 4727-36 (2006)
100. Tichmann-Schumann I, Soemantri P, Behre U, Disselhoff J, Mahler H, Maechler G, Sanger R, Jacquet JM, Schuerman L. Immunogenicity and reactogenicity of four doses of diphtheria-tetanus-three-component acellular pertussis-hepatitis B-inactivated polio virus-Haemophilus influenzae type b vaccine coadministered with 7-valent pneumococcal conjugate Vaccine. Pediatr Infect Dis J. 24(1), 70-7 (2006).
101. Halperin SA, McDonald J, Samson L et al. Simultaneous administration of meningococcal C conjugate vaccine and diphtheria-tetanus-acellular pertussis-inactivated poliovirus-Haemophilus influenzae type b conjugate vaccine in children: a randomized double-blind study. Clin Invest Med. 25(6):243-51 (2002).
102. Scheifele DW, Halperin SA, Smith B, Ochnio J, Meloff K, Duarte-Monteiro D. Assessment of the compatibility of co-administered 7-valent pneumococcal conjugate, DTaP.IPV/PRP-T Hib and hepatitis B vaccines in infants 2-7 months of age. Vaccine. 24(12), 2057-64 (2006).
   * clinical trial showing evidence of bystander interference by CRM197
103. Olivier C, Liese JG, Stojanov S, Tetelboum R, Cottard M , Fritzell B, Laufer D, Petersen G, Corsaro B, Belohradsky. Immunogenicity and safety of the 7-valent pneumococcal conjugate vaccine (7vPNC-Prevenar®) coadministered with a hexavalent DTaP-IPV-HBV-Hib Vaccine (Hexavac ®). Poster G-836. The 42nd Interscience Conference on Antimicrobial Agents and Chemotherapy, San Diego, California. September 27-30 (2002).
104. Dagan R, Eskola J, Leclerc C, Leroy O. Reduced response to multiple vaccines sharing common protein epitopes that are administered simultaneously to infants. Infect Immun. 66(5), 2093-8 (1998).
105. Dagan R, Goldblatt D, Maleckar JR, Yaich M, Eskola J. Reduction of antibody response to an 11-valent pneumococcal vaccine coadministered with a vaccine containing acellular pertussis components. Infect Immun. 72(9), 5383-91 (2004).
106. Buttery JP, Riddell A, McVernon J et al. Immunogenicity and safety of a combination pneumococcal-meningococcal vaccine in infants: a randomized controlled trial. JAMA. 293(14), 1751-8 (2005).
107. Vidor E, Hoffenbach A, Fletcher MA. Haemophilus influenzae type b vaccine: reconstitution of lyophilised PRP-T vaccine with a pertussis-containing paediatric combination vaccine, or a change in the primary series immunization schedule, may modify the serum anti-PRP antibody responses. Curr Med Res Opin. 17(3), 197-209 (2001).
108. Spanjaard L, van den Hof S, de Melker HE, Vermeer-de Bondt PE, van der Ende A, Rijkers GT. Increase in the number of invasive Haemophilus influenzae type b infections. Ned Tijdschr Geneeskd. 149(49), 2738-42 (2005).
109. McVernon J, Howard AJ, Slack MP, Ramsay ME. Long-term impact of vaccination on Haemophilus influenzae type b (Hib) carriage in the United Kingdom. Epidemiol Infect. 132(4), 765-7 (2004).
110. Pabst HF, Spady DW. Effect of breast-feeding on antibody response to conjugate vaccine. Lancet. 1990 Aug 4;336(8710):269-70.
111. Borrow R, Southern J, Andrews N et al. Comparison of antibody kinetics following meningococcal serogroup C conjugate vaccine between healthy adults previously vaccinated with meningococcal A/C polysaccharide vaccine and vaccine-naive controls. Vaccine. 19(23-24), 3043-50 (2001).
112. Madore DV, Johnson-Kraines CL, Rothstein EP, Smith DH. Kinetics of antibody response to Haemophilus influenzae type b vaccines. Pennridge Pediatric Associates. Curr Med Res Opin. 15(2), 105-12 (1999).
113. Pichichero ME, Voloshen T, Passador S. Kinetics of booster responses to Haemophilus influenzae type B conjugate after combined diphtheria-tetanus-acelluar pertussis-Haemophilus influenzae type b vaccination in infants. Pediatr Infect Dis J. 18(12), 1106-8 (1999).
114. Lucas AH, Granoff DM. Imperfect memory and the development of Haemophilus influenzae type B disease. Pediatr Infect Dis J. 20(3), 235-9 (2001).
115. Heath PT, Booy R, McVernon J et al. Hib vaccination in infants born prematurely. Arch Dis Child. 88(3), 206-10 (2003).
116. Corn PG, Anders J, Takala AK, Kayhty H, Hoiseth SK. Genes involved in Haemophilus influenzae type b capsule expression are frequently amplified. J Infect Dis. 167(2), 356-64 (1993).
117. Cerquetti M, Cardines R, Ciofi Degli Atti ML et al. Presence of multiple copies of the capsulation b locus in invasive Haemophilus influenzae type b (Hib) strains isolated from children with Hib conjugate vaccine failure. J Infect Dis. 192(5), 819-23 (2005).
118. Schouls LM, van der Ende A, van de Pol I, Schot C, Spanjaard L, Vauterin P, Wilderbeek D, Witteveen S. Increase in genetic diversity of Haemophilus influenzae serotype b (Hib) strains after introduction of Hib vaccination in The Netherlands. J Clin Microbiol. 43(6), 2741-9 (2005).
119. Aracil B, Slack M, Perez-Vazquez M, Roman F, Ramsay M, Campos J. Molecular epidemiology of Haemophilus influenzae type b causing vaccine failures in the United Kingdom. J Clin Microbiol. 44(5), 1645-9 (2006).
120. National Advisory Committee on Immunization (NACI). Statement on the recommended use of pentavalent and hexavalent vaccines. CCDR: 33 (2007).
121. Insel RA. Potential alterations in immunogenicity by combining or simultaneously administering vaccine components. Ann N YAcad Sci. 754, 35-47 (1995).
122. Avdicova M, Prikazsky V, Hudeckova H, Schuerman L, Willems P. Immunogenicity and reactogenicity of a novel hexavalent DTPa-HBV-IPV/Hib vaccine compared to separate concomitant injections of DTPa-IPV/Hib and HBV vaccines, when administered according to a 3, 5 and 11 month vaccination schedule. Eur J Pediatr. 2002 Nov; 61(11):581-7.
123. Pichichero ME, Bernstein H, Blatter MM, Schuerman L et al. Immunogenicity and safety of a combination diphtheria, tetanus toxoid, acellular pertussis, hepatitis b, and inactivated poliovirus vaccine coadministered with a 7-valent pneumococcal conjugate vaccine and a Haemophilus influenzae type b conjugate vaccine. J Pediatr. In Press.
124. Aristegui J, Dal-Re R, Diez-Delgado J et al. Comparison of the reactogenicity and immunogenicity of a combined diphtheria, tetanus, acellular pertussis, hepatitis B, inactivated polio (DTPa-HBV-IPV) vaccine, mixed with the Haemophilus influenzae type b (Hib) conjugate vaccine and administered as a single injection, with the DTPa-IPV/Hib and hepatitis B vaccines administered in two simultaneous injections to infants at 2, 4 and 6 months of age. Vaccine. 2003 Sep 8;21 (25-26):3593-600.

**Figure 1****:** Results from individual clinical trials with combined DTPa3- and DTPa5-based Hib-TT vaccines. Proportion of subjects with anti-PRP antibody concentrations ≥0.15 µg/ml after 3-dose primary vaccination.
*No data available

**Figure 2****:** Results from individual clinical trials with combined DTPa3- and DTPa5-based Hib-TT vaccines. Anti-PRP antibody GMCs (µg/ml) after 3-dose primary vaccination.

Lowest and highest GMC values presented from [69]

Data for Figures 1 and 2 adapted from [60, 63, 64, 65, 66, 68, 69, 70, 72, 73].

**Table 1: Efficacy and effectiveness studies of Hib conjugate vaccines with serum antibody responses in infants after primary immunization**

| **Vaccine** | **Country** | **Schedule** | **N** | **Anti-PRP µg/ml (Primary vaccination)** | | | **Efficacy % (95%CI)** | |
|---|---|---|---|---|---|---|---|---|
| **Design (control)** | **(year)** | **Primary (booster)** | | **% ≥ 0.15** | **% ≥ 1.0** | **GMC** | **Primary** | **Booster** |
| **PRP-D** | | | | | | | | |
| **RC P (placebo)** | **US (Alaska) 1984-88 [34]** | **2-4-6** | **2102** | **48 (%>0.1)** | **15** | **0.18** | **35 (-57;73)** | **-** |
| **RCP (unvaccinated )** | **Finland 1985-87 [35]** | **3-4-6 (14-18)** | **11400** | **68** | **40** | **0.53** | **90 (70;96)** | **94 (83;98)** |
| **RH** | **Finland 1988-91 [36]** | **4-6 (14-18)** | **60500** | **73** | **32** | **0.63** | **87 (69;96)** | **100 (88;100)** |

| **Hib-CRM197** | | | | | | | | |
|---|---|---|---|---|---|---|---|---|
| **CP (unvaccinated )** | **US 1988-90 [37]** | **2-4-6** | **51480** | **100** | **97** | **18.9** | **100 (68;100)** | **-** |
| **RH** | **Finland 1988-91 [36]** | **4-6 (14-18)** | **56500** | **100** | **78** | **4.32** | **95 (76;99)** | **100 (87;100)** |

| **PRP-OMP** | | | | | | | | |
|---|---|---|---|---|---|---|---|---|
| **RCP (placebo)** | **US (Navajo) 1988-90 [38]** | **2-4** | **735** | **91** | **59** | **1.35** | **95 (72;99)** | **-** |
| **R- randomized, C- controlled, P- prospective, H- Historical controls, N: number enrolled, GMC - geometric mean anti-PRP antibody concentration.** | | | | | | | | |

**Table 2: Effect of IPV on anti-PRP antibody concentrations one month following primary vaccination with Hib-TT (previously unpublished results)**

| **Vaccine (no IPV)** | **N** | **≥0.15 µg/ml %** | **≥1.0 µg/ml %** | **GMC** | **95% CI** | **Vaccine (mixed IPV)** | **N** | **≥0.15 µg/ml %** | **≥1.0 µg/ml %** | **GMC** | **95% CI** |
|---|---|---|---|---|---|---|---|---|---|---|---|
| **Primary vaccination at 3, 4 and 5 months of age in Germany** | | | | | | | | | | | |
| **DTPa3-HBV-Hib + OPV** | **48** | **93.8** | **45.8** | **1.108*** | **0.726-1.690** | **DTPa3-HBV-IPV-Hib** | **46** | **100** | **82.6** | **2.185*** | **1.587-3.007** |
| **DTPa3-Hib+HBV + OPV** | **40** | **97.5** | **72.5** | **1.817** | **1.211-2.727** | **DTPa3-IPV-Hib+HBV** | **38** | **100** | **78.9** | **2.797** | **1.968-3.973** |

| **Primary vaccination at 3 and 5 months of age in Sweden [77]** | | | | | | | | | | | |
|---|---|---|---|---|---|---|---|---|---|---|---|
| **DT/Hib + IPV** | **92** | **83** | **48†** | **0.8**** | **-** | **DT/Hib/IPV** | **91** | **90** | **65†** | **2.0**** | **-** |

| | | | | | | | | | | | |
|---|---|---|---|---|---|---|---|---|---|---|---|
| **N = number of subjects tested, %= number/percentage of subjects with concentrations above the cut-off** **95% CI = 95% confidence intervals, lower and upper limit, statistically significant difference between groups: *95% CI for the GMC ratio between groups does not include 1 (0.51 [0.30 ; 0.86]), ** P<0.01 unpaired t-test, † P<0.01 chi-square test** | | | | | | | | | | | |

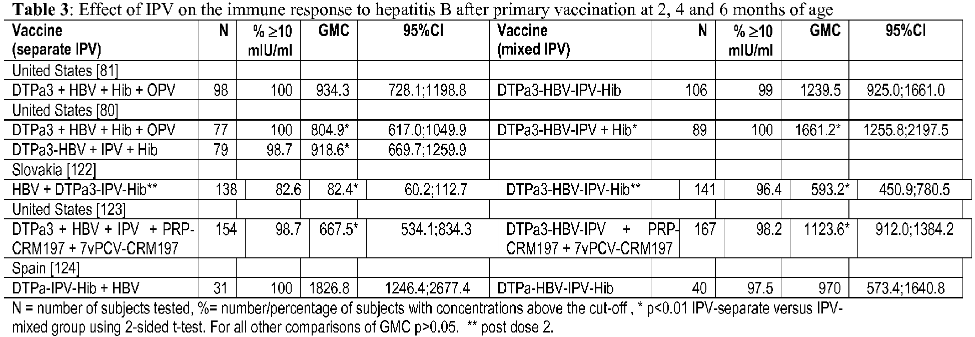

**Table 4 Geometric mean avidity index (GMAI) of anti-PRP IgG antibodies in three clinical trials (adapted from [53])**

| **Study** | **Group** | **N** | **GMAI (95% CI)** | **† P=** |
|---|---|---|---|---|
| ***Study 1: primary vaccination at 2-4-6 months with booster at 15-18 months*** | | | | |
| **Post primary** | **DTPa3 + Hib** | **33** | **0.094 (0.092-0.096)** | **1** |
| | **DTPa3-Hib** | **45** | **0.094 (0.094-0.095)** | |
| **Pre booster** | **DTPa3 + Hib** | **21** | **0.292 (0.221-0.387)** | **0.0189** |
| | **DTPa3-Hib** | **18** | **0.183 (0.138-0.242)** | |
| **Post booster** | **DTPa3 + Hib** | **31** | **0.252 (0.202-0.313)** | **0.0000** |
| | **DTPa3-Hib** | **59** | **0.126 (0.115-0.138)** | |

| ***Study 2: primary vaccination at 3-4-5 months with booster at 15-27 months*** | | | | |
|---|---|---|---|---|
| **Post primary** | **DTPa3 + HBV + OPV + Hib*** | **40** | **0.126 (0.106-0.150)** | **0.0794** |
| | **DTPa3-HBV-IPV-Hib** | **40** | **0.105 (0.094-0.118)** | |
| **Pre booster** | **DTPa3 + Hib*** | **21** | **0.192 (0.143-0.257)** | **0.8164** |
| | **DTPa3-Hib** | **23** | **0.183 (0.135-0.250)** | |
| **Post booster** | **DTPa3 + Hib*** | **34** | **0.189 (0.145-0.246)** | **0.8067** |
| | **DTPa3-Hib** | **37** | **0.182 (0.136-0.193)** | |

| ***Study 3: primary vaccination at 6-10-14 weeks with booster at 15-19 months*** | | | | |
|---|---|---|---|---|
| **Post primary** | **DTPw-HBV-Hib_{2.5}** | **25** | **0.085 (0.070-0.103)** | **0.2398** |
| | **DTPw-HBV-Hib** | **25** | **0.069 (0.051-0.094)** | |
| **Post booster** | **DTPw-HBV-Hib_{2.5}** | **25** | **0.207 (0.167-0.258)** | **0.0744** |
| | **DTPw-HBV-Hib** | **25** | **0.283 (0.214-0.374)** | |

| | | | | |
|---|---|---|---|---|
| **Blood samples collected one month after the 3-dose primary series and before and 4-6 weeks after the booster dose N: Number of subjects tested; NS: no statistical difference; 95% CI: 95% confidence intervals; GMAI: geometric mean avidity index; *OmniHIB™; HBV - hepatitis B vaccine DTPw-HBV/ Hib2.5 : Hib vaccine containing 2.5µg PRP conjugated to TT. Study 1: Germany 20-08-1993 to 28-08-1995. Study 2 US 24-07-1996 to 28-04-1998. Study 3 Myanmar 16-01-1998 to 11-10-1999.† two-sided p-value using one-way ANOVA test to show a difference between groups** | | | | |

**Table 5: Effect of IPV in reducing interference on the Hib response, following co-administration of MenC-CRM197 and Hib-TT for primary vaccination at 2, 3 and 4 months of age**

| | **Germany** | | | | **UK** | | | |
|---|---|---|---|---|---|---|---|---|
| **Vaccine** | **N** | **≥0.15µg/ml %** | **GMC** (95%CI) | **Ref** | **N** | **≥0.15µg/ml %** | **GMC** (95%CI) | **Ref** |
| **DTPa3-Hib** | 387 | 96.4 | 2.022 (1.766;2.316) | [60] | 103 | 96.0 | 1.56 (1.19;2.04) | * |
| **DTPa3-Hib + MenC-CRM197†** | No data | | | | 40 | 82.5 | 0.542 (0.343;0.858) | ** |
| **DTPa3-HBV-IPV-Hib** | 145 | 99.3 | 2.62 (2.1;3.2) | [66] | | No data | | |
| **DTPa3-HBV-IPV-Hib + MenC-CRM197†** | 105 | 97.1 | 2.78 (2.11;3.65) | [67] | | No data | | |

| | | | | | | | | |
|---|---|---|---|---|---|---|---|---|
| *Dr IG Jones previously unpublished data, ** previously unpublished data, †- *Meningitec*™ | | | | | | | | |

### Example 2

A study was performed to investigate the immune response to PRP in Hib upon coadministration of Infanrix-Hexa with different pneumococcal conjugate vaccines containing different amounts of TT as detailed in Table 6 below.
- Experimental design: single-blind, randomized, multi-centre study with 11 parallel groups (60 subjects per group); all groups received a three-dose primary vaccination course.
- Nine groups each received a different formulation of the candidate 11Pn-PD-DiT vaccines with doses of each polysaccharide as shown in Table 6. In addition, one group received the first generation 11Pn-PD vaccine (as comparator) and one group received Prevenar® (as control).
- All groups also received a concomitant injection of DTPa-HBV-IPV/Hib vaccine.
- Blinding: single-blind, however the nine 11Pn-PD-DiT groups were double-blind
- Comparator: 11Pn-PD + DTPa-HBV-IPV/Hib
- Control: Prevenar + DTPa-HBV-IPV/Hib
- Vaccination schedule: three-dose primary vaccination course was given to infants, according to a 2-3-4 month schedule. The first dose of the three-dose vaccination course was given between 8 and 16 weeks (56-118 days) of age, with allowable intervals between the primary vaccination doses of 28-42 days.
- Eight-day follow-up of local and general solicited symptoms and 31-day follow-up for unsolicited adverse events after each vaccine dose. Serious adverse event were recorded throughout the whole study period.
- Two blood samples:
   - Immediately before the 1^{st} dose (4 mL was taken).
   - 1 month after the 3^{rd} dose (4 mL was taken).
- Duration of the study: approximately 6 months with a 3-month enrolment period. For each subject the duration of the study was approximately 3 months.

**Table 6: Dosage of polysaccharide and protein carrier for each serotype in 11Pn-PD-DiT formulations and the lot N°**

| | **6B PS dose** | **18C PS dose** | **23F PS dose** | **19F PS dose** | **1, 3, 4, 5, 7F, 9V and 14 PS dose** | **Free PD** | **Total dose of TT** | **Number of conjugates on TT** | **Total dose of TT in 11Pn-PD-DiT** |
|---|---|---|---|---|---|---|---|---|---|
| Form A | 1 µg TT_{AH} | 3 µg DT_{AH} | 1 µg PD | 3 µg DT | 1 µg PD | - | 63 µg | 1 | 3 µg |
| Form B | 1 µg TT_{AH} | 1 µg TT_{AH} | 1 µg PD | 3 µg DT | 1 µg PD | - | 66 µg | 2 | 6 µg |
| Form C | 1 µg TT_{AH} | 3 µg DT_{AH} | 1 µg TT_{AH} | 3 µg DT | 1 µg PD | - | 66 µg | 2 | 6 µg |
| Form D | 1 µg TT_{AH} | 1 µg TT_{AH} | 1 µg TT_{AH} | 3 µg DT | 1 µg PD | - | 69 µg | 3 | 9 µg |
| Form E | 3 µg TT_{AH} | 1 µg TT_{AH} | 1 µg TT_{AH} | 3 µg DT | 1 µg PD | - | 75 µg | 3 | 15 µg |
| Form F | 1 µg TT_{AH} | 3 µg TT_{AH} | 1 µg TT_{AH} | 3 µg DT | 1 µg PD | - | 75 µg | 3 | 15 µg |
| Form G | 1 µg TT_{AH} | 1 µg TT_{AH} | 3 µg TT_{AH} | 3 µg DT | 1 µg PD | - | 75 µg | 3 | 15 µg |
| Form H | 3 µg TT_{AH} | 1 µg TT_{AH} | 3 µg TT_{AH} | 3 µg DT | 1 µg PD | - | 81 µg | 3 | 21 µg |
| Form I | 1 µg TT_{AH} | 1 µg TT_{AH} | 1 µg TT_{AH} | 3 µg DT | 1 µg PD | 5 µg | 69 µg | 3 | 9 µg |

| | | | | | | | | | |
|---|---|---|---|---|---|---|---|---|---|
| Notes: TT_{AH}: tetanus toxoid with AH spacer, DT_{AH}: diphtheria toxoid with AH spacer, PD: H. influenzae protein D; AH: adipic dihydrazine | | | | | | | | | |

Table 7 shows the seroprotection rates and GMCs for antibodies against the Hib polysaccharide PRP antigen, one month post-vaccination dose III. One month after the third dose of vaccine, all subjects in all groups, with the exception of 1 subject in the Prevenar® (Wyeth) group, reached seroprotective antibody concentrations ≥ 0.15 µg/ml, and at least 83.9% of the subjects receiving the 11Pn-PD-DiT formulations reached a seroprotective antibody concentration ≥ 1 µg/ml. The anti-PRP GMC's observed for the 11Pn-PD-DiT formulations are higher than those observed for the 11Pn-PD (all 11 polysaccharides conjugated to Protein D) and Prevenar® groups.

**Table 7 Seroprotection rates and GMCs for ANTI-PRP antibodies (Total vaccinated cohort)**

| | | | | **≥ 0.15 µg/ml** | | | | ≥ **1 µg/ml** | | | | **GMC** | | |
|---|---|---|---|---|---|---|---|---|---|---|---|---|---|---|
| | | | | | | **95% CI** | | | | **95% CI** | | | **95% CI** | |
| **Antibody** | **Group** | **Timing** | **N** | **n** | **%** | **LL** | **UL** | **n** | **%** | **LL** | **UL** | **value** | **LL** | **UL** |
| ANTI-PRP | 11Pn-PD | PIII(M3) | 58 | 58 | 100 | 93.8 | 100 | 40 | 69.0 | 55.5 | 80.5 | 1.943 | 1.417 | 2.665 |
| | DiT F_A | PIII(M3) | 57 | 57 | 100 | 93.7 | 100 | 48 | 84.2 | 72.1 | 92.5 | 3.811 | 2.793 | 5.200 |
| | DiT F_B | PIII(M3) | 64 | 64 | 100 | 94.4 | 100 | 58 | 90.6 | 80.7 | 96.5 | 4.010 | 3.051 | 5.270 |
| | DiT F_C | PIII(M3) | 58 | 58 | 100 | 93.8 | 100 | 52 | 89.7 | 78.8 | 96.1 | 4.082 | 3.050 | 5.465 |
| | DiT F_D | PIII(M3) | 60 | 60 | 100 | 94.0 | 100 | 51 | 85.0 | 73.4 | 92.9 | 4.167 | 3.035 | 5.721 |
| | DiT F_E | PIII(M3) | 56 | 56 | 100 | 93.6 | 100 | 52 | 92.9 | 82.7 | 98.0 | 5.352 | 3.954 | 7.244 |
| | DiT F_F | PIII(M3) | 62 | 62 | 100 | 94.2 | 100 | 52 | 83.9 | 72.3 | 92.0 | 3.989 | 2.905 | 5.478 |
| | DiT F_G | PIII(M3) | 58 | 58 | 100 | 93.8 | 100 | 50 | 86.2 | 74.6 | 93.9 | 4.660 | 3.324 | 6.535 |
| | DiT F_H | PIII(M3) | 62 | 62 | 100 | 94.2 | 100 | 53 | 85.5 | 74.2 | 93.1 | 4.030 | 3.033 | 5.354 |
| | DiT F_I | PIII(M3) | 65 | 65 | 100 | 94.5 | 100 | 61 | 93.8 | 85.0 | 98.3 | 5.679 | 4.223 | 7.638 |
| | Prevenar | PIII(M3) | 60 | 59 | 98.3 | 91.1 | 100 | 45 | 75.0 | 62.1 | 85.3 | 2.145 | 1.546 | 2.975 |

| | | | | | | | | | | | | | | |
|---|---|---|---|---|---|---|---|---|---|---|---|---|---|---|
| GMC = geometric mean antibody concentration calculated on all subjects N = number of subjects with available results n/% = number/percentage of subjects with concentration within the specified range 95% CI = 95% confidence interval; LL = Lower Limit, UL = Upper Limit MIN/MAX = Minimum/Maximum PIII(M3) = One month after third vaccine dose | | | | | | | | | | | | | | |

Previously, the 11 valent vaccine described in EP983087 where 7 saccharides were conjugated to TT performed disappointingly when given at the same time as DTPa (GAVI Immunisation Focus, March 2002, page 4).

The present inventors have shown there is a correlation of increased anti-PRP GMC and increased TT in the pneumococcal conjugate vaccine until 3-4 conjugates are on TT, when GMC starts to drop. It is therefore clear that incorporation of small coadministered amount of TT leads to improved Hib PRP-TT immune responses.

### Example 3

Randomized, phase II, double blind, controlled study to assess the feasibility of a birth dose of GlaxoSmithKline (GSK) Biologicals' acellular pertussis vaccine (Pa) administered soon after birth, followed by 3-dose primary vaccination with GSK Biologicals' Infanrix hexa™, in accelerating the development of an immune response against pertussis. Primary vaccination is followed in the second year of life by a booster dose of Infanrix hexa™.

**Study design:** Double-blind, randomized (1:1), self-contained single center study conducted in Germany with 2 parallel groups:
- The ***Pa at birth Group*** received a dose of tricomponent acellular pertussis (Pa) vaccine at birth (comprising 25µg pertussis toxoid (PT), 25µg filamentous haemagglutinin (FHA) and 8µg pertactin (PRN))
- The ***Hep B at birth Group*** received a dose of hepatitis B vaccine at birth At 2, 4 and 6 months of age, both groups received GSK Biologicals' Infanrix hexa™ (DTPa-HBV-IPV/Hib) vaccine.

A total of four blood samples were drawn at the following time points in the study: prior to the birth dose of Pa or HBV (pre-dose 1), one month after the first dose, one month after the second dose and one month after the third dose of DTPa-HBV-IPV/Hib vaccine (respectively, post-hexa dose 1, post- hexa dose 2 and post- hexa dose 3).

**Note:** In this study a total of four study vaccine doses were administered (birth dose + 3 doses of Infanrix hexa™). For the sake of clarity with respect to the vaccine doses, the birth dose is referred to as Dose 1, and the post-vaccination time points after vaccination with Infanrix hexa™ are referred to as post-hexa dose 1 post-hexa dose 2 and post-hexa dose 3.

| **Number of subjects:** | **Pa at birth Group** | **HepB at birth Group** | **Total** |
|---|---|---|---|
| *Planned* | 60 | 60 | 120 |
| *Enrolled & vaccinated (=Total vaccinated cohort = According-to-protocol (ATP) cohort for safety)* | 60 | 61 | 121 |
| *ATP cohort for immunogenicity:* | 55 | 57 | 112 |
| *Completed* | 54 | 56 | 110 |
| There were no study withdrawals due to adverse events or serious adverse events | | | |

An objective of this exploratory study was to assess the immunogenicity and safety of a dose of a Pa vaccine administered soon after birth, which included to explore the immunogenicity of a birth dose of Pa followed by three doses of Infanrix hexa™, compared to a routine three dose schedule of Infanrix hexa™, starting at 2 months of age, in terms of all antigens at each time point a serological result was available.

### Immunogenicity results

Total antibodies to the Hib polysaccharide PRP were measured by ELISA. The cut-off of the test was 0.15 µg/ml.

### Anti-PRP antibody response

The seroprotection rates and the GMCs for anti-PRP antibodies are presented in Table 8. One month post-hexa dose 3,

Seroprotective levels (≥ 0.15µg/ml) of anti-PRP antibodies were observed in 88.7% of the subjects in the Pa at birth Group and 98.2% of subjects in the HepB at birth Group.

At least 49.1% of subjects in each group had anti-PRP antibody concentrations ≥ 1 µg/ml.

**Table 8 Seroprotection rates and GMCs for anti-PRP antibodies (ATP cohort for immunogenicity)**

| | | | **≥ 0.15 µg/ml** | | | | **≥ 1 µg/ml** | | | | **GMC** | | |
|---|---|---|---|---|---|---|---|---|---|---|---|---|---|
| | | | | | **95% CI** | | | | **95% CI** | | | **95% CI** | |
| **Group** | **Timing** | **N** | **n** | **%** | **LL** | **UL** | **n** | **%** | **LL** | **UL** | **µg/ml** | **LL** | **UL** |
| **Pa at birth** | PIV(M7) | 53 | 47 | 88.7 | 77.0 | 95.7 | 26 | 49.1 | 35.1 | 63.2 | 0.942 | 0.632 | 1.403 |
| **HepB at birth** | PIV(M7) | 55 | 54 | 98.2 | 90.3 | 100 | 38 | 69.1 | 55.2 | 80.9 | 2.353 | 1.585 | 3.493 |

| | | | | | | | | | | | | | |
|---|---|---|---|---|---|---|---|---|---|---|---|---|---|
| Pa at birth Group: received acellular Pa vaccine at birth and Infanrix hexa™ at 2, 4, 6 months of age ™ HepB at birth Group: received Hepatitis B vaccine at birth and Infanrix hexa™ at 2, 4, 6 months of age GMC = geometric mean antibody concentration, calculated for all subjects. Antibody concentrations below the cut-off of the assays were given an arbitrary value of one half the cut-off for the purpose of calculating the GMC N = number of subjects with available results n/% = number/percentage of subjects with concentration within the specified range 95% CI = 95% confidence interval; LL = Lower Limit, UL = Upper Limit PIV(M7) = Blood sample taken one month after the third dose of Infanrix hexa™ | | | | | | | | | | | | | |

Differences in anti-PRP seroprotection rates (≥ 0.15 µg/ml and ≥ 1 µg/ml) between the Pa at birth Group and the HepB at birth Group with their standardized asymptotic 95% CIs one month after the first and second dose of Infanrix hexa™ for the ≥ 0.15 µg/ml cut-off are presented in Table 9 and in Table 10 for the ≥ 1 µg/ml cut-off.

No significant differences were observed between the Pa at birth Group and the HepB at birth Group in terms of seroprotection rate for anti-PRP antibodies (≥ 0.15 µg/ml) at the post-hexa dose 1 or 2 time points (Table 9). The percentage of subjects with anti-PRP antibodies ≥ 1 µg/ml was statistically significantly lower in the Pa at birth Group than in the HBV at birth Group at post-hexa dose 2 (Table 10).

**Table 9 Differences in seroprotection rates for anti-PRP antibodies (≥ 0.15 µg/ml) between the Pa at birth Group and the HepB at birth Group with their standardized asymptotic 95% CIs one month after the first and second dose of Infanrix hexa (ATP cohort for immunogenicity)**

| | | | | | | **Difference in anti-PRP seroprotection rates (Group 2 minus Group 1)** | | | | |
|---|---|---|---|---|---|---|---|---|---|---|
| | | | | | | | | **95% CI** | | |
| **Group 1** | **N** | **%** | **Group 2** | **N** | **%** | **Difference** | **%** | **LL** | **UL** | **P-value** |
| **At PII(M3)** | | | | | | | | | | |
| **Pa at birth** | 53 | 39.6 | **HepB at birth** | 56 | 46.4 | **HepB at birth - Pa at birth** | 6.81 | -11.70 | 24.81 | 0.562 |

| **At PIII(M5)** | | | | | | | | | | |
|---|---|---|---|---|---|---|---|---|---|---|
| **Pa at birth** | 51 | 70.6 | **HepB at birth** | 52 | 80.8 | **HepB at birth - Pa at birth** | 10.18 | -6.48 | 26.60 | 0.257 |

| | | | | | | | | | | |
|---|---|---|---|---|---|---|---|---|---|---|
| Pa at birth Group: received acellular Pa vaccine at birth and Infanrix hexa™ at 2, 4, 6 months of age ™ HepB at birth Group: received Hepatitis B vaccine at birth and Infanrix hexa™ at 2, 4, 6 months of age PII(M3) = Blood sample taken one month after the first dose of Infanrix hexa™ PIII(M5) = Blood sample taken one month after the second dose of Infanrix hexa™ N = number of subjects with available results % = percentage of subjects with anti-PRP antibody concentrations ≥ 0.15 µg/ml 95% CI = 95% Standardized asymptotic confidence interval; LL = lower limit, UL = upper limit p-value = based on Two-sided Fisher's Exact Test | | | | | | | | | | |

**Table 10 Differences in seroprotection rates for anti-PRP antibodies (≥ 1 µg/ml) between the Pa at birth Group and the HepB at birth Group with their standardized asymptotic 95% CIs one month after the first and second dose of Infanrix hexa (ATP cohort for immunogenicity)**

| | | | | | | **Difference in anti-PRP seroprotection rates (Group 2 minus Group 1)** | | | | |
|---|---|---|---|---|---|---|---|---|---|---|
| | | | | | | | | **95 % CI** | | |
| **Group 1** | **N** | **%** | **Group 2** | **N** | **%** | **Difference** | **%** | **LL** | **UL** | **P-value** |
| **At PII(M3)** | | | | | | | | | | |
| **Pa at birth** | 53 | 5.7 | **HepB at birth** | 56 | 10.7 | **HepB at birth - Pa at birth** | 5.05 | -6.14 | 16.69 | 0.490 |

| **At PIII(M5)** | | | | | | | | | | |
|---|---|---|---|---|---|---|---|---|---|---|
| **Pa at birth** | 51 | 15.7 | **HepB at birth** | 52 | 40.4 | **HepB at birth - Pa at birth** | 24.70 | 7.54 | 40.78 | 0.008 |

| | | | | | | | | | | |
|---|---|---|---|---|---|---|---|---|---|---|
| Pa at birth Group: received acellular Pa vaccine at birth and Infanrix hexa™ at 2, 4, 6 months of age ™ HepB at birth Group: received Hepatitis B vaccine at birth and Infanrix hexa™ at 2, 4, 6 months of age PII(M3) = Blood sample taken one month after the first dose of Infanrix hexa™ PIII(M5) = Blood sample taken one month after the second dose of Infanrix hexa™ N = number of subjects with available results % = percentage of subjects with anti-PRP antibody concentrations ≥ 1 µg/ml 95% CI = 95% Standardized asymptotic confidence interval; LL = lower limit, UL = upper limit p-value = based on Two-sided Fisher's Exact Test | | | | | | | | | | |

The GMC ratios one month after the first and second dose of Infanrix hexa™ for the Pa at birth Group and the HepB at birth Group with 95% CI are presented in Table 11.

No significant differences were observed between the Pa at birth Group and the HepB at birth Group for GMCs of anti-PRP antibodies at post-hexa dose 1.

At the post-hexa dose 2, GMCs of antibodies against PRP were significantly lower in the Pa at birth Group than in the HBV at birth Group.

**Table 11 Anti-PRP GMC ratios one month after the first and second dose of Infanrix hexa for the Pa at birth Group and the HepB at birth Group with 95% CIs (ATP cohort for immunogenicity)**

| | | | | | | **GMC ratio** | | | |
|---|---|---|---|---|---|---|---|---|---|
| | | | | | | | | **95% CI** | |
| **Group** | **N** | **GMC** | **Group** | **N** | **GMC** | **Ratio** | **Value** | **LL** | **UL** |
| **At PII(M3)** | | | | | | | | | |
| **Pa at birth** | 53 | 0.146 | **HepB at birth** | 56 | 0.183 | **Pa at birth / HepB at birth** | 0.8 | 0.5 | 1.2 |

| **At PIII(M5)** | | | | | | | | | |
|---|---|---|---|---|---|---|---|---|---|
| **Pa at birth** | 51 | 0.304 | **HepB at birth** | 52 | 0.771 | **Pa at birth / HepB at birth** | 0.4 | 0.2 | 0.7 |

| | | | | | | | | | |
|---|---|---|---|---|---|---|---|---|---|
| Pa at birth Group: received acellular Pa vaccine at birth and Infanrix hexa™ at 2, 4, 6 months of age ™ HepB at birth Group: received Hepatitis B vaccine at birth and Infanrix hexa™ at 2, 4, 6 months of age PII(M3) = Blood sample taken one month after the first dose of Infanrix hexa™ PIII(M5) = Blood sample taken one month after the second dose of Infanrix hexa™ N = number of subjects with available results GMC = geometric mean antibody concentration calculated on all subjects 95% CI = 95% confidence interval for the GMC ratio (ANOVA model- pooled variance); LL = lower limit, UL = upper limit | | | | | | | | | |

The immune response to the Hib component (PRP conjugated to the tetanus toxoid) of the primary vaccine was significantly reduced in the recipients of the Pa vaccine at birth. Also, the anti-tetanus antibody GMC was significantly lower in the Pa at birth Group, although seroprotection rates were identical (100%) in the two groups. The mechanisms underlying this effect are unknown, though it may be due to bystander interference caused by Pa at birth to PRP-TT during primary immunization in combination with Pa. The fact that the birth dose of Pa vaccine and the DTPa-HBV-IPV/Hib vaccine were administered in the same thigh may have played a part.

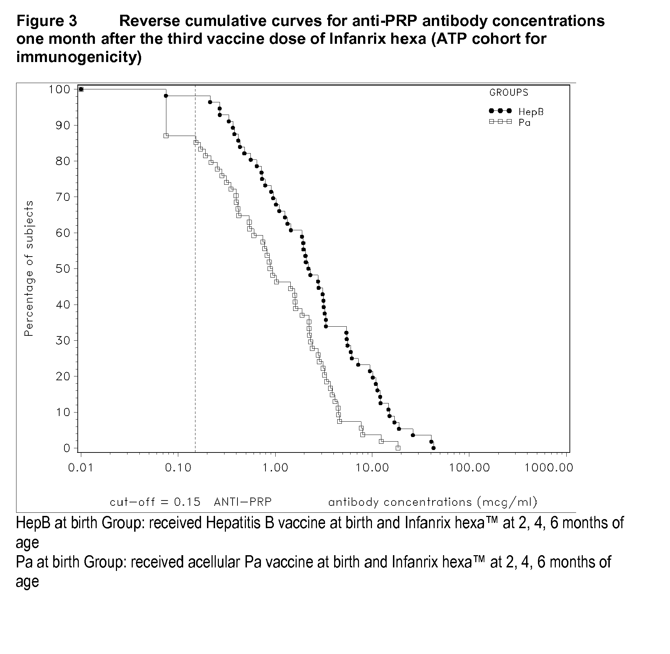

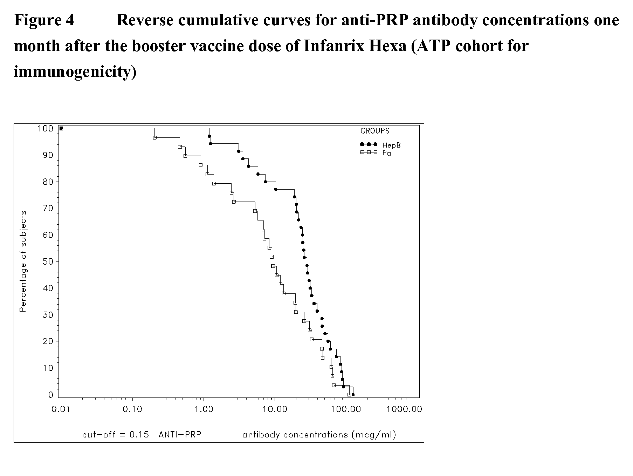

RCCs for anti-PRP antibody concentrations one month after the third dose of Infanrix hexa™ are presented in Figure 3.

### Immunogenicity results from the booster study

**TABLE 12 Seroprotection rates and GMCs for ANTI-PRP antibodies (ATP cohort for immunogenicity)**

| | | | | **>= 0.15 UG/ML** | | | | **>= 1 UG/ML** | | | | **GMC** | | |
|---|---|---|---|---|---|---|---|---|---|---|---|---|---|---|
| | | | | | | **95% CI** | | | | **95% CI** | | | **95% CI** | |
| **Antibody** | **Group** | **Timing** | **N** | **n** | **%** | **LL** | **UL** | **n** | **%** | **LL** | **UL** | **value** | **LL** | **UL** |
| ANTI-PRP | Pa | PIV(M0) | 29 | 17 | 58.6 | 38.9 | 76.5 | 0 | 0.0 | 0.0 | 11.9 | 0.200 | 0.139 | 0.286 |
| | | PV(M1) | 29 | 29 | 100 | 88.1 | 100 | 25 | 86.2 | 68.3 | 96.1 | 8.400 | 4.473 | 15.775 |
| | HepB | PIV(M0) | 33 | 25 | 75.8 | 57.7 | 88.9 | 9 | 27.3 | 13.3 | 45.5 | 0.448 | 0.274 | 0.732 |
| | | PV(M1) | 35 | 35 | 100 | 90.0 | 100 | 35 | 100 | 90.0 | 100 | 22.911 | 15.287 | 34.336 |

| | | | | | | | | | | | | | | |
|---|---|---|---|---|---|---|---|---|---|---|---|---|---|---|
| 1. Pa = Pa at birth and DTPa-HBV-IPV/Hib at 2-4-6 months and booster 2. HepB = HBV at birth and DTPa-HBV-IPV/Hib at 2-4-6 months and booster 3. seroprotection = ANTI-PRP antibody concentration >= 0.15 UG/ML 4. GMC = geometric mean antibody concentration calculated on all subjects 5. N = number of subjects with available results 6. n/% = number/percentage of subjects with concentration within the specified range 7. 95% CI = 95% confidence interval; LL = Lower Limit, UL = Upper Limit 8. PIV(M0) = Pre-booster 9. PV(M1) = One month post-booster | | | | | | | | | | | | | | |

**TABLE 13 Difference between groups in ANTI-PRP seroprotection rate at Pre-Booster (ATP cohort for immunogenicity)**

| | | | | | | **Difference in seroprotection rate (Group 2 minus Group 1)** | | | | |
|---|---|---|---|---|---|---|---|---|---|---|
| | | | | | | | | **95% CI** | | |
| **Group 1** | **N** | **%** | **Group 2** | **N** | **%** | **Difference** | **%** | **LL** | **UL** | **P-value** |
| Pa | 29 | 58.6 | HepB | 33 | 75.8 | HepB - Pa | 17.14 | -6.17 | 39.26 | 0.181 |

| | | | | | | | | | | |
|---|---|---|---|---|---|---|---|---|---|---|
| 1. Pa = Pa at birth and DTPa-HBV-IPV/Hib at 2-4-6 months and booster 2. HepB = HBV at birth and DTPa-HBV-IPV/Hib at 2-4-6 months and booster 3. N = number of subjects with available results 4. % = percentage of subjects with ANTI-PRP concentration >= 0.15 UG/ML 5. 95% CI = 95% Standardized asymptotic confidence interval; LL = lower limit, UL = upper limit 6. P-value = 2-sided Fisher Exact Test | | | | | | | | | | |

**TABLE 14 Difference between groups in ANTI-PRP antibody concentration >=1UG/ML at Pre-Booster (ATP cohort for immunogenicity)**

| | | | | | | **Difference in ANTI-PRP antibody concentration >=1UG/ML (Group 2 minus Group 1)** | | | | |
|---|---|---|---|---|---|---|---|---|---|---|
| | | | | | | | | **95% CI** | | |
| **Group 1** | **N** | **%** | **Group 2** | **N** | **%** | **Difference** | **%** | **LL** | **UL** | **P-value** |
| Pa | 29 | 0.0 | HepB | 33 | 27.3 | HepB - Pa | 27.27 | 14.21 | 44.22 | 0.002 |

| | | | | | | | | | | |
|---|---|---|---|---|---|---|---|---|---|---|
| 1. Pa = Pa at birth and DTPa-HBV-IPV/Hib at 2-4-6 months and booster 2. HepB = HBV at birth and DTPa-HBV-IPV/Hib at 2-4-6 months and booster 3. N = number of subjects with available results 4. % = percentage of subjects with ANTI-PRP concentration >= 1 UG/ML 5. 95% CI = 95% Standardized asymptotic confidence interval; LL = lower limit, UL = upper limit 6. P-value = 2-sided Fisher Exact Test | | | | | | | | | | |

**TABLE 15 Difference between groups in ANTI-PRP seroprotection rate at one month post-booster (ATP cohort for immunogenicity)**

| | | | | | | **Difference in seroprotection rate (Group 2 minus Group 1)** | | | | |
|---|---|---|---|---|---|---|---|---|---|---|
| | | | | | | | | **95% CI** | | |
| **Group 1** | **N** | **%** | **Group 2** | **N** | **%** | **Difference** | **%** | **LL** | **UL** | **P-value** |
| Pa | 29 | 100 | HepB | 35 | 100 | HepB - Pa | 0.00 | -9.89 | 11.70 | . |

| | | | | | | | | | | |
|---|---|---|---|---|---|---|---|---|---|---|
| 1. Pa = Pa at birth and DTPa-HBV-IPV/Hib at 2-4-6 months and booster 2. HepB = HBV at birth and DTPa-HBV-IPV/Hib at 2-4-6 months and booster 3. N = number of subjects with available results 4. % = percentage of subjects with ANTI-PRP concentration >= 0.15 UG/ML 5. 95% CI = 95% Standardized asymptotic confidence interval; LL = lower limit, UL = upper limit 6. P-value = 2-sided Fisher Exact Test | | | | | | | | | | |

**TABLE 16 Difference between groups in ANTI-PRP antibody concentration >=1.0 UG/ML at one month post-booster (ATP cohort for immunogenicity)**

| | | | | | | **Difference in ANTI-PRP antibody concentration >=1UG/ML (Group 2 minus Group 1)** | | | | |
|---|---|---|---|---|---|---|---|---|---|---|
| | | | | | | | | **95% CI** | | |
| **Group 1** | **N** | **%** | **Group 2** | **N** | **%** | **Difference** | **%** | **LL** | **UL** | **P-value** |
| Pa | 29 | 86.2 | HepB | 35 | 100 | HepB - Pa | 13.79 | 3.17 | 30.56 | 0.037 |

| | | | | | | | | | | |
|---|---|---|---|---|---|---|---|---|---|---|
| 1. Pa = Pa at birth and DTPa-HBV-IPV/Hib at 2-4-6 months and booster 2. HepB = HBV at birth and DTPa-HBV-IPV/Hib at 2-4-6 months and booster 3. N = number of subjects with available results 4. % = percentage of subjects with ANTI-PRP concentration >= 1 UG/ML 5. 95% CI = 95% Standardized asymptotic confidence interval; LL = lower limit, UL = upper limit 6. P-value = 2-sided Fisher Exact Test | | | | | | | | | | |

**TABLE 17 Ratios of ANTI-PRP GMCs at one month post-booster (ATP cohort for immunogenicity)**

| | | | | **GMC ratio (Pa / HepB)** | | |
|---|---|---|---|---|---|---|
| **Pa** | | **HepB** | | | **95% CI** | |
| **N** | **GMC** | **N** | **GMC** | **Value** | **LL** | **UL** |
| 29 | 8.400 | 35 | 22.911 | 0.37 | 0.18 | 0.75 |

| | | | | | | |
|---|---|---|---|---|---|---|
| 1. Pa = Pa at birth and DTPa-HBV-IPV/Hib at 2-4-6 months and booster 2. HepB = HBV at birth and DTPa-HBV-IPV/Hib at 2-4-6 months and booster 3. GMC = geometric mean antibody concentration 4. N = Number of subjects with post-vaccination results available 5. 95% CI = 95% confidence interval for the GMC ratio (Anova model - pooled variance); LL = lower limit, UL = upper limit | | | | | | |

### Preferred embodiments

The invention also relates to the following preferred embodiments:
1. A kit comprising at least nine saccharide conjugates, wherein between two and seven saccharide conjugates inclusive are conjugated to CRM carrier protein, said kit being suitable for use in a primary immunisation schedule, said kit comprising:
   a first container comprising
      a) a Hib saccharide conjugate in the presence of CRM, DT or any other DT derivative, but which is not conjugated to the CRM, DT or any other DT derivative;
      b) optionally at least one saccharide conjugate conjugated to CRM; and
      c) optionally at least one other saccharide conjugate not conjugated to CRM, DT or any other DT derivative,
   and a second container comprising
      d) at least one saccharide conjugate conjugated to CRM;
      e) optionally at least one other saccharide conjugate not conjugated to CRM, DT or any other DT derivative,
   and optionally a third container optionally comprising at least one saccharide conjugate, wherein
      f) optionally at least one saccharide conjugate is conjugated to CRM;
      g) optionally at least one saccharide conjugate is not conjugated to CRM, DT or any other DT derivative.
2. The kit of embodiment 1, wherein the average CRM dose per CRM-conjugated saccharide conjugate present in the kit is 1-15µg, 1-10µg, 1-5 µg or 1-3µg.
3. The kit of embodiment 1 or 2, wherein the total CRM load in the kit is less than 35µg, for instance 2-30µg, 5-25µg or 10-20µg.
4. The kit of any of embodiments 1-3, wherein the Hib saccharide conjugate is present at a dose of 1-15, 2-10, 3-8, or 4-6 µg saccharide, for instance approximately 10 µg saccharide.
5. The kit of any of embodiments 1-4, wherein the Hib saccharide conjugate is present at a dose of approximately 2.5µg saccharide.
6. The kit of any of embodiments 1-5, wherein the Hib saccharide conjugate is not adsorbed onto aluminium salts or is adsorbed onto aluminium phosphate.
7. The kit of any of embodiments 1-6, wherein the first or third container further comprises Hepatitis B surface antigen (HB), optionally adsorbed onto aluminium phosphate.
8. The kit of embodiment 7, wherein HB surface antigen is present at a dose of approximately 10µg.
9. A kit comprising at least seven saccharide conjugates, wherein between two and six saccharide conjugates inclusive are conjugated to CRM carrier protein, said kit being suitable for use in a primary immunisation schedule, said kit comprising:
   a first container comprising
      a) HB in the presence of CRM, DT or any other DT derivative, optionally adsorbed onto aluminium phosphate;
      b) optionally at least one saccharide conjugate conjugated to CRM; and
      c) optionally at least one saccharide conjugate not conjugated to CRM, DT or any other DT derivative,
   and a second container comprising
      d) at least one saccharide conjugate conjugated to CRM;
      e) optionally at least one saccharide conjugate not conjugated to CRM, DT or any other DT derivative,
   and optionally a third container optionally comprising at least one saccharide conjugate wherein
      f) optionally at least one saccharide conjugate is conjugated to CRM;
      g) optionally at least one saccharide conjugate is not conjugated to CRM, DT or any other DT derivative.
10. The kit of embodiment 9, wherein the average CRM dose per CRM-conjugated saccharide conjugate present in the kit is 1-9µg, 1-6µg, 1-5µg or 1-3µg.
11. The kit of embodiment 9 or 10, wherein the total CRM load in the kit is less than 20µg, for instance 2-18µg or 5-15µg.
12. The kit of any of embodiments 9-11, wherein the HB surface antigen is present at a dose of approximately 10µg.
13. The kit of any of embodiments 9-12, further comprising Hib saccharide conjugate in the first, second or third container.
14. The kit of embodiment 13, wherein the Hib saccharide conjugate is present at a dose of 1-15, 2-10, 3-8, or 4-6 µg saccharide, for instance approximately 10 µg saccharide.
15. The kit of embodiment 13 or 14, wherein the Hib saccharide conjugate is present at a dose of approximately 2.5µg saccharide.
16. The kit of any of embodiments 13-15, wherein the Hib saccharide conjugate is not adsorbed onto aluminium salts or is adsorbed onto aluminium phosphate.
17. The kit of any of embodiments 1-16, wherein the first container comprises at least one saccharide conjugate conjugated to CRM.
18. A kit comprising at least eight saccharide conjugates conjugated to CRM carrier protein, suitable for use in a primary immunisation schedule, said kit comprising:
   a first container comprising
      a) a sensitive antigen not in the presence of CRM, DT or any other DT derivative; and
      b) optionally at least one saccharide conjugate not conjugated to CRM, DT or any other DT derivative,
   and a second container comprising
      c) at least seven, eight, ten, eleven or thirteen saccharide conjugates conjugated to CRM;
      d) optionally at least one other saccharide conjugate not conjugated to CRM,
         DT or any other DT derivative,
   and optionally a third container optionally comprising at least one saccharide conjugate wherein
      e) optionally at least one saccharide conjugate is conjugated to CRM;
      f) optionally at least one saccharide conjugate is not conjugated to CRM, DT or any other DT derivative.
19. The kit of embodiment 18, wherein the sensitive antigen is Hib saccharide conjugate, not conjugated to CRM, DT or any other DT derivative.
20. The kit of embodiment 19, wherein the Hib saccharide conjugate is present at a dose of 1-15, 2-10, 3-8, or 4-6 µg saccharide, for instance approximately 10 µg saccharide.
21. The kit of embodiment 19 or 20, wherein the Hib saccharide conjugate is present at a dose of approximately 2.5µg saccharide.
22. The kit of any of embodiments 19-21, wherein the Hib saccharide conjugate is not adsorbed onto aluminium salts or is adsorbed onto aluminium phosphate.
23. The kit of any of embodiments 18-22, wherein the sensitive antigen is HB, optionally adsorbed onto aluminium phosphate.
24. The kit of embodiment 23, wherein HB surface antigen is present at a dose of approximately 10µg.
25. The kit of any of embodiments 1-24, wherein the first container comprises at least one saccharide conjugate not conjugated to CRM, DT or any other DT derivative.
26. The kit of any of embodiments 1-25, wherein the second container comprises at least one saccharide conjugate not conjugated to CRM, DT or any other DT derivative.
27. The kit of any of embodiments 1-26 comprising a third container.
28. The kit of embodiment 27, wherein the third container comprises at least one saccharide conjugate which is conjugated to CRM.
29. The kit of embodiment 27 or 28, wherein the third container comprises at least one saccharide conjugate which is not conjugated to CRM, DT or any other DT derivative.
30. The kit of any of embodiments 1-29, wherein the first container further comprises a DTP vaccine (if appropriate), or that the third container is present in the kit and comprises a DTP vaccine.
31. The kit of embodiment 30, wherein DT is present, optionally at a dose between 10-120µg, 50-100µg, 70-100µg or 80-95µg.
32. The kit of embodiment 30 or 31, wherein TT is present, optionally at a dose between 10-60µg, 20-50µg or 30-48µg.
33. The kit of any of embodiments 30-32, wherein the DTP vaccine comprises Pa.
34. The kit of embodiment 33, wherein the Pa comprises PT (or a PT derivative), FHA and pertactin (PRN).
35. The kit of embodiment 33 or 34, wherein PT (or PT derivative) is present in Pa and is at a dose which does not exceed 10µg, 1-9, 1.5-8, 2-6, 2.5-5µg per 0.5 mL dose.
36. The kit of any of embodiments 33-35, wherein FHA is present in Pa and is at a dose which does not exceed 10µg, 1-9, 1.5-8, 2-6, 2.5-5µg per 0.5 mL dose.
37. The kit of any of embodiments 33-36, wherein PRN is present in Pa and is at a dose which does not exceed 6µg, 0.5-6, 0.8-5, 1-4, 2-3µg per 0.5 mL dose.
38. The kit of any of embodiments 33-37, wherein PT is present at a dose of approximately 2.5µg, FHA is present at a dose of approximately 2.5µg and PRN is present at a dose of approximately 0.8µg per 0.5mL dose.
39. The kit of any of embodiments 33-37, wherein PT is present at a dose of approximately 5µg, FHA is present at a dose of approximately 5µg and PRN is present at a dose of approximately 2.5µg per 0.5mL dose.
40. The kit of any of embodiments 30-32, wherein the DTP vaccine comprises Pw.
41. The kit of any of embodiments 1-40, wherein the first or third container further comprises IPV.
42. The kit of embodiment 41, wherein the IPV is present at a dose of approximately 40 D-antigen units of IPV-1, 8 D-antigen units of IPV-2 and 32 D-antigen units of IPV-3.
43. The kit of any of embodiments 1-42, wherein the saccharide conjugate(s) conjugated to CRM in the second container is a pneumococcal capsular saccharide conjugate.
44. The kit of embodiment 43, wherein the second container comprises a 13-valent pneumococcal capsular saccharide conjugate vaccine.
45. The kit of any of embodiments 1-44, wherein the saccharide conjugate(s) conjugated to CRM in the second container is a *Neisseria meningitidis* capsular saccharide conjugate.
46. The kit of any of embodiments 1-45, wherein the third container comprises a DTP vaccine.
47. The kit of embodiment 46, wherein DT is present, optionally at a dose between 10-120µg, 50-100µg, 70-100µg or 80-95µg.
48. The kit of embodiment 46 or 47, wherein TT is present, optionally at a dose between 10-60µg, 20-50µg or 30-48µg.
49. The kit of any of embodiments 46-48, wherein the DTP vaccine comprises Pa.
50. The kit of embodiment 49, wherein the Pa comprises PT (or a PT derivative), FHA and pertactin (PRN).
51. The kit of embodiment 49 or 50, wherein PT (or PT derivative) is present in Pa and is at a dose which does not exceed 10µg, 1-9, 1.5-8, 2-6, 2.5-5µg per 0.5 mL dose.
52. The kit of any of embodiments 49-51, wherein FHA is present in Pa and is at a dose which does not exceed 10µg, 1-9, 1.5-8, 2-6, 2.5-5µg per 0.5 mL dose.
53. The kit of any of embodiments 49-52, wherein PRN is present in Pa and is at a dose which does not exceed 6µg, 0.5-6, 0.8-5, 1-4, 2-3µg per 0.5 mL dose.
54. The kit of any of embodiments 49-53, wherein PT is present at a dose of approximately 2.5µg, FHA is present at a dose of approximately 2.5µg and PRN is present at a dose of approximately 0.8µg per 0.5mL dose.
55. The kit of any of embodiments 49-53, wherein PT is present at a dose of approximately 5µg, FHA is present at a dose of approximately 5µg and PRN is present at a dose of approximately 2.5µg per 0.5mL dose.
56. The kit of any of embodiments 46-48, wherein the DTP vaccine comprises Pw.
57. The kit of any of embodiments 43-56, wherein the third container further comprises IPV.
58. The kit of embodiment 57, wherein the IPV is present at a dose of approximately 40 D-antigen units of IPV-1, 8 D-antigen units of IPV-2 and 32 D-antigen units of IPV-3.
59. The kit of any of embodiments 1-58, wherein the third container comprises one or more *Neisseria meningitidis* capsular saccharide conjugate(s), optionally conjugated to CRM, and/or wherein the first container comprises one or more *Neisseria meningitidis* capsular saccharide conjugate(s), optionally not conjugated to CRM, DT or any other DT derivative.
60. The kit of embodiment 18, wherein the first container comprises Hib-MenC capsular saccharide conjugates, not conjugated to CRM, DT or any other DT derivative, optionally conjugated to TT.
61. The kit of embodiment 60, wherein the second container comprises a 13-valent pneumococcal capsular saccharide conjugate vaccine conjugated to CRM.
62. The kit of embodiment 60 or 61, wherein the third container comprises a DTP vaccine.
63. A kit comprising seven or more saccharide conjugates wherein fewer than seven saccharide conjugates are conjugated to CRM carrier protein, said kit being suitable for use in a primary immunisation schedule, said kit comprising:
   a first container comprising
      a) Hib saccharide conjugate, not conjugated to CRM, DT or any other DT derivative;
      b) optionally at least one saccharide conjugate not conjugated to CRM, DT or any other DT derivative,
   and a second container comprising
   c) fewer than seven saccharides conjugated to CRM,
   and optionally a third container comprising
   d) optionally at least one saccharide conjugate not conjugated to CRM, DT or any other DT derivative.
64. The kit of embodiment 63, wherein the at least one saccharide conjugate in the first container is present and is not conjugated to CRM, DT or any other DT derivative.
65. The kit of any of embodiments 63 or 64 comprising a third container comprising at least one saccharide conjugate not conjugated to CRM, DT or any other DT derivative.
66. The kit of any of embodiments 63-65, wherein in the first container the Hib saccharide conjugate is not in the presence of CRM, DT or any other DT derivative.
67. The kit of any of embodiments 63-65, wherein in the first container the Hib saccharide conjugate is in the presence of CRM, DT or any other DT derivative.
68. The kit of any of embodiments 63-67, wherein the first container comprises a *Neisseria meningitidis* capsular saccharide conjugate, not conjugated to CRM, DT or any other DT derivative.
69. The kit of embodiment 68, wherein the *Neisseria meningitidis* capsular saccharide conjugate in the first container is conjugated to TT.
70. The kit of any of embodiments 63-69, wherein DT is only present in the kit as free DT.
71. The kit of any of embodiments 63-70, wherein the first container further comprises a DTP vaccine, or that the third container is present in the kit and comprises a DTP vaccine.
72. The kit of embodiment 71, wherein DT is present, optionally at a dose between 10-120µg, 50-100µg, 70-100µg or 80-95µg.
73. The kit of embodiment 71 or 72, wherein TT is present, optionally at a dose between 10-60µg, 20-50µg or 30-48µg.
74. The kit of any of embodiments 71-73, wherein the DTP vaccine comprises Pa.
75. The kit of embodiment 74, wherein the Pa comprises PT (or a PT derivative), FHA and pertactin (PRN).
76. The kit of embodiment 74 or 75, wherein PT (or PT derivative) is present in Pa and is at a dose which does not exceed 10µg, 1-9, 1.5-8, 2-6, 2.5-5µg per 0.5 mL dose.
77. The kit of any of embodiments 74-76, wherein FHA is present in Pa and is at a dose which does not exceed 10µg, 1-9, 1.5-8, 2-6, 2.5-5µg per 0.5 mL dose.
78. The kit of any of embodiments 74-77, wherein PRN is present in Pa and is at a dose which does not exceed 6µg, 0.5-6, 0.8-5, 1-4, 2-3µg per 0.5 mL dose.
79. The kit of any of embodiments 74-78, wherein PT is present at a dose of approximately 2.5µg, FHA is present at a dose of approximately 2.5µg and PRN is present at a dose of approximately 0.8µg per 0.5mL dose.
80. The kit of any of embodiments 74-78, wherein PT is present at a dose of approximately 5µg, FHA is present at a dose of approximately 5µg and PRN is present at a dose of approximately 2.5µg per 0.5mL dose.
81. The kit of any of embodiments 71-73, wherein the DTP vaccine comprises Pw.
82. The kit of any of embodiments 63-81, wherein the first or third container further comprises IPV.
83. The kit of embodiment 82, wherein the IPV is present at a dose of approximately 40 D-antigen units of IPV-1, 8 D-antigen units of IPV-2 and 32 D-antigen units of IPV-3.
84. The kit of any of embodiments 63-83, wherein the first or third container further comprises HB, optionally adsorbed onto aluminium phosphate.
85. The kit of embodiment 84, wherein the HB surface antigen is present at a dose of approximately 10µg.
86. The kit of any of embodiments 63-85, comprising a third container.
87. The kit of embodiment 86, wherein the third container does not contain CRM.
88. The kit of embodiments 86 or 87, wherein the third container comprises one or more *Neisseria meningitidis* capsular saccharide conjugate(s), optionally conjugated to TT.
89. The kit of any of embodiments 86-88, wherein the third container further comprises a DTP vaccine.
90. The kit of embodiment 89, wherein DT is present, optionally at a dose between 10-120µg, 50-100µg, 70-100µg or 80-95µg.
91. The kit of embodiment 89 or 90, wherein TT is present, optionally at a dose between 10-60µg, 20-50µg or 30-48µg.
92. The kit of any of embodiments 89-91, wherein the DTP vaccine comprises Pa.
93. The kit of embodiment 92, wherein the Pa comprises PT (or a PT derivative), FHA and pertactin (PRN).
94. The kit of embodiment 92 or 93, wherein PT (or PT derivative) is present in Pa and is at a dose which does not exceed 10µg, 1-9, 1.5-8, 2-6, 2.5-5µg per 0.5 mL dose.
95. The kit of any of embodiments 92-94, wherein FHA is present in Pa and is at a dose which does not exceed 10µg, 1-9, 1.5-8, 2-6, 2.5-5µg per 0.5 mL dose.
96. The kit of any of embodiments 92-95, wherein PRN is present in Pa and is at a dose which does not exceed 6µg, 0.5-6, 0.8-5, 1-4, 2-3µg per 0.5 mL dose.
97. The kit of any of embodiments 92-96, wherein PT is present at a dose of approximately 2.5µg, FHA is present at a dose of approximately 2.5µg and PRN is present at a dose of approximately 0.8µg per 0.5mL dose.
98. The kit of any of embodiments 92-96, wherein PT is present at a dose of approximately 5µg, FHA is present at a dose of approximately 5µg and PRN is present at a dose of approximately 2.5µg per 0.5mL dose.
99. The kit of any of embodiments 89-91, wherein the DTP vaccine comprises Pw.
100. The kit of any of embodiments 86-99, wherein the third container further comprises IPV.
101. The kit of embodiment 100, wherein the IPV is present at a dose of approximately 40 D-antigen units of IPV-1, 8 D-antigen units of IPV-2 and 32 D-antigen units of IPV-3.
102. The kit of any of embodiments 86-101, wherein the third container further comprises HB, optionally adsorbed onto aluminium phosphate.
103. The kit of embodiment 102, wherein HB surface antigen is present at a dose of approximately 10µg.
104. The kit of any of embodiments 63-103, wherein no saccharide conjugates in the kit are conjugated to CRM.
105. The kit of any of embodiments 63-104, wherein 1, 2 or 3 saccharide conjugates in the kit are conjugated to DT, optionally in the second container.
106. The kit of any of embodiments 63-105, wherein 1, 2, 3 or 4 saccharide conjugates in the kit are conjugated to TT, optionally in the second container.
107. The kit of any of embodiments 63-106, comprising Synflorix, optionally in the second container.
108. The kit of any of embodiments 71-107, wherein the DTPa vaccine is Infanrix-Hexa.
109. The kit of any of embodiments 71-107, wherein the DTPa vaccine is Pediacel.
110. A combination vaccine suitable for primary immunisation comprising nine or more saccharide conjugates;
   a) wherein Hib saccharide conjugate is present but is not conjugated to CRM, DT or any other DT derivative;
   b) wherein between two and seven saccharide conjugates inclusive are conjugated to CRM;
   c) wherein one or more other saccharide conjugate(s) is not conjugated to CRM.
111. The combination vaccine of embodiment 110, wherein the average CRM dose per CRM-conjugated saccharide conjugate present in the kit is 1-15µg, 1-10µg, 1-5 µg or 1-3µg.
112. The combination vaccine of embodiment 110 or 111, wherein the total CRM load in the kit is less than 35µg, for instance 2-30µg, 5-25µg or 10-20µg.
113. The combination vaccine of any of embodiments 110-112, further comprising HB, optionally adsorbed onto aluminium phosphate.
114. The combination vaccine of embodiment 113, wherein the HB surface antigen is present at a dose of approximately 10µg.
115. A combination vaccine suitable for primary immunisation comprising seven or more saccharides;
   a) wherein HB is present;
   b) wherein between two and six saccharide conjugates inclusive are conjugated to CRM;
   c) wherein one or more other saccharide conjugate(s) is not conjugated to CRM.
116. The combination vaccine of embodiment 115, wherein the average CRM dose per CRM-conjugated saccharide conjugate present in the kit is 1-9µg, 1-6µg, 1-5µg or 1-3µg.
117. The combination vaccine of embodiment 115 or 116, wherein the total CRM load in the kit is less than 20µg, for instance 2-18µg or 5-15µg.
118. The combination vaccine of any of embodiments 115-117, wherein HB surface antigen is adsorbed onto aluminium phosphate and is optionally present at a dose of approximately 10µg.
119. The combination vaccine of any of embodiments 115-118, further comprising Hib saccharide conjugate.
120. The combination vaccine of any of embodiments 110-119, wherein the Hib saccharide conjugate is present at a dose of 1-15, 2-10, 3-8, or 4-6 µg saccharide, for instance approximately 10 µg saccharide.
121. The combination vaccine of any of embodiments 110-120, wherein the Hib saccharide conjugate is present at a dose of approximately 2.5µg saccharide.
122. The combination vaccine of any of embodiments 110-121, wherein the Hib saccharide conjugate is conjugated to TT.
123. The combination vaccine of any of embodiments 110-122, wherein one, two or three saccharide conjugates other than Hib saccharide conjugate are conjugated to TT.
124. The combination vaccine of any of embodiments 110-123, wherein the Hib saccharide conjugate is not adsorbed onto aluminium salts or is adsorbed onto aluminium phosphate.
125. The combination vaccine of any of embodiments 110-124, wherein the saccharide conjugates conjugated to CRM are pneumococcal capsular saccharide conjugates.
126. The combination vaccine of any of embodiments 110-125, wherein the one or more other saccharide conjugate(s) is a *Neisseria meningitidis* capsular saccharide conjugate.
127. The combination vaccine of any of embodiments 110-126, further comprising a DTP vaccine.
128. The combination vaccine of embodiment 127, wherein DT is present, optionally at a dose between 10-120µg, 50-100µg, 70-100µg or 80-95µg.
129. The combination vaccine of embodiment 127 or 128, wherein TT is present, optionally at a dose between 10-60µg, 20-50µg or 30-48µg.
130. The combination vaccine of any of embodiments 127-129, wherein the DTP vaccine comprises Pa.
131. The combination vaccine of embodiment 130, wherein the Pa comprises PT (or a PT derivative), FHA and pertactin (PRN).
132. The combination vaccine of embodiment 130 or 131, wherein PT (or PT derivative) is present in Pa and is at a dose which does not exceed 10µg, 1-9, 1.5-8, 2-6, 2.5-5µg per 0.5 mL dose.
133. The combination vaccine of any of embodiments 130-132, wherein FHA is present in Pa and is at a dose which does not exceed 10µg, 1-9, 1.5-8, 2-6, 2.5-5µg per 0.5 mL dose.
134. The combination vaccine of any of embodiments 130-133, wherein PRN is present in Pa and is at a dose which does not exceed 6µg, 0.5-6, 0.8-5, 1-4, 2-3µg per 0.5 mL dose.
135. The combination vaccine of any of embodiments 130-134, wherein PT is present at a dose of approximately 2.5µg, FHA is present at a dose of approximately 2.5µg and PRN is present at a dose of approximately 0.8µg per 0.5mL dose.
136. The combination vaccine of any of embodiments 130-134, wherein PT is present at a dose of approximately 5µg, FHA is present at a dose of approximately 5µg and PRN is present at a dose of approximately 2.5µg per 0.5mL dose.
137. The combination vaccine of any of embodiments 127-129, wherein the DTP vaccine comprises Pw.
138. The combination vaccine of any of embodiments 110-137, further comprising IPV.
139. The combination vaccine of embodiment 138, wherein the IPV is present at a dose of approximately 40 D-antigen units of IPV-1, 8 D-antigen units of IPV-2 and 32 D-antigen units of IPV-3.
140. A method of administering the kit or combination vaccine of any of embodiments 1-139 in a primary immunisation schedule.
141. The method of embodiment 140, wherein Pa is administered at birth and DTPa-Hib is administered within the primary immunisation schedule.
142. A method of administering the components of the kit of any of embodiments 1-109, wherein the contents of the fist container is co-administered with the contents of the second container.
143. A method of administering the components of the kit of any of embodiments 1-109 wherein the contents of the first container is administered in a staggered fashion with the contents of the second container.
144. The method of embodiment 142, wherein the contents of the third container is co-administered with the contents of the first and second containers.
145. The method of embodiment 143, wherein the contents of the third container is co-administered with the contents of the first container.
146. The method of embodiment 143, wherein the contents of the third container is co-administered with the contents of the second container.
147. The method of embodiment 143, wherein the contents of the third container is administered in a staggered fashion with the contents of the first and second containers.
148. A method of decreasing bystander interference of CRM on a sensitive antigen in a primary immunisation schedule of a vaccine comprising one or more of the following steps
   a) decreasing the amount of CRM and/or number of conjugates on CRM in the vaccine;
   b) including IPV in the vaccine comprising the sensitive antigen;
   c) including Pw in the vaccine comprising the sensitive antigen;
   d) decreasing DT dose in the vaccine comprising the sensitive antigen;
   e) increasing dose of the sensitive antigen;
   f) if Pa is present in vaccine comprising sensitive antigen, reducing the Pa dose or number of Pa components;
   g) removing CRM from the vaccine comprising the sensitive antigen, or removing CRM entirely from the kit, or removing CRM, DT and DT derivatives from the vaccine comprising the sensitive antigen.
149. The method of embodiment 148, wherein the sensitive antigen is Hib not conjugated to CRM, DT or any other DT derivative or HB.
150. The method of embodiment 148 or 149, comprising the additional step of including additional TT in the vaccine if Hib saccharide conjugate is present and is conjugated to TT.
151. The method of embodiment 150, wherein additional TT is provided in a separate container from the Hib saccharide conjugate, for co-administration.
152. The method of embodiment 150 or 151, wherein additional TT is conjugated to no more than three other saccharides.
153. The method of any of embodiments 148-152, wherein if Pa is present, PT (or PT derivative) is present in Pa and is at a dose which does not exceed 10µg, 1-9, 1.5-8, 2-6, 2.5-5µg per 0.5 mL dose.
154. The method of any of embodiments 148-153, wherein if Pa is present, FHA is present in Pa and is at a dose which does not exceed 10µg, 1-9, 1.5-8, 2-6, 2.5-5µg per 0.5 mL dose.
155. The method of any of embodiments 148-154, wherein if Pa is present, PRN is present in Pa and is at a dose which does not exceed 6µg, 0.5-6, 0.8-5, 1-4, 2-3µg per 0.5 mL dose.
156. The method of any of embodiments 148-155, wherein if Pa is present, PT is present at a dose of approximately 2.5µg, FHA is present at a dose of approximately 2.5µg and PRN is present at a dose of approximately 0.8µg per 0.5mL dose.
157. The method of any of embodiments 148-155, wherein if Pa is present, PT is present at a dose of approximately 5µg, FHA is present at a dose of approximately 5µg and PRN is present at a dose of approximately 2.5µg per 0.5mL dose.
158. The method of any of embodiments 148-157, wherein PT is recombinant.
159. The method of embodiment 158, wherein the dose of PT is reduced.
160. The method of any of embodiments 148-159 wherein additional TT is provided by co-administering Synflorix.
161. The method of any of embodiments 148-160, wherein additional TT is provided by co-administering MenC-TT and Infanrix-hexa.
162. The method of any of embodiments 148-161, wherein IPV is provided by Infanrix-hexa.
163. The method of any of embodiments 148-162, wherein Hib saccharide conjugate is administered separately from DTP-IPV-HB vaccine.
164. A method of decreasing bystander interference on a sensitive antigen when using a kit comprising eight or more saccharide conjugates conjugated to CRM, comprising a first container comprising
   a) a sensitive antigen(s) in the presence of CRM, DT or any other DT derivative;
   and a second container comprising
   b) seven or more saccharide conjugates conjugated to CRM;
   c) optionally at least one other saccharide conjugate not conjugated to CRM, DT or any other DT derivative;
   and optionally a third container optionally comprising at least one saccharide conjugate which is
   d) optionally conjugated to CRM;
   e) optionally not conjugated to CRM
   comprising the step of removing all CRM, DT or any other DT derivative from the container comprising the sensitive antigen or reducing the number of saccharide conjugates conjugated to CRM to no more than seven.
165. A method of immunising against disease caused by *Bordetella pertussis, Clostridium tetani, Corynebacterium diphtheriae,* Hepatitis B virus, *Haemophilus influenzae* type b, *Streptococcus pneumonia* and *Neisseria meningitidis* using the kit or combination vaccine of any of embodiments 1-156, wherein
   a) each antigen in the kit or the combination vaccine is administered 2-3 times in a primary immunisation schedule;
   b) Hib is not conjugated to CRM, DT or any other DT derivative;
   c) there are 7 or more *Streptococcus pneumonia* capsular saccharide antigens conjugates;
   d) the number of *Streptococcus pneumonia* and *Neisseria meningitidis* capsular saccharide antigens conjugated to CRM are fewer than 8.
166. The method of embodiment 165, wherein the *Streptococcus pneumonia* saccharide antigen conjugates are comprised in Synflorix.
167. The method of embodiment 165 or 166, wherein Synflorix is co-administered or administered in a staggered fashion with Infanrix-Hexa and a MenC conjugate containing vaccine.
168. The method of embodiment 165 or 166, wherein Synflorix is co-administered or administered in a staggered fashion with Infanrix-Penta and a Hib-MenC conjugate containing vaccine.
169. The method of embodiment 165 or 166, wherein Synflorix is co-administered or administered in a staggered fashion with an Infanrix Hexa-MenC containing vaccine.
170. A method of immunising against disease caused by *Bordetella pertussis, Clostridium tetani, Corynebacterium diphtheriae,* Hepatitis B virus, *Haemophilus influenzae* type b, *Streptococcus pneumonia* and *Neisseria meningitidis* using the kit or combination vaccine of any of embodiments 1-139, wherein
   a) each antigen in the kit or combination vaccine is administered 2-3 times in a primary immunisation schedule;
   b) the Pa dose or number of Pa components are reduced.
171. The kit, the combination vaccine or the method of embodiments 1-170, wherein CRM is CRM-197.
172. A kit of vaccines for coadministration comprising two or more containers, wherein
   the first container comprises
   a) Hib comprising PRP conjugated to TT
   b) optionally DTP
   c) optionally one or more further antigens
   the second container comprises
   d) a vaccine comprising one or more protein-conjugated bacterial saccharides one or more of which is/are conjugated to TT
   e) optionally one or more further antigens
   and, optionally, a third container comprises
   f) a vaccine comprising TT
   wherein the total amount of TT in the kit not conjugated to Hib is 31µ to 55µg, 35µg to 50µg or 40µg to 45µg.
173. A kit of vaccines for coadministration comprising two or more containers, wherein
   the first container comprises
   a) Hib comprising PRP conjugated to TT
   b) optionally DTP
   c) optionally one or more further antigens
   the second container comprises
   d) a vaccine comprising one or more protein-conjugated bacterial saccharides one or more of which is/are conjugated to TT
   e) optionally one or more further antigens
   and, optionally, a third container comprises
   f) a vaccine comprising TT
   wherein the total amount of TT in the kit not in the first container is 1µg to 25µg, 5µg to 20µg, or 10µg to 15µg.
174. The kit of embodiment 173 which is the kit of embodiment 172.
175. A kit of vaccines for coadministration comprising two or more containers, wherein
   the first container comprises
   a) Hib comprising PRP conjugated to TT
   b) optionally DTP
   c) optionally one or more further antigens
   the second container comprises
   d) a vaccine comprising one or more protein-conjugated bacterial saccharides one or more of which is/are conjugated to TT
   e) optionally one or more further antigens
   and, optionally, a third container comprises
   f) a vaccine comprising TT
   wherein the amount of TT present in the first container, but not conjugated to PRP in Hib, is 20µg to 40µg or 25 to 35µg or around or exactly 30µg.
176. The kit of embodiment 175 which is a kit of embodiments 172-174.
177. A kit of vaccines for coadministration comprising two or more containers, wherein
   the first container comprises
   a) Hib comprising PRP conjugated to TT
   b) optionally DTP
   c) optionally one or more further antigens
   the second container comprises
   d) a vaccine comprising one or more protein-conjugated bacterial saccharides one or more of which is/are conjugated to TT
   e) optionally one or more further antigens
   and, optionally, a third container comprises
   f) a vaccine comprising TT
   wherein the amount of TT conjugated to PRP in Hib is 10µg to 40µg, 15µg to 35µg, 20µg to 30µg or around or exactly 25µg.
178. The kit of embodiment 177 which is a kit of embodiments 172-176.
179. The kit of embodiments 172-178, wherein PRP in Hib is conjugated through an ADH linker to TT.
180. The kit of embodiments 172-176, wherein the number of different protein-conjugated bacterial saccharides in the second container which are conjugated to TT is 1, 2, 3 or 4.
181. The kit of embodiments 172-180, wherein the first container comprises b) DTPa or DTPw.
182. The kit of embodiments 172-181, wherein the first container comprises c) IPV and/or HB.
183. The kit of embodiments 172-180, comprising a third container comprising f) DTPa or DTPw.
184. The kit of embodiment 183, wherein the third container further comprises IPV and/or HB.
185. The kit of embodiments 172-184, wherein the first container comprises c) MenC capsular saccharide conjugated to TT and/or MenY capsular saccharide conjugated to TT.
186. The kit of embodiments 172-184, wherein the third container comprises f) MenC capsular saccharide conjugated to TT and/or MenY capsular saccharide conjugated to TT.
187. The kit of embodiments 172-186, wherein the second container comprises d) a pneumococcal vaccine comprising 9, 10, 11, 12, 13, 14 or 15 or more different pneumococcal capsular saccharides conjugated to protein carriers.
188. The kit of embodiment 187, wherein the second container comprises d) serotype 18C capsular saccharide conjugated to TT.
189. The kit of embodiments 172-184, wherein the second container comprises d) MenC capsular saccharide conjugated to TT and/or MenY capsular saccharide conjugated to TT.
190. The kit of embodiments 172-189 which is a kit as described in any one of embodiments 1-109.
191. A method of decreasing bystander interference on a vaccine comprising a sensitive antigen administered in a primary immunisation schedule caused by administering Pa at birth, comprising one or more of the following steps
   h) reducing the Pa at birth dose or number of Pa components;
   a) including IPV in the vaccine comprising the sensitive antigen;
   b) including Pw in the vaccine comprising the sensitive antigen;
   c) decreasing DT dose in the vaccine comprising the sensitive antigen;
   d) increasing dose of the sensitive antigen;
   e) if CRM is present, decreasing the amount of CRM and/or number of saccharide conjugates on CRM;
   f) if Hib is the sensitive antigen, administering Hib separately from a combination vaccine comprising DTPa;
   g) if HB is the sensitive antigen, administering HB separately from a combination vaccine comprising DTPa;
   h) administering Pa-HB at birth to reduce immune interference on HB.
192. The method of any of embodiment 191, wherein the sensitive antigen is Hib, HB and/or pneumococcal capsular saccharide PS6B conjugated to a carrier protein.
193. The method of embodiment 191, wherein the sensitive antigen is Hib and comprises PRP conjugated to TT, wherein the method of decreasing bystander interference comprises the additional step of including further TT in the vaccine.
194. The method of embodiment 193, wherein the further TT is provided in a separate container.
195. The method of embodiment 193 or 194, wherein the further TT is conjugated to no more than three other saccharides.
196. A method of administering Pa at birth and Hib in a primary immunisation schedule to a patient wherein:
   - Pa is administered at birth; and
   - Hib in the primary immunisation schedule is administered in a vaccine not comprising DTPa.
197. The method of embodiment 196, wherein Hib is administered in combination with MenC and/or MenY capsular saccharide conjugates.
198. A method of administering Pa at birth and HB in a primary immunisation schedule to a patient wherein:
   - Pa is administered at birth; and
   - HB in the primary immunisation schedule is administered in a vaccine not comprising DTPa.
199. The method of any of embodiments 191-198, wherein PT (or PT derivative) is present in Pa at birth and is at a dose which does not exceed 10µg, 1-9, 1.5-8, 2-6, 2.5-5µg per 0.5 mL dose.
200. The method of any of embodiments 191-199, wherein FHA is present in Pa at birth and is at a dose which does not exceed 10µg, 1-9, 1.5-8, 2-6, 2.5-5µg per 0.5 mL dose.
201. The method of any of embodiments 191-200, wherein PRN is present in Pa at birth and is at a dose which does not exceed 6µg, 0.5-6, 0.8-5, 1-4, 2-3µg per 0.5 mL dose.
202. The method of any of embodiments 191-201, wherein PT is present in Pa at birth at a dose of approximately 2.5µg, FHA is present at a dose of approximately 2.5µg and PRN is present at a dose of approximately 0.8µg per 0.5mL dose.
203. The method of any of embodiments 191-201, wherein PT is present in Pa at birth at a dose of approximately 5µg, FHA is present at a dose of approximately 5µg and PRN is present at a dose of approximately 2.5µg per 0.5mL dose.
204. The method of any of embodiments 191-203, wherein PT in Pa at birth is recombinant.
205. The method of embodiments 191-204, wherein Pa at birth comprises PT.
206. The method of embodiments 191-205, wherein Pa at birth comprises FHA.
207. The method of embodiments 191-206, wherein Pa at birth comprises PRN.
208. The method of embodiments 191-207, wherein Pa at birth does not comprise PT.
209. The method of embodiments 191-208, wherein Pa at birth does not comprise FHA.
210. The method of embodiments 1-19, wherein Pa at birth does not comprise PRN.

## Claims

1. A method of decreasing bystander interference on a vaccine comprising a sensitive antigen administered in a primary immunisation schedule caused by administering Pa at birth, comprising one or more of the following steps
a) reducing the Pa at birth dose or number of Pa components;
b) including IPV in the vaccine comprising the sensitive antigen;
c) including Pw in the vaccine comprising the sensitive antigen;
d) decreasing DT dose in the vaccine comprising the sensitive antigen;
e) increasing dose of the sensitive antigen;
f) if CRM is present, decreasing the amount of CRM and/or number of saccharide conjugates on CRM;
g) if Hib is the sensitive antigen, administering Hib separately from a combination vaccine comprising DTPa;
h) if HB is the sensitive antigen, administering HB separately from a combination vaccine comprising DTPa;
i) administering Pa-HB at birth to reduce immune interference on HB.

2. The method of claim 1, wherein the sensitive antigen is Hib, HB and/or pneumococcal capsular saccharide PS6B conjugated to a carrier protein.

3. The method of claim 1 wherein the sensitive antigen is Hib and comprises PRP conjugated to TT, wherein the method of decreasing bystander interference comprises the additional step of including further TT in the vaccine, optionally wherein said further TT is provided in a separate container.

4. The method of claim 3, wherein the further TT is conjugated to no more than three other saccharides.

5. Hib or HB for use in a primary immunisation schedule in a patient to whom Pa has been administered at birth, wherein in said primary immunisation schedule said Hib or HB is in a vaccine not comprising DTPa.

6. Hib for the use as claimed in claim 5, wherein said Hib is administered in combination with MenC and/or MenY capsular saccharide conjugates.

7. The method of, or the Hib or HB for the use as claimed in, any one of claims 1 to 6, wherein,
said Pa administered at birth comprises PT;
said Pa administered at birth comprises FHA; and/or
said Pa administered at birth comprises PRN.

8. The method of, or the Hib or HB for the use as claimed in, any one of claims 1 to 7, wherein,
PT (or PT derivative) is present in said Pa administered at birth and is at a dose which does not exceed 10µg, 1-9, 1.5-8, 2-6, 2.5-5µg per 0.5 mL dose;
FHA is present in said Pa administered at birth and is at a dose which does not exceed 10µg, 1-9, 1.5-8, 2-6, 2.5-5µg per 0.5 mL dose; and/or
PRN is present in said Pa administered at birth and is at a dose which does not exceed 6µg, 0.5-6, 0.8-5, 1-4, 2-3µg per 0.5 mL dose.

9. The method of, or the Hib or HB for the use as claimed in, any one of claims 1 to 8, wherein,
PT is present in said Pa administered at birth at a dose of approximately 2.5µg, FHA is present at a dose of approximately 2.5µg and PRN is present at a dose of approximately 0.8µg per 0.5mL dose; or
PT is present in said Pa administered at birth at a dose of approximately 5µg, FHA is present at a dose of approximately 5µg and PRN is present at a dose of approximately 2.5µg per 0.5mL dose.

10. The method of, or the Hib or HB for the use as claimed in, any one of claims 1 to 9, wherein PT in said Pa administered at birth is recombinant.

11. The method of, or the Hib or HB for the use as claimed in, any one of claims 1 to 8, wherein,
said Pa administered at birth does not comprise PT;
said Pa administered at birth does not comprise FHA; and/or
said Pa administered at birth does not comprise PRN.

12. A kit of vaccines for coadministration comprising two or more containers, wherein
the first container comprises
a) Hib comprising PRP conjugated to TT
b) optionally DTP
c) optionally one or more further antigens
the second container comprises
d) a vaccine comprising one or more protein-conjugated bacterial saccharides one or more of which is/are conjugated to TT
e) optionally one or more further antigens
and, optionally, a third container comprises
f) a vaccine comprising TT
wherein:
(i) the total amount of TT in the kit not conjugated to Hib is 31µg to 55µg, 35µg to 50µg or 40µg to 45µg;
(ii) the total amount of TT in the kit not in the first container is 1µg to 25µg, 5µg to 20µg, or 10µg to 15µg;
(iii) the amount of TT present in the first container, but not conjugated to PRP in Hib, is 20µg to 40µg or 25µg to 35µg or around or exactly 30µg; and/or
(iv) the amount of TT conjugated to PRP in Hib is 10µg to 40µg, 15µg to 35µg, 20µg to 30µg or around or exactly 25µg.

13. The kit of claim 12, wherein PRP in Hib is conjugated through an ADH linker to TT.

14. The kit of claim 12 or claim 13, wherein the number of different protein-conjugated bacterial saccharides in the second container which are conjugated to TT is 1, 2, 3 or 4.

15. The kit of claims 12-14, wherein the second container comprises d) a pneumococcal vaccine comprising 9, 10, 11, 12, 13, 14 or 15 or more different pneumococcal capsular saccharides conjugated to protein carriers.
